# FASCICULE DE BREVET EUROPEEN

(11) **EP 3 400 210 B1**
(45) Date de publication et mention de la délivrance du brevet: **29.09.2021**
(21) Numéro de dépôt: 17701176.4
(22) Date de dépôt: 05.01.2017
(51) Int. Cl.: C07C 259/06, A61K 31/16, A61K 31/165, A61K 31/403, A61K 31/404, A61K 31/44, A61K 31/47, A61K 31/517, A61K 45/06, C07C 259/10, C07D 213/81, C07D 215/14, C07D 239/74, C07C 235/38, C07C 235/64, C07D 209/42, C07D 209/86, C07D 213/65, A61P 35/00

(54) **COMPOSÉS"MULTI-CIBLES"À ACTIVITÉ INHIBITRICE DES HISTONE-DÉSACÉTYLASES ET DE LA POLYMÉRISATION DE LA TUBULINE POUR SON UTILISATION DANS LE TRAITEMENT DU CANCER**
»MULTI-TARGET -VERBINDUNGEN MIT INHIBITORISCHER AKTIVITÄT GEGEN HISTON-DEACETYLASEN UND TUBULINPOLYMERISIERUNG ZUR VERWENDUNG BEI DER BEHANDLUNG VON KREBS
"MULTI-TARGET" COMPOUNDS WITH INHIBITORY ACTIVITY TOWARDS HISTONE DEACETYLASES AND TUBULIN POLYMERISATION, FOR USE IN THE TREATMENT OF CANCER

(30) Priorité: 05.01.2016 FR 1650043
(43) Date de publication de la demande: 14.11.2018
(73) Titulaire: Université Paris-Saclay, 91190 Gif-sur-Yvette (FR); Centre National de la Recherche Scientifique (CNRS), 75016 Paris (FR)
(72) Inventeur: ALAMI, Mouâd, 77600 Bussy Saint Georges (FR); HAMZE, Abdallah, 91300 Massy (FR); BRION, Jean-Daniel, 95320 Saint Leu La Foret (FR); BIGNON, Jérôme, 91530 Le Val Saint Germain (FR); DUBOIS, Joëlle, 91400 Gometz-la-ville (FR)
(74) Mandataire: Regimbeau
(86) Numéro de dépôt international: PCT/FR2017/050032
(87) Numéro de publication internationale: WO 2017/118822

(56) Documents cités:
- WO-A1-2008/122620
- WO-A1-2013/026942
- XUAN ZHANG ET AL: "The discovery of colchicine-SAHA hybrids as a new class of antitumor agents", BIOORGANIC & MEDICINAL CHEMISTRY, vol. 21, no. 11, 31 mars 2013 (2013-03-31) , pages 3240-3244, XP055300402, GB ISSN: 0968-0896, DOI: 10.1016/j.bmc.2013.03.049
- XUAN ZHANG ET AL: "Design, synthesis and biological evaluation of colchicine derivatives as novel tubulin and histone deacetylase dual inhibitors", EUROPEAN JOURNAL OF MEDICINAL CHEMISTRY., vol. 95, 18 mars 2015 (2015-03-18), pages 127-135, XP055300400, FR ISSN: 0223-5234, DOI: 10.1016/j.ejmech.2015.03.035
- MAHAL KATHARINA ET AL: "Biological evaluation of 4,5-diarylimidazoles with hydroxamic acid appendages as novel dual mode anticancer agents", CANCER CHEMOTHERAPY AND PHARMACOLOGY, SPRINGER VERLAG, BERLIN, vol. 75, no. 4, 25 janvier 2015 (2015-01-25), pages 691-700, XP035472840, ISSN: 0344-5704, DOI: 10.1007/S00280-015-2685-Z [extrait le 2015-01-25]
- MAHAPATRA DEBARSHI KAR ET AL: "Anti-cancer chalcones: Structural and molecular target perspectives", EUROPEAN JOURNAL OF MEDICINAL CHEMISTRY, vol. 98, 14 mai 2015 (2015-05-14), pages 69-114, XP029163227, ISSN: 0223-5234, DOI: 10.1016/J.EJMECH.2015.05.004
- CHEN GUO-LIANG ET AL: "Discovery of a small molecular compound simultaneously targeting RXR and HADC: Design, synthesis, molecular docking and bioassay", BIORGANIC & MEDICINAL CHEMISTRY LETTERS,, vol. 23, no. 13, 7 May 2013 (2013-05-07), pages 3891-3895, XP028564890, ISSN: 0960-894X, DOI: 10.1016/J.BMCL.2013.04.067

## Description

### DOMAINE DE L'INVENTION

La présente invention concerne la conception de nouvelles molécules dites « multi-cibles » possédant un double pharmacophore et agissant à la fois comme des inhibiteurs des histone-désacétylases (HDAC) et comme agents antivasculaires. L'invention concerne également le procédé de synthèse des molécules « multi-cibles » et leur application dans le traitement du cancer.

### ETAT DE LA TECHNIQUE

Le cancer est une cause majeure de décès dans le monde, à l'origine de 7,6 millions de décès en 2008. Le nombre de décès par cancer dans le monde entier devrait continuer à augmenter, pour atteindre près de 13 millions de décès en 2030. Il existe plus de 100 types différents de cancer ; les cinq cancers les plus mortels sont : le cancer du poumon, le cancer du côlon, le cancer du foie, le cancer de la prostate et le cancer du sein. Le cancer est une pathologie complexe caractérisée par la présence d'une (ou de plusieurs) tumeur maligne formée à partir de la transformation par mutation ou instabilité génétique (anomalies cytogénétiques) d'une cellule initialement normale.

La transformation cellulaire tumorale se traduit notamment par une perte de contrôle du cycle cellulaire, une insensibilité à l'apoptose, des anomalies de la réparation de l'ADN.

Parmi les thérapies utilisées, la chimiothérapie dite conventionnelle impliquant des agents cytotoxiques, seule ou associée à la chirurgie, la radiothérapie, occupe une place majeure. Toutefois les traitements sont fréquemment accompagnés d'effets indésirables, par manque de sélectivité vis-à-vis des cellules tumorales. De plus, la multi-résistance, le principal mécanisme par lequel de nombreux cancers échappent aux traitements, est un facteur important de l'échec de nombreuses chimiothérapies.

Par conséquent, celle-ci se doit d'évoluer sans cesse afin de lever ces principales barrières.

Les progrès récents accomplis dans le traitement des cancers sont liés à l'arrivée des "thérapies ciblées" visant spécifiquement certains mécanismes impliqués dans la régulation et la croissance cellulaires. Cette approche donc plus rationnelle a modifié de façon importante la prise en charge des patients. Les principes actifs utilisés sont généralement mieux tolérés et n'entraînent pas les effets secondaires propres aux chimiothérapies conventionnelles (alopécie, nausées, vomissements). Cependant, ces principes actifs peuvent être à l'origine de toxicités telle que une hausse de la pression artérielle, des maux de tête, de protéinurie, de réactions allergiques, ou d'atteintes digestives.

Les traitements ciblés regroupent plusieurs familles de médicaments antitumoraux : les anticorps monoclonaux, les inhibiteurs de récepteurs à activité tyrosine kinase et les inhibiteurs de l'angiogenèse. Malgré tout l'intérêt de ces thérapies ciblées, les traitements qui visent une cible unique ont montré des résultats limités, en raison de la grande diversité biologique des cancers et l'apparition de phénomènes de résistance. La combinaison de plusieurs principes actifs (ou polychimiothérapie) ayant des mécanismes d'action différents et ciblant les altérations les plus critiques de cette maladie semble être une solution à la condition que la toxicité de chaque principe actif pris séparément ne soit pas cumulée.

Ainsi les pistes de développement s'attachent maintenant à l'utilisation de molécules duales qui inhibent ou modulent plusieurs cibles simultanément. Cette approche visant la découverte de médicaments à cibles multiples (Multi-Target Drug Discovery, MTDD) suscite l'intérêt des industries pharmaceutique.

Les inhibiteurs de tyrosine kinases (ITK) représentent un bon exemple de ce concept de MTDD capables de bloquer la signalisation de VEGFR, PDGFR et d'autres kinases membranaires et/ou cytoplasmiques.

Par exemple, l'imatinib, un ITK ciblant l'enzyme BCR-Abl dans la leucémie myéloïde chronique, a dû faire face rapidement au problème de la rechute. Beaucoup de patients ont développé une résistance à l'imatinib, principalement en raison de l'activation des voies alternatives de récepteur de tyrosine kinases.

Récemment, la FDA a approuvé l'utilisation du sorafénib, un inhibiteur ciblant plusieurs kinases qui a montré une activité antitumorale chez les patients atteints de carcinome rénal avancé et de carcinome hépatocellulaire. Les cibles moléculaires multiples du sorafénib (serine/thréonine kinase « Raf» et récepteurs tyrosine-kinase « VEGFR-2, VEGFR-3, et PDGFR-β») peuvent expliquer ses larges activités précliniques et cliniques. Ainsi, dans le domaine de la découverte de médicaments, différents scenarios ont émergé, comme par exemple, une nouvelle génération de médicaments antitumoraux capables d'inhiber simultanément plusieurs voies biologiques, constituant ainsi une avancée majeure dans cette thérapie.

Par exemple, la demande WO 2007/131364 concerne de nouvelles molécules hybrides possédant deux pharmacophores, un de type calcitriol et l'autre de type comportant une chaîne alkyle ou alcényle possédant un groupement terminal acide hydroxamique, agissant tous deux de façon synergique comme des agonistes des récepteurs de la vitamine D et comme des inhibiteurs de HDAC.

Dans l'article Chen et al. Biorg. Med. Chem. Lett. 2013, 23, p. 3891-3895, des agents ciblant à la fois le récepteur RXR (retinoid X receptor) et l'HDAC sont décrits. Ils sont dérivés du bexarotène et de l'acide subéroylanilide hydroxamique (SAHA).

De nombreuses recherches ont aussi porté sur la molécule naturelle, la combrétastatine A-4 (CA-4). La CA-4 est un stilbène de configuration Z substitué par deux noyaux aromatiques de type 3,4,5-trimethoxyphenyle (cycle A) et 3-hydroxy-4-methoxyphényle (cycle B).

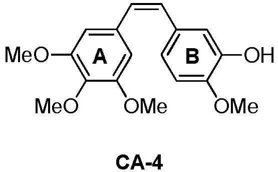

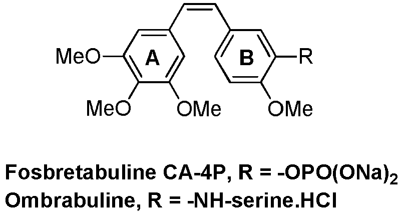

La CA-4 se révèle être très cytotoxique (Cl₅ₒ = 1-2 nM) vis-à-vis de nombreuses lignées cancéreuses humaines ainsi que vis-à-vis de lignées résistantes aux thérapies conventionnelles. Par ailleurs, la CA-4 inhibe la polymérisation de la tubuline en microtubule, en interagissant au niveau du site de fixation de la colchicine. La CA-4 est également connue pour cibler le système vasculaire des tumeurs solides provoquant ainsi un arrêt du flux sanguin, à l'origine d'une nécrose rapide.

Une prodrogue phosphate hydrosoluble de la CA-4, la fosbrétabuline est actuellement en développement clinique de phase III dans le traitement du cancer de la thyroïde, en développement clinique de phase II dans les cancers des poumons à petites cellules et dans les traitements des cancers ovariens résistants au cisplatine. De même, le chlorhydrate de l'analogue amine de la CA-4, l'ombrabuline, est utilisé cliniquement dans le traitement des sarcomes avancés des tissus mous.

Bien que le mécanisme d'action de la fosbrétabuline et de l'ombrabuline ne soit pas complètement élucidé à ce jour, les études montrent que ces VDA (Vascular Disrupting Agents) ciblent la tubuline au niveau du site de la colchicine. Ils inhibent la polymérisation de la tubuline des cellules endothéliales en empêchant la formation des microtubules entrainant un changement morphologique des cellules endothéliales qui s'arrondissent et se détachent de la paroi des vaisseaux provoquant une thrombose. Il en résulte un arrêt du flux sanguin, à l'origine d'une nécrose rapide, particulièrement marquée dans la région centrale des tumeurs, en général résistante aux thérapies conventionnelles. En dépit de leur intérêt en chimiothérapie antitumorale, l'administration de ces agents antivasculaires est fréquemment accompagnée d'effets indésirables délétères liés à une neuro- et une cardiotoxicité chez de nombreux patients, interdisant le traitement chez des patients atteints par ailleurs d'arythmies, d'hypertension non contrôlée ou en période d'infarctus mais également chez des patients asymptomatiques.

Contrairement aux antimitotiques classiques (vinca-alcaloïdes, taxanes et colchicine), la fosbrétabuline et l'ombrabuline exercent leur action antivasculaire à des doses nettement inférieures à la dose maximale tolérée et par conséquent, présentent une fenêtre thérapeutique plus large. Cependant, les VDA induisent en monothérapie une nécrose centrale associée en périphérie à la persistance d'une couronne de cellules cancéreuses viables à l'origine de la réactivation tumorale. Ces résultats justifient les essais cliniques très prometteurs, associant un VDA à un agent chimiothérapeutique conventionnel en vue d'un effet synergique. Néanmoins, si la fosbrétabuline et l'ombrabuline sont utilisés en clinique, il n'en reste pas moins qu'ils sont grevés d'un inconvénient majeur qui est une instabilité chimique imputable à l'isomérisation de la double liaison Z conduisant à l'isomère inactif E imposant une conservation à basse température et à l'abri de la lumière.

Un moyen de contourner le problème récurrent d'instabilité de la double liaison Z de la CA- 4 a été développé dans la demande WO 2008/122620. L'isocombrétastatine A-4 (isoCA-4), et l'isoaminocombrétastatine A-4 (isoNH₂CA-4) ont été identifiées comme deux chefs de file dont le profil biologique (cytotoxicité, inhibition de la polymérisation de la tubuline, induction de l'apoptose, etc.) est rigoureusement identique à celui de la molécule naturelle, sans toutefois présenter le risque d'isomérisation de la double liaison. Ces molécules, sont particulièrement stables et ne se métabolisent pas en présence d'hépatocytes. L'utilisation des deux prodrogues hydrosolubles de l'isoCA-4 et de l'isoNH₂CA-4 a induit, chez la souris nude xénogreffée par la lignée cellulaire humaine tumorale de colon LS174T, une réduction significative de la densité vasculaire autour de la tumeur comparable à celle observée pour l'ombrabuline, prise comme témoin.

En termes d'efficacité tumorale, il a également été montré un effet synergique de l'isoCA-4 en combinaison avec la gemcitabine au sein d'une formulation nanoparticulaire multifonctionnelle (ACS Nano 2014, 8, 2018). L'efficacité antitumorale de ces nanomédicaments isoCA4/Gem-SQ contenant deux principes actifs antitumoraux ayant des mécanismes d'action différents a été évaluée *in vivo* sur un modèle de xénogreffe de tumeurs humaines (LS-174T) sur des souris nude. Il a été montré qu'à la dose de 21 µmol/kg et en comparaison à différents contrôles, la nanoparticule isoCA4/Gem-SQ a conduit à une quasi complète régression tumorale (93%) chez la souris et n'a montré aucune toxicité vis-à-vis des animaux après plusieurs semaines.

D'autre part, l'épigénome humain qui correspond à l'ensemble des modifications intervenant dans la régulation des gènes, est, à la différence du patrimoine génétique, variable et contrôle des régions de l'ADN qui sont actives. Le génome humain s'enroule auteur d'un axe constitué par les histones, qui sont marquée par des enzymes avec des groupements comme l'acétyle et le méthyle. Le marquage et l'enroulement autour des histones déterminent tous deux quels sont les gènes (fragments d'ADN) qui sont actifs et quels sont ceux qui ne le sont pas.

L'acétylation des histones fait partie intégrante de la régulation de la transcription des gènes et elle est étroitement contrôlée dans les cellules normales. Deux enzymes clés jouent un rôle majeur dans ce phénomène d'acétylation : les Histones Acétyl Transférases (HAT) et les Histones Désacétylases (HDAC).

Des changements dans le comportement de ces deux types d'enzymes épigénétiques, HAT et HDAC, semblent jouer un rôle dans le développement de nombreux cancer en induisant l'activation d'un mauvais groupe de gènes. Le réajustement de l'équilibre HDAC/HAT constitue une stratégie anti-tumorale prouvée et a mené au développement d'une famille de médicaments appelée les inhibiteurs de HDAC (HDACi), qui sont actuellement en tête de liste des antitumoraux.

En général, un défaut d'acétylation est lié à une condensation de la chromatine et à une répression de la transcription, alors qu'une hyperacétylation décompacte la chromatine et active la transcription.

Inhiber l'activité des histone-désacétylases (HDAC) induit une hyperacétylatlon, des modifications d'expression de gènes et finalement une différenciation, un arrêt du cycle cellulaire et une mort des cellules tumorales.

Les HDACi peuvent faire régresser des cancers du sang comme des leucémies et des lymphomes, mais aussi des tumeurs solides comme des tumeurs de la prostate, du colon et des reins. A titre d'exemple, le vorinostat ou SAHA (ATU en France), un inhibiteur de HDAC, est indiqué dans le traitement du lymphome réfractaire cutané à cellules T.

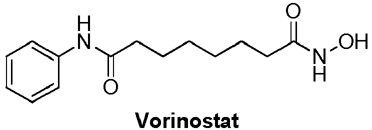

Ainsi de nombreuses recherches ont été faites dans le développement d'antitumoraux utilisés de façon combinée pour obtenir des résultats optimaux. L'activité des inhibiteurs HDAC peut être synergique ou additive avec différents agents antitumoraux à la fois dans les tumeurs malignes hématologiques et solides.

Les composés selon la présente invention ont montré une activité à la fois comme inhibiteurs de HDAC et comme agents antivasculaires. Les molécules visées vont comporter d'une part un motif 1,1'-diaryléthylène ou 1,1'-aryl-hétéroaryléthylène ou 1,1'-dihétéroaryléthylène, responsable de l'inhibition de la polymérisation de la tubuline en microtubule, et d'autre part sur ledit motif, une fonctionnalité greffée responsable de l'inhibition des histones-désacétylases (HDAC).

Ces deux pharmacophores vont être liés par une liaison covalente stable non *« cleavable » in vitro.* Ces composés possèdent des activités cytotoxiques nanomolaires sur diverses lignées cancéreuses humaines comprenant également des lignées résistantes aux traitements usuels.

### EXPOSE DE L'INVENTION

La présente description décrit de nouveaux composés de formule (I) suivante : 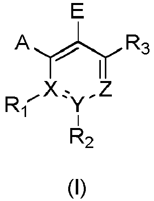 dans laquelle :
- R₂ et R₃ sont différents et l'un des deux parmi R₂ et R₃ représente un groupement A1 de formule générale suivante : 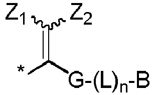 dans lequel :
   B représente un groupement chélatant du zinc ;
   - n représente un entier choisi parmi 0 ou 1 ;
   - L représente :
      - -(CH₂)ᵣ- ;
      - -CH=CH-(CH₂)ᵣ-;
      - -CH=CH-CH=CH-(CH₂)ᵣ-;
      - -C=C-CH≡CH-(CH₂)ᵣ-;
      - -C≡C-(CH₂)ᵣ- ;
      - -C≡C-CH=CH-(CH₂)ᵣ-;
      où r est un entier allant de 0 à 6, de préférence allant de 0 à 4 ;
   - Z₁ représente un atome d'hydrogène, d'halogène ;
   - Z₂ représente un atome choisi parmi un hydrogène, un halogène, un groupement choisi parmi un nitrile, et un groupement B, à la condition que si Z₂=B alors le groupement -(L)ₙ-B est absent de G ;
   - les liaisons 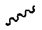 signifient que la double liaison portant Z₁, respectivement, Z₂ est de stéréochimie E ou Z ;
   - * est l'atome de carbone portant R₂ ou R₃;
   - G représente un phényle ou un hétéroaryle:
      - Lorsque G est un phényle, il est substitué par un groupement R₂₀ choisi parmi OMe et SMe en position *para,* relativement à la position de la double liaison portant Z₁ et Z₂;
      - Lorsque G est un hétéroaryle, il est choisi parmi pyridines, indoles, 1-méthylindoles, indolines, carbazoles, benzothiophènes et benzofuranes ;
- l'autre parmi R₂ et R₃ représente :
   - un groupement OMe, lorsque X=Y=Z est un atome de carbone ;
   - lorsque l'un de X, Y, Z est un atome d'azote ;
      ▪ un atome d'hydrogène ;
      ▪ un atome d'halogène ;
      ▪ un groupe hydroxyle ;
      ▪ un groupe nitrile ;
      ▪ un groupe -COYR₁₀ avec Y désignant O ou N et R₁₀ désignant H ou un groupe alkyle en C₁ à C₆, un groupe alcényle en C₂ à C₄, un groupe alcynyle en C₂ à C₄;
      ▪ un groupe -SO₂NR₁₀R₁₁ avec R₁₀, R₁₁, désignant chacun, indépendamment l'un de l'autre, H ou un groupe alkyle en C₁ à C₆, un groupe alcényle en C₂ à C₄, un groupe alcynyle en C₂ à C₄;
      ▪ un groupe -NHSO₂R₁₂ avec R₁₂ désignant un groupe alkyle en C₁ à C₆, un groupe alcényle en C₂ à C₄, un groupe alcynyle en C₂ à C₄, un groupe aryle, un groupe hétéroaryle;
      ▪ un groupe alkyle en C₁ à C₆ ;
      ▪ un groupe alcényle en C₂ à C₄;
      ▪ un groupe alcynyle en C₂ à C₄;
      ▪ un groupe alkoxy en C₁ à C₆ ; ou
      ▪ un groupe -NR₁₃R₁₄ avec R₁₃ et R₁₄ représentant, indépendamment l'un de l'autre un hydrogène ou un groupe alkyle en C₁ à C₆;
- X, Y et Z représentent, indépendamment l'un de l'autre, un atome de carbone ou d'azote à la condition que si X et Z représentent un atome d'azote, Y représente un atome de carbone;
- E représente :
   - un atome d'hydrogène, un groupement -OMe, lorsque X=Y=Z= carbone et R₂ = A₁ ;
   - un atome d'hydrogène, d'halogène, lorsque X=Y=Z= carbone et R₃ = A₁;
   - un atome d'hydrogène, d'halogène, un groupement nitrile, lorsque l'un de X, Y ou Z = azote;
- A représente :
   - un groupement -OMe, lorsque X=Y=Z= carbone ;
   - un atome d'hydrogène, d'halogène, un groupement nitrile, lorsque l'un de X, Y ou Z = azote;
- R₁ représente :
   - un atome d'hydrogène, un groupement -OMe, lorsque X=Y=Z= carbone et R₃ = A₁;
   - un atome d'hydrogène, d'halogène, lorsque X=Y=Z= carbone et R₂ = A₁;
   - un atome d'hydrogène, d'halogène, un groupement nitrile, lorsque l'un de X, Y ou Z = azote;
   ou bien,
- A et E participent ensemble à un cycle aromatique accolé de formule suivante : 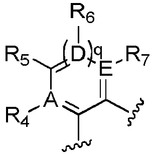
- Alors A, E, D représentent :
   - un atome de carbone ou d'azote ;
- R₁, R₄, R₅, R₆, R₇, si présents, représentent, indépendamment l'un de l'autre :
   - un atome d'hydrogène ;
   - un atome d'halogène ;
   - un groupe hydroxyle ;
   - un groupe alkyle en C₁ à C₆ ;
   - un groupe alcényle en C₂ à C₄;
   - un groupe alcynyle en C₂ à C₄;
   - un groupe alkoxy en C₁ à C₆ ; ou
   - un groupe -NR₁₃R₁₄ avec R₁₃ et R₁₄ représentant, indépendamment l'un de l'autre un hydrogène ou un groupe alkyle en C₁ à C₆ ;
      - q représente un entier compris entre 0 et 2 ;

   et au moins un de A, D, E, X, Y et Z représente un atome d'azote à la condition que si X et Z représentent un atome d'azote, Y représente un atome de carbone ;
   ainsi que ses sels pharmaceutiquement acceptables, ses stéréoisomères et ses prodrogues de conjugaison avec un antigène,
   à l'exception du composé de formule suivante :

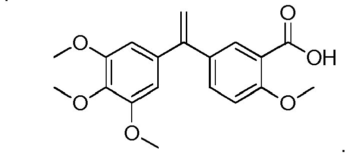

Dans le cadre de la présente invention, B représente un groupement chélatant du zinc choisi parmi la liste comprenant les motifs de formules générales suivantes :

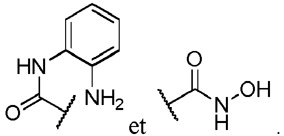

### DESCRIPTION DES FIGURES

La figure 1 décrit le potentiel inhibiteur de la molécule 2 vis-à-vis des HDAC 1 à 11, avec plus particulièrement une activité sélective pour les HDAC8 et HDAC 11. La figure 1 représente plus particulièrement le pourcentage d'inhibition des valeurs contrôle (M) à une concentration test de 1.0⁻⁵ M.
La figure 2 est une illustration permettant la détermination de la Cl₅₀ du composé 2 vis-à-vis des HDAC 8. La figure 2 représente plus particulièrement le pourcentage des valeurs contrôle en fonction du logarithme de la concentration (M) du composé 2.
La figure 3 est une illustration permettant la détermination de la Cl₅₀ du composé 2 vis-à-vis des HDAC 11. La figure 3 représente plus particulièrement le pourcentage des valeurs contrôle en fonction du logarithme de la concentration (M) du composé 2.
La figure 4 décrit le potentiel inhibiteur de la molécule 3 vis-à-vis des HDAC 1 à 11, avec plus particulièrement une activité sélective pour les HDAC8 et HDAC 11. La figure 4 représente plus particulièrement le pourcentage d'inhibition des valeurs contrôle (M) à une concentration test de 1.0⁻⁵ M.
La figure 5 est une illustration permettant la détermination de la Cl₅₀ du composé 3 vis-à-vis des HDAC 11. La figure 5 représente plus particulièrement le pourcentage des valeurs contrôle en fonction du logarithme de la concentration (M) du composé 3.
La figure 6 décrit le potentiel inhibiteur de la molécule 11 vis-à-vis des HDAC 1 à 11, avec plus particulièrement une activité sélective pour les HDAC6 et HDAC 8. La figure 6 représente plus particulièrement le pourcentage d'inhibition des valeurs contrôle (M) à une concentration test de 1.0⁻⁵ M.
La figure 7 décrit le potentiel inhibiteur de la molécule 8 vis-à-vis des HDAC 1 à 11, avec plus particulièrement une activité sélective pour les HDAC6, HDAC8 et HDAC11.

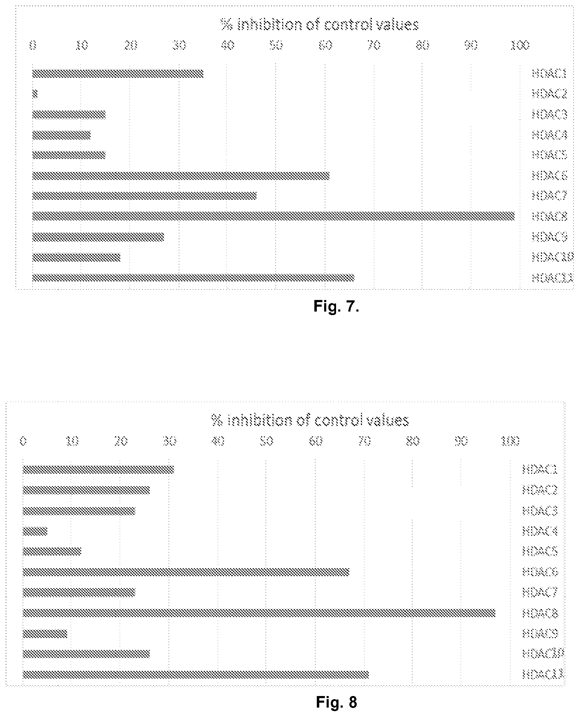

La figure 7 représente plus particulièrement le pourcentage d'inhibition des valeurs contrôle (M) à une concentration test de 1.0⁻⁵ M.
La figure 8 décrit le potentiel inhibiteur de la molécule 12 vis-à-vis des HDAC 1 à 11, avec plus particulièrement une activité sélective pour les HDAC6, HDAC8 et HDAC 11.
La figure 8 représente plus particulièrement le pourcentage d'inhibition des valeurs contrôle (M) à une concentration test de 1.0⁻⁵ M.

### DEFINITIONS

Le terme "halogène", tel qu'utilisé dans la description de la présente invention, désigne les atomes de fluor, chlore, brome et iode. De manière avantageuse, il s'agira du fluor, du brome et du chlore et encore plus avantageusement du fluor ou du chlore. Le terme "alkyle en C₁ à C₆", tel qu'utilisé dans la description de la présente invention, désigne tout groupe hydrocarboné saturé comportant de 1 à 6 atomes de carbone, linéaire ou ramifié, en particulier les groupes méthyle, éthyle, n-propyle, isopropyle, n-butyle, iso-butyle, sec-butyle, tert-butyle, pentyle et hexyle. Le terme "alcényle en C₂ à C₄", tel qu'utilisé dans la description de la présente invention, désigne tout groupe hydrocarboné comportant de 2 à 4 atomes de carbone, linéaire ou ramifié, et comportant au moins une double liaison, tel qu'un groupe vinyle (éthényle).

Le terme "alcynyle en C₂ à C₄", tel qu'utilisé dans la description de la présente invention, désigne tout groupe hydrocarboné comportant de 2 à 4 atomes de carbone, linéaire ou ramifié, et comportant au moins une triple liaison, tel qu'un groupe éthynyle ou propynyle. Le terme "alkoxy en C₁ à C₆", tel qu'utilisé dans la description de la présente invention, désigne tout groupe -O-alkyle, l'alkyle étant tel que défini ci-dessus. Des exemples de groupes alkoxy incluent les groupes méthoxy, éthoxy, propoxy, n-butoxy, iso-butoxy et *tert*-butoxy.

Le terme "(het)Aryle", tel qu'utilisé dans la description de la présente invention, désigne un aryle ou hétéroaryle. Le terme " aryle", tel qu'utilisé dans la description de la présente invention, désigne un ou plusieurs cycles aromatiques ayant de 5 à 10 atomes de carbone, pouvant être accolés. En particulier, les groupes aryles peuvent être des groupes monocycliques ou bicycliques, comme par exemple le groupe phényle ou naphtyle. Avantageusement, le groupe aryle est un phényle. Le terme "hétéroaryle", tel qu'utilisé dans la description de la présente invention, désigne un groupe aromatique comprenant de 5 à 10 atomes cycliques. Les atomes cycliques comprennent des atomes de carbone et un ou plusieurs hétéroatomes, tels que par exemple des atomes de soufre, d'azote ou d'oxygène. L'hétéroaryle selon la présente invention peut être constitué par un ou deux cycles accolés. De préférence, le groupe hétéroarylesera un groupe indolyle, benzothiophényle, benzofuranyle ou benzoimidazolyle. Le terme nitrile, tel qu'utilisé dans la description de la présente invention, désigne un groupe -CN. La formule « -COYR' », telle qu'utilisée dans la description de la présente invention, désigne un acide ou un ester lorsque Y est O, un amide lorsque Y est N. La formule « -SO₂NR'R" » ou « - NHSO₂R' », telle qu'utilisée dans la description de la présente invention, désigne un sulfonamide. L'expression "pharmaceutiquement acceptable", telle qu'utilisée dans la description de la présente invention, désigne ce qui est utile dans la préparation d'une composition pharmaceutique, qui est généralement sûr, non toxique et ni biologiquement ni autrement non souhaitable et qui est acceptable pour une utilisation vétérinaire et/ou pharmaceutique humaine. L'expression "sels pharmaceutiquement acceptables", telle qu'utilisée dans la description de la présente invention, désigne des sels d'un composé qui sont pharmaceutiquement acceptables, comme définis ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphosulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide ptoluènesulfonique, l'acide triméthylacétique, l'acide trifluoroacétique et similaires.
   Avantageusement, il s'agit de l'acide chlorhydrique ; ou
(3) les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium. Avantageusement, le proton acide est déplacé par un ion Na+, notamment en utilisant de l'hydroxyde de sodium. Les sels d'addition d'acide sont formés en particulier avec une fonction amine ou avec une pyridine. Les sels d'addition de base sont formés en particulier avec une fonction acide carboxylique (-COOH), phosphate (-OP(O)(OH)₂) ou encore sulfate (-OSO₃H).

Le terme « stéréoisomères », tel qu'utilisé dans la description de la présente invention, désigne des diastéréoisomères ou des énantiomères. Il s'agit donc d'isomères de configuration. Les stéréoisomères qui ne sont pas des images dans un miroir l'un de l'autre sont ainsi désignés par « diastéréoisomères », et les stéréoisomères qui sont des images l'un de l'autre dans un miroir mais non superposables sont désignés par « énantiomères », encore appelés « isomères optiques ». Un atome de carbone lié à quatre substituants non identiques est appelé un « centre chiral ». Lorsqu'une molécule possède un tel centre chiral, elle est dite chirale et possède deux formes énantiomères. Lorsqu'une molécule possède plusieurs centres chiraux, alors elle possédera plusieurs formes diastéréoisomères et énantiomères. Un mélange équimolaire de deux énantiomères est appelé mélange racémique.

L'expression « composés de la présente invention » ou « composés de formule (I) » telle qu'utilisée dans la présente description désigne des composés de formule (I), mais également de formules plus précises (II), et (III), tels que définis de manière détaillée ci-dessous.

### DESCRIPTION DETAILLEE DE L'INVENTION

Les inventeurs ont mis en évidence que les composés de la présente invention ont une activité à la fois comme inhibiteurs de HDAC et comme agents antivasculaires. Les molécules visées vont comporter d'une part un motif 1,1'-diaryléthylène ou 1,1'-aryl-hétéroaryléthylène ou 1,1'-dihétéroaryléthylène, responsable de l'inhibition de la polymérisation de la tubuline en microtubule, et d'autre part sur ledit motif, une fonctionnalité greffée responsable de l'inhibition des histones-désacétylases (HDAC). Ces deux pharmacophores vont être liés par une liaison covalente non sensible à l'hydrolyse. Ces composés possèdent des activités cytotoxiques nanomolaires sur diverses lignées cancéreuses humaines comprenant également des lignées résistantes aux traitements usuels.

### Composés de la présente invention

L'invention concerne les composés de formule (I) suivante : 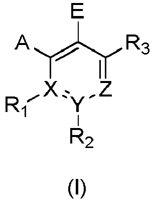 dans laquelle :
- R₂ et R₃ sont différents et l'un des deux parmi R₂ et R₃ représente un groupement A₁ de formule générale suivante : 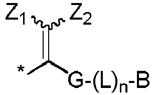 dans lequel :
   - B représente un groupement chélatant du zinc choisi parmi la liste comprenant les motifs de formules générales suivantes : 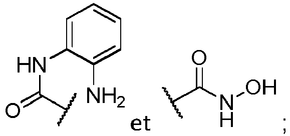
   - n représente un entier choisi parmi 0 ou 1 ;
   - L représente :
      - -(CH₂)ᵣ- ;
      - -CH=CH-(CH₂)ᵣ- ;
      - -CH=CH-CH=CH-(CH₂)ᵣ- ;
      - -C=C-CH≡CH-(CH₂)ᵣ- ;
      - -C≡C-(CH₂)ᵣ- ;
      - -C≡C-CH=CH-(CH₂)ᵣ-;
      où r est un entier allant de 0 à 6, de préférence allant de 0 à 4 ;
   - Z₁ représente un atome d'hydrogène, d'halogène ;
   - Z₂ représente un atome choisi parmi un hydrogène, un halogène, un groupement choisi parmi un nitrile, et un groupement B, à la condition que si Z₂=B alors le groupement -(L)ₙ-B est absent de G ;
   - les liaisons 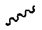 signifient que la double liaison portant Z₁, respectivement, Z₂ est de stéréochimie E ou Z ;
   - * est l'atome de carbone portant R₂ ou R₃ ;
   - G représente un phényle ou un hétéroaryle:
   - Lorsque G est un phényle, il est substitué en position *para* par un groupement R₂₀ choisi parmi OMe et SMe, relativement à la position de la double liaison portant Z₁ et Z₂;
   - Lorsque G est un hétéroaryle, il est choisi parmi pyridines, indoles, 1-méthylindoles, indolines, carbazoles, benzothiophènes et benzofuranes ;
- l'autre parmi R₂ et R₃ représente :
   - un groupement OMe, lorsque X=Y=Z est un atome de carbone ;
   - lorsque l'un de X, Y, Z est un atome d'azote ;
      ▪ un atome d'hydrogène ;
      ▪ un atome d'halogène choisi parmi le fluor, le chlore et le brome, de préférence un atome de chlore ;
      ▪ un groupe hydroxyle ;
      ▪ un groupe nitrile ;
      ▪ un groupe -COYR₁₀ avec Y désignant O ou N et R₁₀ désignant H ou un groupe alkyle en C₁ à C₆, un groupe alcényle en C₂ à C4, un groupe alcynyle en C₂ à C₄;
      ▪ un groupe -SO₂NR₁₀R₁₁ avec R₁₀, R₁₁, désignant chacun, indépendamment l'un de l'autre, H ou un groupe alkyle en C₁ à C₆, un groupe alcényle en C₂ à C₄, un groupe alcynyle en C₂ à C₄;
      ▪ un groupe -NHSO₂R₁₂ avec R₁₂ désignant un groupe alkyle en C₁ à C₆, un groupe alcényle en C₂ à C₄, un groupe alcynyle en C₂ à C₄, un groupe aryle, un groupe hétéroaryle;
      ▪ un groupe alkyle en C₁ à C₆ ;
      ▪ un groupe alcényle en C₂ à C₄;
      ▪ un groupe alcynyle en C₂ à C₄;
      ▪ un groupe alkoxy en C₁ à C₆ ; ou
      ▪ un groupe -NR₁₃R₁₄ avec R₁₃ et R₁₄ représentant, indépendamment l'un de l'autre un hydrogène ou un groupe alkyle en C₁ à C₆;
- X, Y et Z représentent, indépendamment l'un de l'autre, un atome de carbone ou d'azote à la condition que si X et Z représentent un atome d'azote, Y représente un atome de carbone;
- E représente :
   - un atome d'hydrogène, un groupement -OMe, lorsque X=Y=Z= carbone et R₂ = A₁;
   - un atome d'hydrogène, d'halogène, lorsque X=Y=Z= carbone et R₃ = A₁;
   - un atome d'hydrogène, d'halogène, un groupement nitrile, lorsque l'un de X, Y ou Z = azote;
- A représente :
   - un groupement -OMe, lorsque X=Y=Z= carbone ;
   - un atome d'hydrogène, d'halogène, un groupement nitrile, lorsque l'un de X, Y ou Z = azote;
- R₁ représente :
   - un atome d'hydrogène, un groupement -OMe, lorsque X=Y=Z= carbone et R₃ = A₁ ;
   - un atome d'hydrogène, d'halogène, lorsque X=Y=Z= carbone et R₂ = A₁;
   - un atome d'hydrogène, d'halogène, un groupement nitrile, lorsque l'un de X, Y ou Z = azote;
   ou bien,
- A et E participent ensemble à un cycle aromatique accolé de formule suivante : 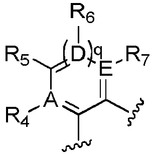
- Alors A, E, D représentent :
   - un atome de carbone ou d'azote ;
- R₁, R₄, R₅, R₆, R₇, si présents, représentent, indépendamment l'un de l'autre :
   - un atome d'hydrogène ;
   - un atome d'halogène choisi parmi le fluor, le chlore et le brome, de préférence un chlore ;
   - un groupe hydroxyle ;
   - un groupe alkyle en C₁ à C₆ ;
   - un groupe alcényle en C₂ à C₄;
   - un groupe alcynyle en C₂ à C₄;
   - un groupe alkoxy en C₁ à C₆ ; ou
   - un groupe -NR₁₃R₁₄ avec R₁₄ et R₁₄ représentant, indépendamment l'un de l'autre un hydrogène ou un groupe alkyle en C₁ à C₆ ;
      - q représente un entier compris entre 0 et 2 ;
   et au moins un de A, D, E, X, Y et Z représente un atome d'azote à la condition que si X et Z représentent un atome d'azote, Y représente un atome de carbone ;
   ainsi que ses sels pharmaceutiquement acceptables, ses stéréoisomères et ses prodrogues de conjugaison avec un antigène.

L'expression « R₁ R₄, R₅, R₆, R₇, si présents » telle qu'utilisée dans la description de la présente invention signifie que les groupements R₁ R₄, R₅, R₆, R₇ seront présents si la valence de l'atome auquel le groupement est, ou serait, lié le permet. L'homme du métier saura aisément déterminer si un tel groupement est présent.

Dans un mode de réalisation, R₁ R₄, R₅, R₆, R₇ sont identiques et représentent chacun un atome d'hydrogène.

Dans certains modes de réalisation, lorsque l'un de R₂ et R₃ représente un groupement A₁, alors l'autre parmi R₂ et R₃ représente un atome d'hydrogène.

Dans d'autres modes de réalisation, lorsque l'un de R₂ et R₃ représente un groupement A₁, alors l'autre parmi R₂ et R₃ représente un atome de chlore.

Dans un mode de réalisation, G est un phényle.

Dans un autre mode de réalisation, G représente une pyridine. Dans un autre mode de réalisation, G représente un indole. Dans un mode de réalisation supplémentaire, G qui représente un carbazole.

La présente description décrit des groupements B chélatant du zinc choisis parmi la liste comprenant les motifs de formules générales suivantes :

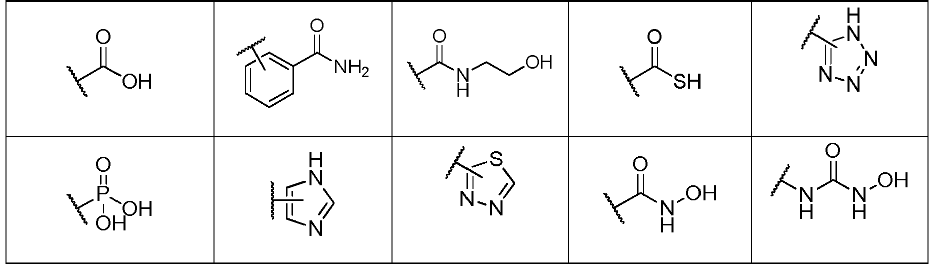

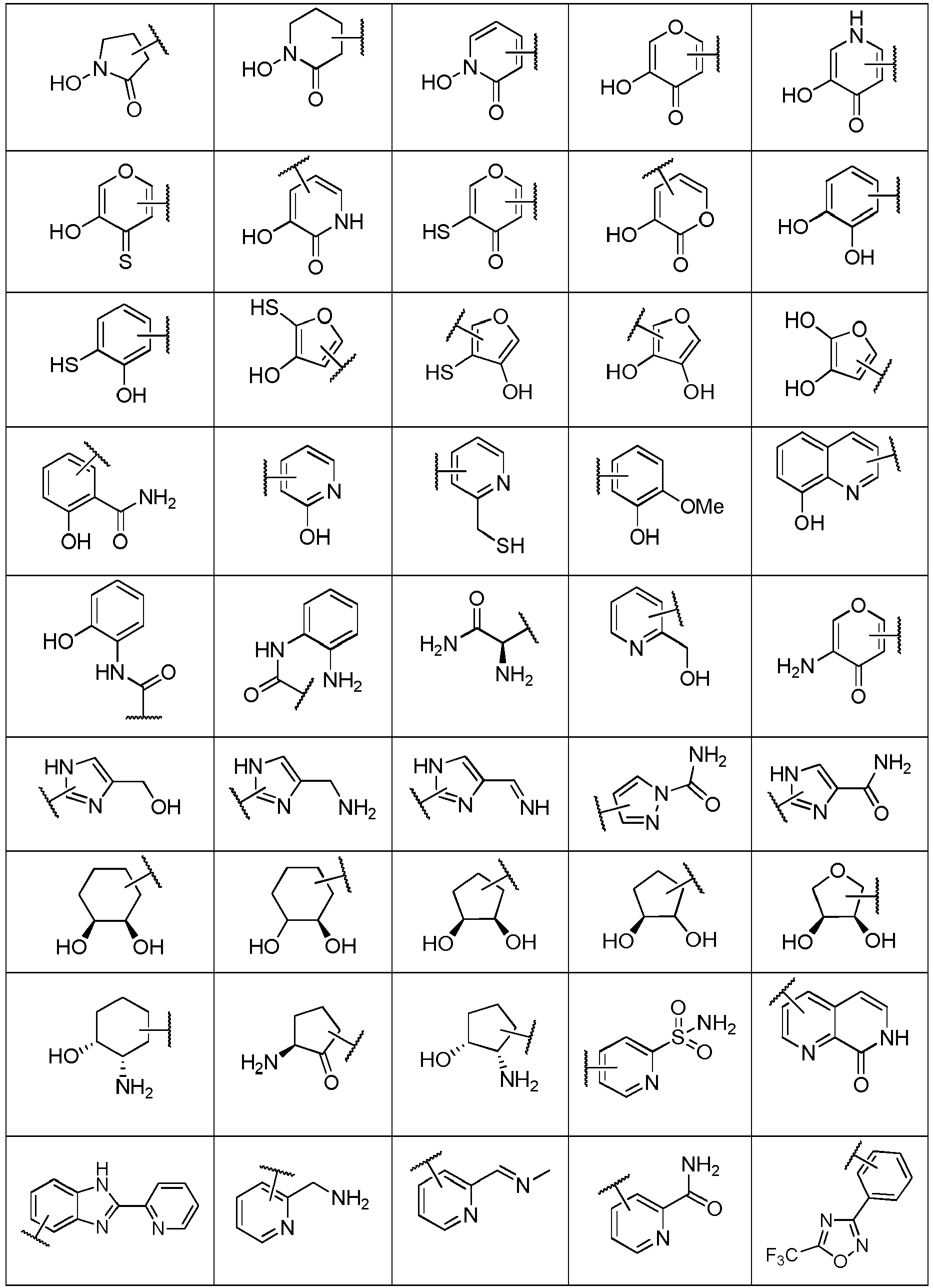

La présente description décrit notamment des groupements B chélatant du zinc choisis parmi la liste comprenant les motifs de formules générales suivantes :

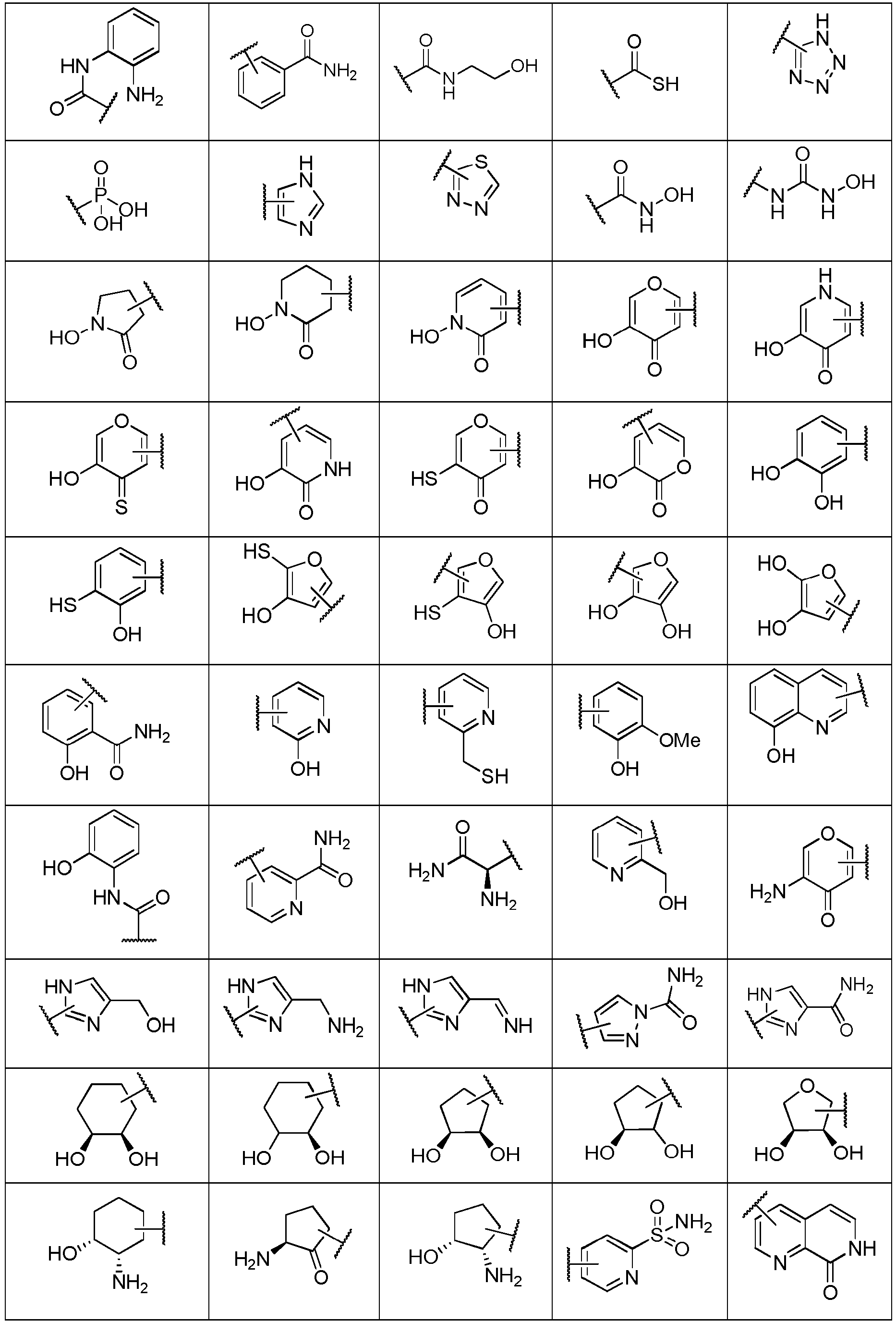

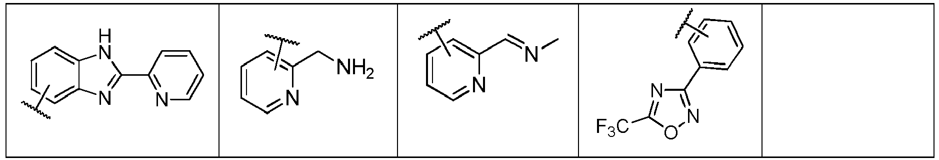

La présente description décrit également un groupement B chélatant du zinc choisi parmi un amide de formule générale suivante : 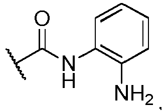 un acide hydroxamique, un acide carboxylique, un benzamide, un n-hydroxyéthylformamide, un mercaptocétone, un tétrazole, un acide phosphonique, un imidazole, et un thiadiazole, de préférence un acide hydroxamique, un benzamide, un n-hydroxyéthylformamide, un mercaptocétone, un tétrazole, un acide phosphonique, un imidazole, un thiadiazole et

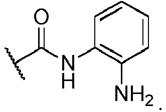

Dans le cadre de la présente invention, le groupement B est choisi parmi un amide de formule générale suivante : 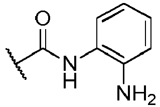 et un acide hydroxamique selon le formule "-CONHOH", encore plus préférentiellement B est un groupement acide hydroxamique selon le formule "-CONHOH".

Dans une variante de réalisation, n=0 et le groupement B est alors directement relié à au groupement G.

Dans une autre variante, n=1 et L est alors choisi parmi la liste telle que définie précédemment.

L'invention concerne plus particulièrement le composé de formule générale suivante 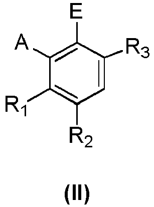 où R₁, R₂, R₃, A et E sont tels que définis soit précédemment soit dans les variantes et modes de réalisations particuliers définis par la suite.

Le composé de la présente invention répond avantageusement à la formule générale suivante :

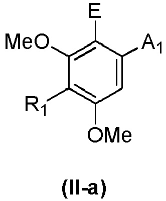

Où R₁, E, et le groupement A₁ sont tels que définis soit précédemment soit dans les variantes et modes de réalisations particuliers définis par la suite.

Dans un mode de réalisation, le composé de formule (**II-a**) est caractérisé en ce que R₁ représente un groupement -OMe, et E représente un atome d'hydrogène. Dans un autre mode de réalisation, le composé de formule (**II-a**) est caractérisé en ce que R₁ représente un groupement -OMe et E représente un atome d'halogène, ledit atome d'halogène est choisi parmi un atome de fluor, de chlore, de brome, encore plus avantageusement E est un atome de chlore.

Dans une variante de réalisation, le composé de formule générale (**II-a**) est caractérisé en ce que R₁ et E sont identiques et représentent chacun un atome d'hydrogène. Dans une autre variante, le composé de formule générale (**II-a**) est caractérisé en ce que R₁ représente un atome d'hydrogène et E représente un atome de chlore.

L'invention concerne aussi le composé de formule générale suivante : 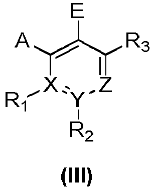 dans laquelle :
- X, Y, Z représentent chacun indépendamment l'un de l'autre un atome d'azote ou un atome de carbone, et ;
   A, E, R₁, R₂ et R₃ sont tels que définis soit précédemment soit dans les variantes et modes de réalisation particuliers définit dans la suite.

L'invention concerne plus particulièrement le composé de formule générale suivante 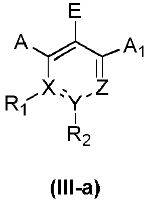 où
- X, Y et Z représentent, indépendamment l'un de l'autre, un atome de carbone ou d'azote à la condition que si X et Z représentent un atome d'azote, Y représente un atome de carbone et ;
- R₂ et R₃ sont tels que définis soit précédemment soit dans les variantes et modes de réalisations particuliers définis par la suite.
- E représente un atome d'hydrogène, d'halogène choisi parmi le fluor, le brome, le chlore, ou un groupement nitrile ;
- A représente un atome d'hydrogène, d'halogène choisi parmi le fluor, le brome, le chlore, un groupement nitrile ;
- R₁ représente un atome d'hydrogène, d'halogène choisi parmi le fluor, le brome, le chlore, un groupement nitrile.

Dans un mode de réalisation, le composé répond avantageusement à la formule générale suivante : 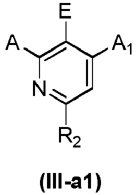 où A₁ est tel que défini soit précédemment soit dans les variantes et modes de réalisations particuliers définis par la suite. Et A, E, R₂ sont tels que définis ci-dessus pour le composé de formule (**III-a**).

Dans une variante de réalisation, le composé de formule (**III-a1**) est caractérisé en ce que A, E et R₂ sont identiques et représentent chacun un atome d'hydrogène.

Dans une variante de réalisation supplémentaire, le composé de formule générale (**III-a1**) est caractérisé en ce que R₂ représente un groupement méthyle, et A, E sont identiques et représentent chacun un atome d'hydrogène.

Dans une autre variante de réalisation, le composé de formule générale (**III-a1**) est caractérisé en ce que R₂ représente un groupement nitrile, et A, E sont identiques et représentent chacun un atome d'hydrogène.

Dans une autre variante de réalisation, le composé de formule générale (**III-a1**) est caractérisé en ce que R₂ représente un atome de chlore et A, E sont identiques et représentent chacun un atome d'hydrogène.

Dans une variante additionnelle, le composé de formule générale (**III-a1**) est caractérisé en ce que R₂ représente un groupement -OMe et A, E sont identiques et représentent chacun un atome d'hydrogène.

Dans un autre mode de réalisation particulier, le composé répond avantageusement à la formule générale suivante : 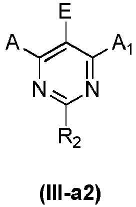 où A₁ est tel que défini soit précédemment soit dans les variantes et modes de réalisations particuliers définis par la suite.et A, E, R₂ sont tels que définis ci-dessus pour le composé de formule (**III-a**).

Dans une autre variante de réalisation, le composé de formule (**III-a2**) est caractérisé en ce que A, E et R₂ sont identiques et représentent chacun un atome d'hydrogène.

Dans une variante de réalisation supplémentaire, le composé de formule générale (**III-a2**) est caractérisé en ce que R₂ représente un groupement méthyle, et A, E sont identiques et représentent chacun un atome d'hydrogène.

Dans une autre variante de réalisation, le composé de formule générale (**III-a2**) est caractérisé en ce que R₂ représente un groupement nitrile, et A, E sont identiques et représentent chacun un atome d'hydrogène.

Dans une autre variante de réalisation, le composé de formule générale (**III-a2**) est caractérisé en ce que R₂ représente un atome de chlore et A, E sont identiques et représentent chacun un atome d'hydrogène.

Dans une variante additionnelle, le composé de formule générale (**III-a2**) est caractérisé en ce que R₂ représente un groupement -OMe et A, E sont identiques et représentent chacun un atome d'hydrogène.

L'invention concerne également le composé de formule générale suivante : 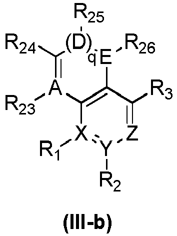 où au moins l'un de A, D, E, X, Y et Z représente un atome d'azote, à la condition que si X et Z représentent un atome d'azote, Y représente un atome de carbone et ;
- q, R₂, R₃ et R₁, R₄, R₅, R₆, R₇, si présents, sont tels que définis soit précédemment soit dans des variantes et modes de réalisation tels que définis dans la suite.

Dans un mode de réalisation préféré, le composé de la présente invention répond à la formule générale (**III-b**), où R₃ = A₁.

Dans un mode de réalisation, le composé répond avantageusement à la formule générale suivante : 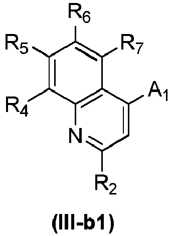 où A₁, et R₂, R₄, R₅, R₆, R₇, sont tels que définis ci-dessus pour le composé de formule générale (**III-b**).

Dans un autre mode de réalisation, le composé de formule générale **(III-b1)** est caractérisé en ce que R₂, R₄, R₅, R₆, R₇ sont identiques et représentent chacun un atome d'hydrogène.

Dans une variante de réalisation supplémentaire, le composé de formule générale **(III-b1)** est caractérisé en ce que R₂ représente un groupement méthyle, et R₄, R₅, R₆, R₇ sont identiques et représentent chacun un atome d'hydrogène.

Dans une autre variante de réalisation, le composé de formule générale **(III-b1)** est caractérisé en ce que R₂ représente un groupement nitrile, et R₄, R₅, R₆, R₇ sont identiques et représentent chacun un atome d'hydrogène.

Dans une autre variante de réalisation, le composé de formule générale **(III-b1)** est caractérisé en ce que R₂ représente un atome de chlore et R₄, R₅, R₆, R₇ sont identiques et représentent chacun un atome d'hydrogène.

Dans une variante additionnelle, le composé de formule générale **(III-b1)** est caractérisé en ce que R₂ représente un groupement -OMe et R₄, R₅, R₆, R₇ sont identiques et représentent chacun un atome d'hydrogène.

Dans un autre mode de réalisation, le composé répond avantageusement à la formule générale suivante : 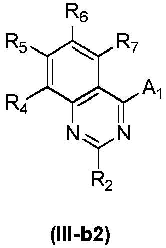 où A₁, et R₂, R₄, R₅, R₆, R₇, sont tels que définis ci-dessus pour le composé de formule générale (**III-b**).

Dans un mode de réalisation particulier, le composé de formule générale (**III-b2**) est caractérisé en ce que R₂, R₄, R₅, R₆, R₇ sont identiques et représentent chacun un atome d'hydrogène.

Dans une variante de réalisation supplémentaire, le composé de formule générale (**III-b2**) est caractérisé en ce que R₂ représente un groupement méthyle, et R₄, R₅, R₆, R₇ sont identiques et représentent chacun un atome d'hydrogène.

Dans une autre variante de réalisation, le composé de formule générale (**III-b2**) est caractérisé en ce que R₂ représente un groupement nitrile, et R₄, R₅, R₆, R₇ sont identiques et représentent chacun un atome d'hydrogène.

Dans une autre variante de réalisation, le composé de formule générale (**III-b2**) est caractérisé en ce que R₂ représente un atome de chlore et R₄, R₅, R₆, R₇ sont identiques et représentent chacun un atome d'hydrogène.

Dans une variante additionnelle, le composé de formule générale (**III-b2**) est caractérisé en ce que R₂ représente un groupement -OMe et R₄, R₅, R₆, R₇ sont identiques et représentent chacun un atome d'hydrogène.

Dans un mode de réalisation préféré et quel que soit le mode de réalisation précédent, les composés de la présente invention sont caractérisés en ce que le groupement A₁ est avantageusement choisi parmi les groupements de formule générales suivantes :

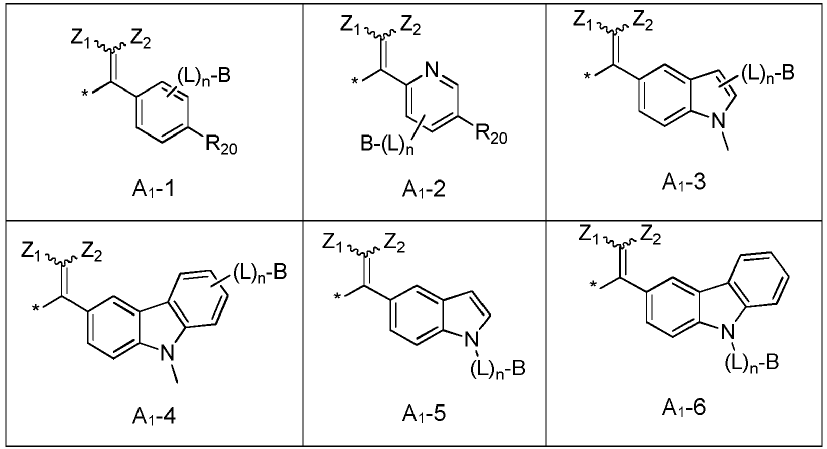

- Z₁ et Z₂ représentent chacun indépendamment de l'autre un atome d'hydrogène, un atome d'halogène choisi parmi le fluor, le chlore et le brome, de préférence le fluor, ou un groupement nitrile ;
- R₂₀, B, L et n sont tels que définis précédemment, et ;
- les liaisons 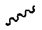 signifient que la double liaison portant Z₁, respectivement, Z₂ est de stéréochimie E ou Z ;
- * est l'atome de carbone portant R₂ ou R₃.

Avantageusement, Z₁ et Z₂ sont identiques et représentent un atome d'hydrogène ou un atome d'halogène choisi parmi le fluor, le chlore, et le brome, de préférence le fluor. Plus particulièrement, Z₁ et Z₂ sont identiques et représentent chacun un atome d'hydrogène ou un atome de fluor.

Dans une variante de l'invention Z₁ et Z₂ sont différents et représentent indépendamment l'un de l'autre un atome d'hydrogène et un groupement nitrile, et le composé de la présente invention est sous la forme d'un mélange de stéréoisomères E ou Z.

Dans un mode de réalisation, pour les groupements A₁-5 et A₁-6, n=1 et L représente avantageusement -(CH₂)ᵣ où r est un entier allant de 0 à 6, de préférence 0 à 4.

Dans une variante de réalisation, pour les groupements A₁-1 à A₁-4, n=0. Dans autre une variante de réalisation, pour les groupements A₁-1 à A₁-4, n=1.

Dans un autre mode de réalisation et quel que soit le mode de réalisation précédents, les composés selon l'invention sont caractérisés en ce que le groupement A₁ est choisi parmi les groupements de formule générales suivantes :

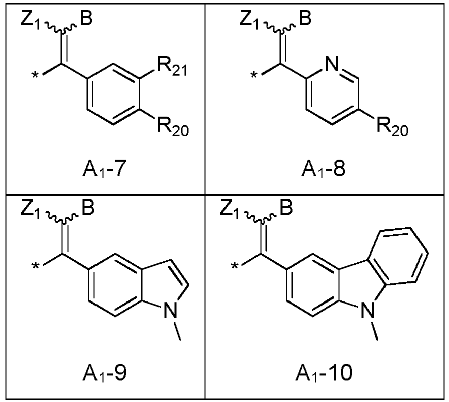

- Z₁ représente un atome d'hydrogène, un atome d'halogène choisi parmi le fluor, le chlore et le brome, de préférence le fluor, ou un groupement nitrile ;
- R₂₀, B, sont tels que définis précédemment ;
- R₂₁ représente un atome d'hydrogène, un groupement choisi parmi -OH, -NH₂, F, N₃, -C=CH, -C≡C(CH₂)ₘOH, où m est un entier compris entre 0 et 5, (E)-CH=CHCH₂OH, (*E*)-CH=CHCOOR, où R est un atome d'hydrogène ou un groupement alkyle en C₁ à C₄, et ;
- les liaisons 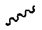 signifient que la double liaison portant Z₁, respectivement, Z₂ est de stéréochimie E ou Z ;
- * est l'atome de carbone portant R₂ ou R₃.

Quel que soit le groupement A₁ défini précédemment, B représente un groupement choisi parmi un acide hydroxamique et un amide de formule générale suivante :

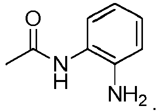

Les composés de la présente invention sont de préférence choisis parmi le groupe suivant :

| | |
|---|---|
| 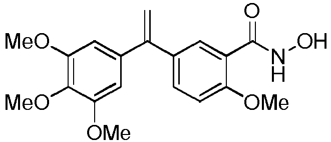 | 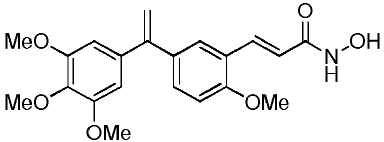 |
| **1** | **2** |
| 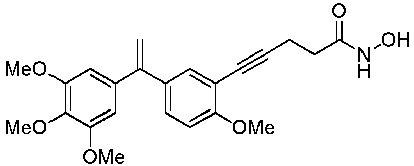 | 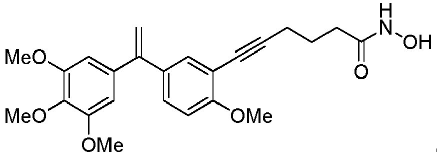 |
| **3** | **4** |
| 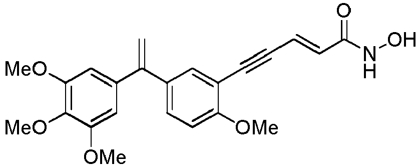 | 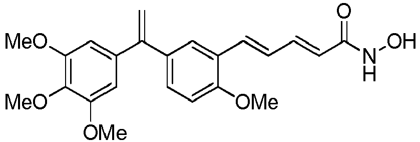 |
| **5** | **6** |
| 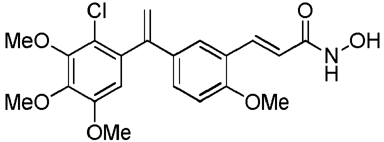 | 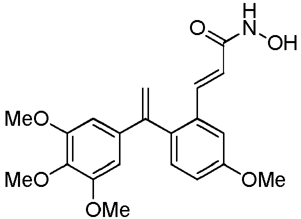 |
| **7** | **8** |
| 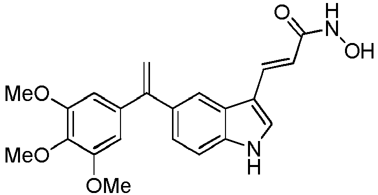 | 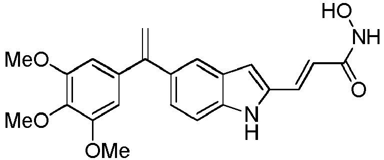 |
| **9** | **10** |
| 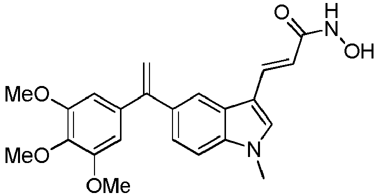 | 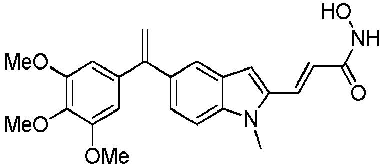 |
| **11** | **12** |
| 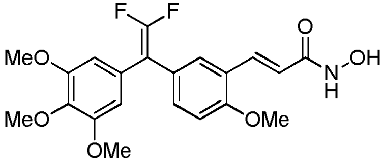 | 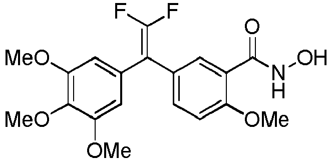 |
| **13** | **14** |
| 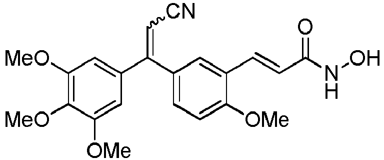 | 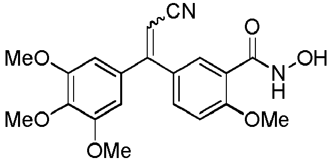 |
| **15** | **16** |
| 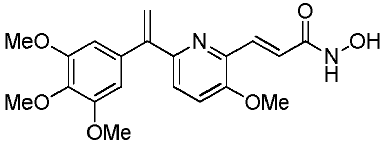 | 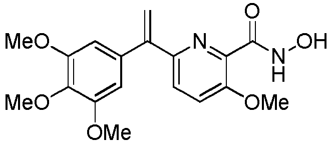 |
| **17** | **18** |
| 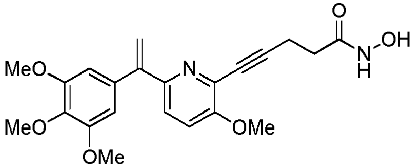 | 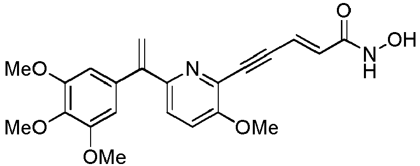 |
| **19** | **20** |
| 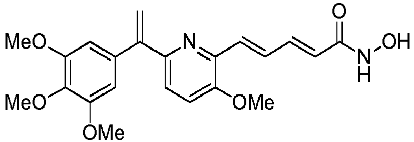 | 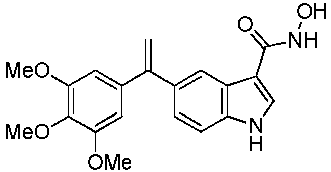 |
| **21** | **22** |
| 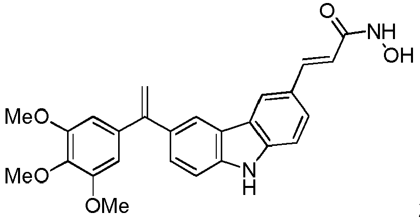 | 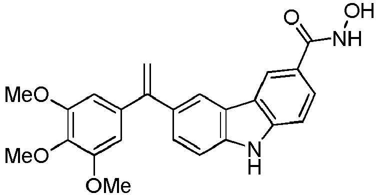 |
| **23** | **24** |
| 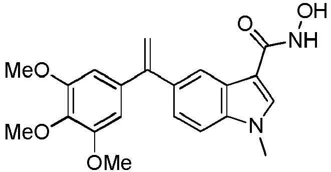 | 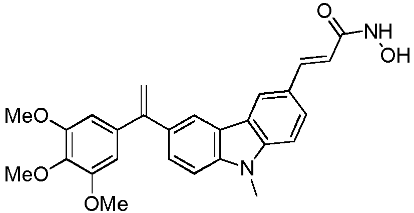 |
| **25** | **26** |
| 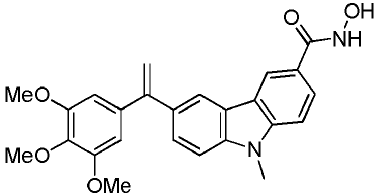 | 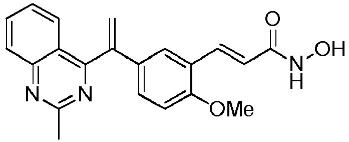 |
| **27** | **28** |
| 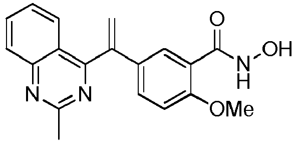 | 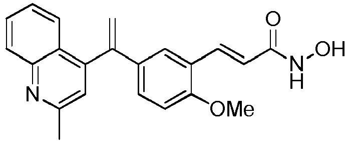 |
| **29** | **30** |
| 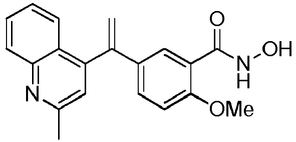 | 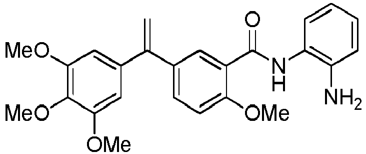 |
| **31** | **32** |
| 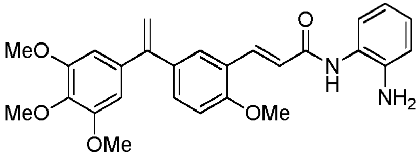 | 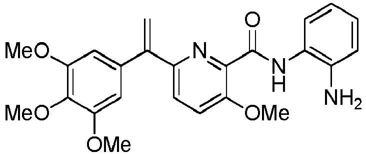 |
| **33** | **34** |
| 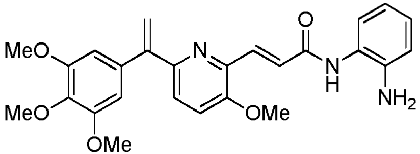 | 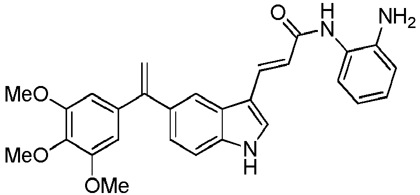 |
| **35** | **36** |
| 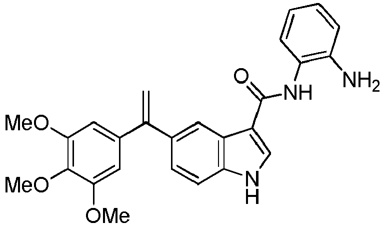 | 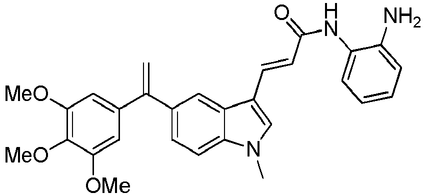 |
| **37** | **38** |
| 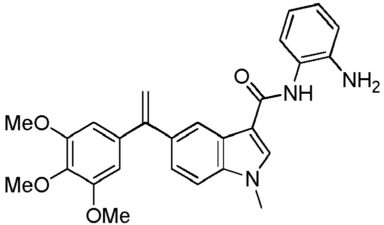 | 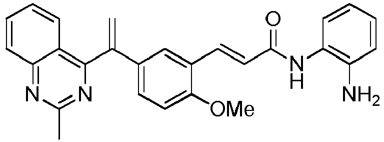 |
| **39** | **40** |
| 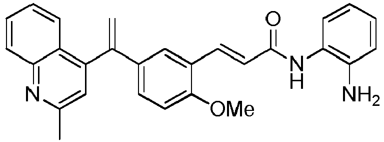 | 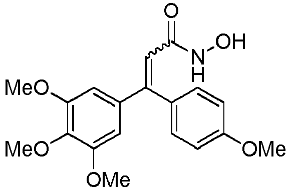 |
| 41 | **42** |
| 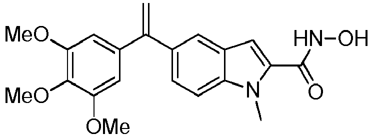 | 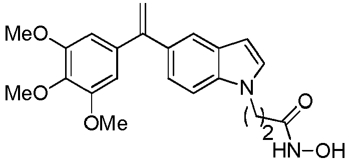 |
| **43** | **44** |

Avantageusement, les composés selon l'invention sont choisis parmi le groupe suivant :

| | |
|---|---|
| 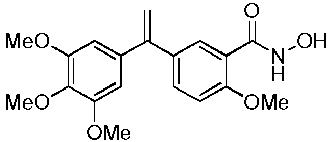 | 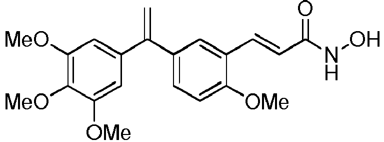 |
| **1** | **2** |
| 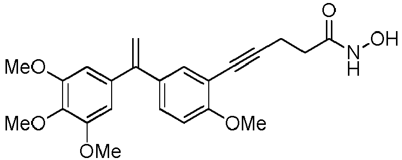 | 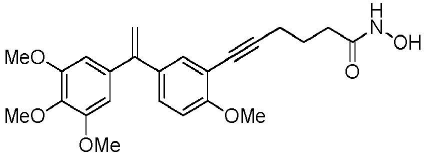 |
| **3** | 4 |
| 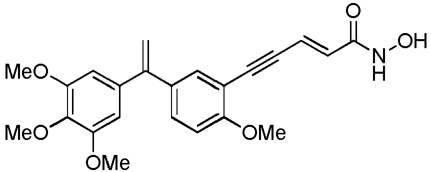 | 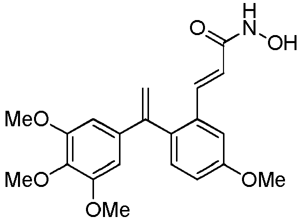 |
| **5** | **8** |
| 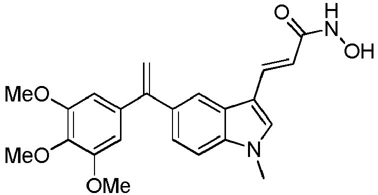 | 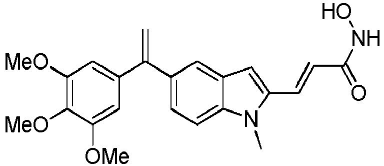 |
| **11** | **12** |
| 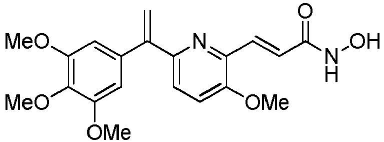 | 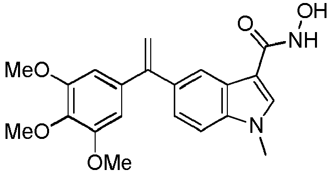 |
| **17** | **25** |
| 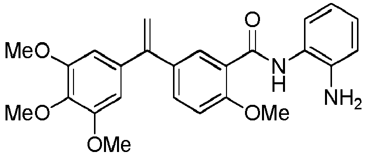 | 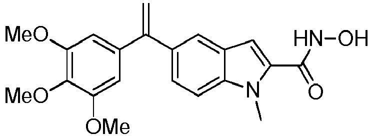 |
| **32** | **43** |
| 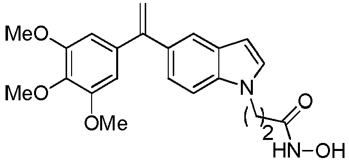 | |
| **44** | |

### Procédé de synthèse des composés de la présente invention

L'invention a également pour objet les procédés de synthèse des composés de la présente invention. Les procédés de synthèse sont courts, comprenant avantageusement 4 étapes. Ces procédés sont compatibles avec les exigences industrielles.

Le couplage d'une tosylhydrazone, aisément accessible, avec un dérivé halogéné conduit aux composés selon l'invention avec d'excellents rendements, avantageusement sans avoir recours à des étapes de protection-déprotection.

Les composés selon l'invention peuvent être préparés selon des procédés connus de l'homme du métier, à partir de produits disponibles dans le commerce ou préparés selon des méthodes connues de l'homme du métier.

Les composés de formule (**II**), (**III**-**a**) et **(III-b1)** peuvent être préparés par un procédé comprenant les étapes successives suivantes :
1 Réaction du composé de formule générale suivante : 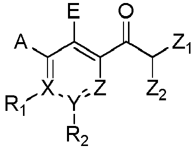 avec la tosylhydrazine pour conduire à la tosylhydrazone de formule générale suivante : 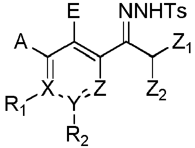 où Z₁, Z₂, X, Y, Z, A, E, R₁, R₂, sont tels que définis précédemment ;
2 Couplage métallo-catalysé de la tosylhydrazone obtenue à l'étape précédente avec un composé de formule générale I-G-Hal pour l'obtention du composé de formule générale suivante : 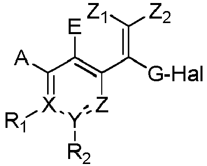 le couplage métallo-catalysé étant avantageusement réalisé avec du palladium, et Hal représente un halogène choisi parmi un atome de brome ou un atome de chlore, où G est tel que défini précédemment ;
3 Couplage métallo-catalysé réalisé sur le composé obtenu à l'étape précédente suivi d'un traitement permettant l'introduction du groupement -(L)ₙ-B et l'obtention du composé de formule générale suivante : 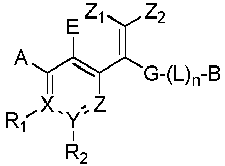 avantageusement le couplage métallo catalysé est réalisé avec du palladium ou du cuivre, et où L, n et B sont tels que définis précédemment.

Les composés de la présente invention, pour lesquels Z₂=B, peuvent être préparés par un procédé comprenant les étapes successives suivantes :
a. Réaction du composé de formule générale suivante : 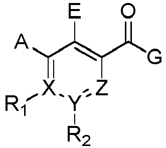 avec un phosphonate de formule générale suivante : 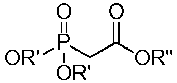 où R' et R" représentent indépendamment l'un de l'autre un alkyle en C₁ à C₄, et permettant l'obtention du composé de formule générale suivante : 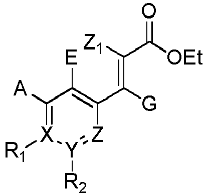 où Z₁, X, Y, Z, A, E, R₁, R₂ et G sont tels que définis précédemment ;
b. Introduction d'un groupement choisi parmi un acide hydroxamique et un amide de formule générale suivante : 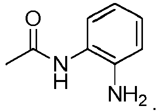 permettant l'obtention du composé de formule générale suivante :

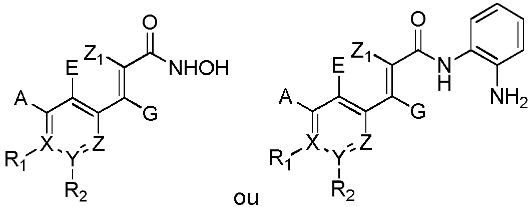

Les composés de formule (**III-b2**) sont préparés préférentiellement par un procédé comprenant les étapes successives suivantes :
1 Réaction du composé de formule générale suivante : 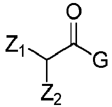 avec la tosylhydrazine pour conduire à la tosylhydrazone de formule générale suivante : 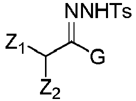 où Z₁, Z₂, et G sont tels que définis à la précédemment ;
2 Couplage métallo-catalysé de la tosylhydrazone obtenue à l'étape précédente avec un composé de formule générale suivante 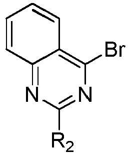 pour l'obtention du composé de formule générale suivante : 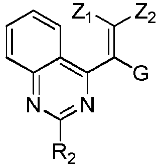 le couplage métallo-catalysé étant avantageusement réalisé avec du palladium, et où R₂ est tel que défini précédemment ;
3 Couplage métallo-catalysé réalisé sur le composé obtenu à l'étape précédente suivi d'un traitement permettant l'introduction du groupement - (L)ₙ-B et l'obtention du composé de formule générale suivante : 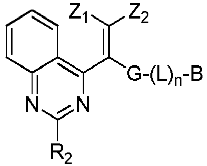 avantageusement le couplage métallo catalysé est réalisé avec du palladium ou du cuivre, et où L, n et B sont tels que définis précédemment.

### Utilisation des composés de la présente invention

Dans certains aspects, l'invention porte sur des composés de formule générale (**I**) ainsi que leurs sels pharmaceutiquement acceptables, leurs stéréoisomères et leurs prodrogues, pour leur utilisation en tant que médicament.

En particulier, les composés de la présente invention peuvent être utilisés en tant que médicaments agissant comme inhibiteur à la fois de HDAC et de la polymérisation de la tubuline, avantageusement en tant que médicaments destinés à traiter ou à prévenir le cancer.

La présente invention concerne préférentiellement une composition pharmaceutique comprenant au moins un composé de formule (I), ainsi que leurs sels pharmaceutiquement acceptables, en association avec un ou plusieurs excipients pharmaceutiquement acceptables.

Dans un mode préféré de réalisation, la composition pharmaceutique comprend au moins un autre principe actif, avantageusement choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-1H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

Avantageusement, le composé de formule générale (**I**) ou la composition pharmaceutique est utilisé dans la prévention ou le traitement du cancer, typiquement les lymphomes malins et les leucémies humaines.

L'invention concerne également un conjugué « anticorps-médicament », aussi appelé ADC, qui est définit par l'association de :
- un anticorps, un fragment d'anticorps, ou un équivalent, de préférence, l'anticorps est un anticorps monoclonal ;
- une molécule de liaison, et ;
- un composé selon la présente invention,
   liés entre eux de façon covalente.

L'anticorps est capable de reconnaître un antigène spécifique d'une cellule tumorale et de s'y lier. Une fois l'ensemble « antigène-ADC » fixé à la paroi de la cellule cancéreuse, ce dernier est internalisé dans la cellule, *via* un endosome. L'ADC est ensuite dégradé dans les conditions caractéristiques du milieu intracellulaire. Le composé de la présente invention est alors libéré pour agir dans la cellule cancéreuse. Ce mécanisme d'action permet de cibler la délivrance du médicament aux zones qui en ont besoin et permet de renforcer l'efficacité du médicament tout en diminuant sa toxicité.

L'invention concerne également une composition pharmaceutique comprenant au moins un conjugué tel que défini ci-dessus. La composition peut comprendre un ou plusieurs excipients pharmaceutiquement acceptables.

Les composés et compositions selon l'invention peuvent être administrés par voie orale, sublinguale, parentérale, sous-cutanée, intramusculaire, intraveineuse, transdermique, locale ou rectale.

Les composés selon la présente invention peuvent être utilisés à des doses comprises entre 0,01 mg et 1000 mg par jour, donnés en une seule dose une fois par jour ou de préférence administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

Les composés selon l'invention peuvent être à la fois utilisés pour diminuer ou inhiber la polymérisation de la tubuline, et diminuer ou inhiber les HDAC, notamment *in vitro* et également *in vivo.*

La présente invention porte également sur une composition pharmaceutique comprenant :
(i) au moins un composé de formule générale (I), et ;
(ii) au moins un autre principe actif,
en tant que produit de combinaison pour une utilisation simultanée, séparée ou étalée dans le temps, avantageusement pour la prévention ou le traitement du cancer, typiquement les lymphomes malins, les leucémies humaines, le carcinome colorectal, le cancer du poumon, les leucémies myéloïdes chroniques, les leucémies myéloïdes chroniques résistantes à l'imatinib, le cancer du sein, le cancer de la prostate, les glioblastomes, les ostéosarcomes, et lignées cellulaires tumorales pancréatiques.

L'invention concerne enfin une composition pharmaceutique comprenant au moins un composé selon la présente invention en association avec un anticorps. La composition peut comprendre un ou plusieurs excipients pharmaceutiquement acceptables. L'anticorps permet de cibler la tumeur. En particulier, la composition pharmaceutique peut comprendre au moins un composé selon la présente invention en association avec un anticorps monoclonal. L'association du composé selon la présente invention avec l'anticorps peut se faire sous la forme de conjugués « anticorps-composé de la présente invention ». L'anticorps et le composé de la présente invention sont typiquement liés de manière covalente par l'intermédiaire d'un lien. En particulier, ce lien sera avantageusement greffé sur le groupement G des composés de la présente invention. L'homme du métier saura déterminer la nature du lien adapté à la liaison du composé selon l'invention avec un anticorps. Ainsi, dans un mode de réalisation, l'invention porte sur un conjugué comprenant un composé selon la présente invention, lié de manière covalente à un anticorps.

L'invention va maintenant être illustrée, de manière non limitative, par les exemples qui suivent.

### EXEMPLES

### Abréviations :

RMN : résonnance magnétique nucléaire
HRMS : spetrométrie de masse haute résolution
MS : spectrométrie de masse
ESI+ : ionisation par électrospray en mode positif
ES : electrospray
CCM : chromatographie sur couche mince
Rf: rapport frontal
Pf : point de fusion
CDCl₃ : chloroforme deutéré
CD₃COCD₃ acétone deutéré
CHCl₃ : chloroforme
MeOH : méthanol
CH₂Cl₂ : dichlorométhane
Et₂O : diéthyléther
AcOEt : acétate d'éthyle
DMF : diméthylformamide
THF : tétrahydrofurane
*t*BuOLi : tert butanolate de lithium
   Pd₂dba₃ : Tris(dibenzylideneacetone)dipalladium
X-Phos : 2-Dicyclohexylphosphino-2',4',6'-triisopropylbiphenye
K₂CO₃ carbonate de potassium
MgSO₄ : sulfate de magnésium
Na₂SO₄ : sulfate de sodium
Et₃N : triéthylamine
PdCl₂(PPh₃)₂ : dichlorure de bis(triphenylphosphine)palladium
CuI : iodure de cuivre
ACN : acétonitrile

### Procédure générale:

Les pics de solvant sont utilisés comme valeurs de référence en RMN ¹H et ¹³C :
- CDCl₃ à 7.26 ppm en RMN ¹H, et à 77.16 ppm en RMN ¹³C ;
- CD₃COCD₃ à 2.05 ppm en RMN ¹H, et à 29.84 ppm en RMN ¹³C.

Les déplacements chimiques δ sont donnés en ppm, et les abréviations suivantes sont données : singulet (s), doublet (d), doublet de doublet (dd), triplet (t), multiplet (m) and large singulet (bs).

Le suivi de réaction et les mélanges de produits sont réalisés par CCM, et les produits sont révélés avec de l'acide phosphomolybdique, ou avec du *para-* anisaldéhyde, ou avec de la vaniline.

Les purifications sont réalisées sur gel de silice 60 (40-63 mm, type 230-400) à pression moyenne (200 mbar). Le dioxane, le dichlorométhane, le cyclohexane et le tétrahydrofurane sont séchés suivant les procédures décrites dans « D. Perrin Purification of Laboratory Chemicals ». Les extraits organiques sont, en général, séchés sur MgSO₄ or Na₂SO₄. Les spectres de masse haute résolution sont enregistrés avec MicrOTOF-Q II. Tous les produits présentés, ci-dessous, sont en accord avec les données RMN ¹H et ¹³C.

### Procédure de synthèse de l'isoCA-4.

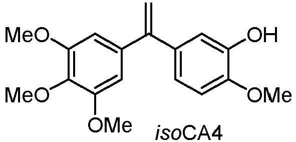

A une solution de *N*-tosylhydrazone (0.42 mmol), de *t*BuOLi (84 mg, 1.05 mmol), de Pd₂dba₃.CHCl₃ (44 mg, 0.042 mmol), et de X-Phos (40 mg, 0.084 mmol) dans le dioxane (6 mL) est ajouté le tert-butyl(5-iodo-2-methoxyphenoxy)dimethylsilane (0.42 mmol) dans du dioxane (1 mL). Le mélange est agité à 90 °C pendant 5 h. Le CH₂Cl₂ (10 mL) est ajouté au mélange refroidit et ensuite filtré sur celite. Après concentration, le résidue est dissout dans du MeOH (3 mL), K₂CO₃ (116.0 mg, 0.84 mmol) est ajouté, et l'agitation est maintenue pendant 2 h. Water (10 mL) est ajouté et la phase aqueuse est extraite avec Et₂O (3 X 10 mL). La phase organique est lavée au Brine (15 mL), séchée sur MgSO₄, et concentrée sous vide pour donner le produit brut qui est ensuite purifié sur gel de silice. Rendement molaire isoCA-4: 69% sous la forme d'une poudre blanche.

Pf: 109-110 °C. CCM: Rf 0.21 (Cyclohexane/AcOEt: 80/20). ¹H RMN (300 MHz, CDCl₃): 6.97 (d, 1H, J = 2.1 Hz), 6.82 (m, 2H), 6.55 (s, 2H), 5.61 (bs, 1H), 5.37 (d, 1H, J = 1.5 Hz), 5.30 (d, 1H, J = 1.5 Hz), 3.91 (s, 3H), 3.87 (s, 3H), 3.81 (s, 6H). ¹³C NMR (75 MHz, CDCl3): 152.8, 149.5, 148.4 (2C), 145.2, 137.8, 137.4, 134.7, 120.2, 114.4, 112.8, 110.1, 105.8 (2C), 60.9, 56.1 (2C), 55.9. *m*/*z* MS (ESI+) 317.24 (M+H)+.

### Ethyl 5-(2-methoxy-5-(1-(3,4,5-trimethoxyphenyl)vinyl)phenyl)pent-4-ynoate (45)

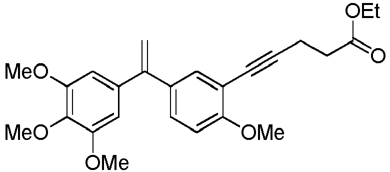

Et₃N (0.7 mL), PdCl₂(PPh₃)₂ (35 mg, 0.05 mmol), et Cul (15 mg, 0.08 mmol) sont ajoutés à une solution de 5-(1-(3-iodo-4-methoxyphenyl)vinyl)-1,2,3-trimethoxybenzene (190 mg, 0.446 mmol) dans THF (3.0 mL). Le 4-pentynoate d'éthyle (122 mg, 0.98 mmol) dans le THF (3.0 mL) est ensuite ajouté au mélange, puis agité à 60 °C pendant 16 h. Après refroidissement, AcOEt (25 mL) est ajouté au mélange brut, qui est lavé avec une solution de NH₄Cl. Après extraction avec AcOEt, la phase organique est séchée avec MgSO₄ et concentrée sous vide. La purification sur gel de silice permet d'obtenir 180 mg de 45 (rendement molaire : 95%) sous la forme d'une huile marron. CCM: Rf = 0.4 (AcOEt /Cyclohexane 3/7, SiO₂). ¹H RMN (300 MHz, CDCl₃) δ 7.32 (d, *J* = 2.3 Hz, 1H), 7.15 (dd, J = 8.6, 2.3 Hz, 1H), 6.74 (d, *J* = 8.7 Hz, 1H), 6.45 (s, 2H), 5.28 (d, *J* = 1.2 Hz, 1H), 5.26 (d, J = 1.2 Hz, 1H), 4.09 (q, *J* = 7.1 Hz, 2H), 3.82 (s, 3H), 3.81 (s, 3H), 3.74 (s, 6H), 2.76 - 2.68 (m, 2H), 2.62 - 2.52 (m, 2H), 1.19 (t, *J* = 7.1 Hz, 3H). ¹³C RMN (75 MHz, CDCl₃) δ 153.1 (C), 149.1 (C), 137.3 (C), 133.8 (C), 133.7 (CH), 129.3 (CH), 113.1 (C), 110.4 (CH), 105.8 (2CH), 92.5 (C), 61.1 (CH₃), 60.8 (CH₂), 56.3 (CH₃), 56.1 (CH₃), 33.9 (CH₂), 15.9 (CH₂), 14.4 (CH₃). HRMS (ES) (M + H)⁺ : *m*/*z* calc. pour C₂₅H₂₉O₆ 425.1964, trouvé 425.1960.

### methyl (E)-3-(2-methoxy-5-(1-(3,4,5-trimethoxyphenyl)vinyl)phenyl)acrylate (46)

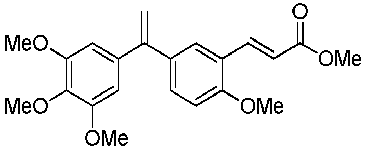

Un mélange de 250 mg (0.587 mmol) de 5-(1-(3-iodo-4-methoxyphenyl)vinyl)-1,2, 3-trimethoxybenzène, 303 mg (3.52 mmol) d'acrylate de méthyle, 7 mg (0.03 mmol) de diacetate de palladium, 18 mg (0.06 mmol) de tri-o-tolylphosphine, et de 3 mL de triethylamine distillée sont chauffée à 110 °C pendant 24 h sous argon dans un tube scellé en Pyrex. Au mélange refroidit est ajouté de l'eau puis de l'AcOEt, après extraction, les phases organiques sont combinées puis lavées avec de l'eau, séchées sur MgSO₄, et concentré sous vide. La purification sur gel de silice permet d'obtenir 185 mg de 46 (rendement molaire : 85%), sous la forme d'une huile incolore. CCM: Rf = 0.3 (AcOEt /Cyclohexane 3/7, SiO₂). ¹H RMN (300 MHz, CDCl₃) δ 7.99 (d, *J* = 16.2 Hz, 1H), 7.53 (d, *J* = 2.3 Hz, 1H), 7.36 (dd, *J* = 8.6, 2.3 Hz, 1H), 6.91 (d, *J* = 8.6 Hz, 1H), 6.56 (s, 2H), 6.54 (d, *J* = 16.2 Hz, 1H), 5.40 (d, *J* = 1.2 Hz, 1H), 5.39 (d, *J* = 1.2 Hz, 1H), 3.94 (s, 3H), 3.91 (s, 3H), 3.84 (s, 6H), 3.82 (s, 3H). ¹³C RMN (75 MHz, CDCl₃) δ 140.3 (CH), 134.0 (C), 131.5 (CH), 129.0 (CH), 118.9 (CH), 113.3 (CH₂), 111.0 (CH), 105.8 (2CH), 61.1 (CH₃), 56.3 (2 CH₃), 55.8 (CH₃), 51.7 (CH₃). HRMS (ES) (M + H)⁺ : *m*/*z* calc. pour C₂₂H₂₅O₆ 385.1651, trouvé 385.1648.

### Préparation d'une solution fraiche d'hydroxylamine.

Une solution d'hydroxyde de potassium (11.2 g, 199.6 mmol) dans le méthanol (28 mL) est ajoutée à une solution d'hydroxylamine hydrochlorure (9.34 g, 134.4 mmol) dans le méthanol (48 mL) sous agitation à 0 °C. Le mélange réactionnel est agité à 0 °C pendant 30 min. le précipité formé est ensuite filtré. Le filtrat est récupéré pour donner la solution d'hydroxylamine qui est conserve au réfrigérateur avant utilisation.

### N-Hydroxy-5-(2-methoxy-5-(1-(3,4,5-trimethoxyphenyl)vinyl)phenyl)pent-4-ynamide (3)

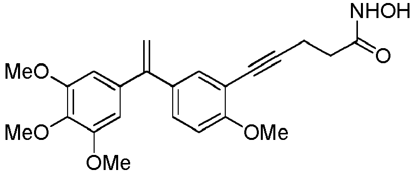

L'ester **45** (170 mg, 0.425 mmol) est ajouté à la solution fraichement préparée d'hydroxylamine (3 mL) à 0 °C. Le mélange réactionnel est ramené à température ambiante et agité à 25 °C pendant 5 h jusqu'à ce l'ester **45** soit totalement converti (la réaction est suivie par CCM). Le solvant est évaporé sous vide donnant le produit brut de la réaction. La purification sur gel de silice permet d'obtenir 150 mg de **3** (rendement molaire : 85%), sous la forme d'un solide blanc. Pf = 73-75 °C. CCM: Rf = 0.15 (CH₂Cl₂/MeOH 95/5, SiO₂). ¹H RMN (300 MHz, Acetone-d₆) δ 10.01 (s, 1H), 8.00 (s, 1H), 7.35 - 7.24 (m, 2H), 7.00 (d, *J* = 8.5 Hz, 1H), 6.60 (s, 2H), 5.38 (d, *J = 0.6* Hz, 2H), 3.89 (s, 3H), 3.79 (s, 6H), 3.76 (s, 3H), 2.72 (t, *J* = 7.2 Hz, 2H), 2.39 (t, *J = 7.3* Hz, 2H). ¹³C RMN (75 MHz, Acetone) δ 150.0 (C), 145.6 (C), 134.4 (C), 133.7 (C), 130.3 (CH), 129.9 (CH), 113.3 (CH₂), 111.5 (CH), 106.7 (2CH), 103.9 (C), 60.6 (CH₃), 56.5 (2CH₃), 56.1 (CH₃), 32.8 (CH₂), 16.4 (CH₂). HRMS (ES) (M + H)⁺ : *m*/*z* calc pour C₂₃H₂₆NO₆ 412.1760, trouvé 412.1758.

### (E)-N-Hydroxy-3-(2-methoxy-5-(1-(3,4,5-trimethoxyphenyl)vinyl)phenyl)acrylamide (2)

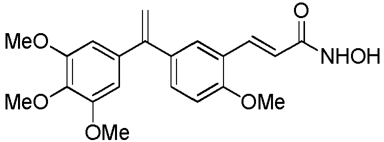

L'ester **46** (348 mg, 0.906 mmol) est ajouté à la solution fraichement préparée d'hydroxylamine (3 mL) à 0 °C. Le mélange réactionnel est ramené à température ambiante et agité à 25 °C pendant 5 h jusqu'à ce l'ester **46** soit totalement converti (la réaction est suivie par CCM). Le solvant est évaporé sous vide donnant le produit brut de la réaction. La purification sur gel de silice permet d'obtenir 210 mg de **2** (rendement molaire : 60%), sous la forme d'un solide blanc. Pf = 93-95 °C. CCM: Rf = 0.30 (CH₂Cl₂/MeOH 95/5, SiO₂). ¹H RMN (300 MHz, Acetone-d6) δ 10.26 (s, 1H), 7.90 (d, *J =* 15.4 Hz, 1H), 7.57 (s, 1H), 7.36 (dd, *J* = 8.6, 1.9 Hz, 1H), 7.08 (d, *J* = 8.6 Hz, 1H), 6.71 - 6.54 (m, 3H), 5.42 (s, 2H), 3.95 (s, 3H), 3.79 (s, 6H), 3.77 (s, 3H). ¹³C RMN (75 MHz, Acetone) δ 158.8 (C), 154.2 (2C), 150.2 (C), 139.3 (C), 137.8 (C), 134.7 (C), 131.6 (C), 128.6 (CH), 124.3 (CH), 119.3 (C), 113.4 (CH₂), 112.1 (CH), 106.7 (2CH), 60.6 (CH₃), 56.5 (2CH₃), 56.1 (CH₃). HRMS (ES) (M + H)⁺ : *m*/*z* calc. pour C₂₁H₂₄NO₆ 386.1604, trouvé 386.1599.

### Procédure générale pour le couplage entre les N-tosylhydrazones et le bromure d'aryle (méthode A)

Sphos (10 mol %), Pd(OAc)₂ (35 mg, 5 mol %) et LiO*t*Bu (2,4 équiv.) ont été ajoutés à une solution d'aryle halogéné (951 mg, 1 équiv.) dans du 1,4-dioxane (12 mL), le mélange a été agité à 110 °C. Une solution de *N*-tosylhydrazone (1,5 équiv.) dans du 1,4-dioxane (12 ml) a été ajoutée au mélange goutte à goutte en 1 heure à 110 °C. Le mélange a été agité à 110 °C pendant 1 h supplémentaire. Après refroidissement, de l'EtOAc a été ajouté et le mélange a été filtré à travers une couche de Celite. Le solvant a été évaporé sous pression réduite et le produit brut a été purifié par chromatographie sur gel de silice.

### Procédure générale pour le couplage de Sonogashira (méthode B).

Et₃N (6 ml / mmol de bromure d'aryle), PdCl₂(PPh₃)₂ (5 mole %) et Cul (mole 10%) sont ajoutés à du bromure d'aryle (1 équiv.). L'alcyne (2 équivalents) a été ajouté au mélange et agité à 50 °C pendant 16 h. Après refroidissement, du cyclohexane a été ajouté au mélange brut et filtré avec de la Celite. Le solvant a été évaporé sous pression réduite et le produit brut a été purifié par chromatographie flash sur gel de silice.

### Procédure générale pour le couplage de Heck (méthode C).

Un mélange d'halogénure d'aryle (1 équivalent), d'acrylate de méthyle (6 équivalents), de Pd(OAc)₂ (3 mol %), de P(*o*-Tol)₃ (6 mol %) et de 5 ml de triéthylamine anhydre a été chauffé à 110 °C pendant 24 heures dans un tube de Pyrex à paroi épaisse et coiffé et qui a été balayé avec de l'Argon sec. Après refroidissement, de l'eau et de l'EtOAc sont ajoutés, après extraction, les solutions organiques combinées ont été lavées à l'eau et séchées sur MgSO₄. Le solvant a été évaporé sous pression réduite et le produit brut a été purifié par chromatographie sur gel de silice.

### Procédure générale pour la formation de dérivés d'acide hydroxamique par couplage avec EDCI (méthode D).

***Saponifiation.*** Une solution d'hydroxyde de sodium (1N, 2 équivalents) est ajoutée à une solution d'ester d'indole (1 équivalent) dans l'éthanol, et le mélange a été chauffé à reflux pendant 1 à 4 heures. A la fin de la réaction (suivi par CCM), l'éthanol est évaporé et la phase aqueuse a été acidifiée avec du HCl 1N, puis extraite par de l'acétate d'éthyle. La phase organique a été séchée sur MgSO₄ et concentrée sous pression réduite. ***Couplage.*** L'acide indole carboxylique (1 mmol, 1 équiv.) est dissout dans 11 ml de DMF, puis le 1-hydroxybenzotriazole (HOBt) (1,5 mmol, 1,5 équiv.) est ajouté en une fois, suivie par l'addition de N-(3-diméthylaminopropyl)-N'-éthylcarbodiimide (EDC.HCl) (1,6 mmol, 1,6 équiv.) et le mélange a été agité à température ambiante pendant 5 h. À cette solution, du chlorhydrate d'hydroxylamine (5 mmol, 5 équiv.) et de la triéthylamine (5 mmol, 5 équiv.) sont ajoutés et l'agitation est poursuivie pendant 15 h. La suspension a été diluée avec de l'eau (40 ml), une extraction avec de l'acétate d'éthyle est effectuée et la couche organique est séchée sur MgSO₄ et concentrée sous pression réduite. Le brut réactionnel a été purifié par HPLC préparatoire.

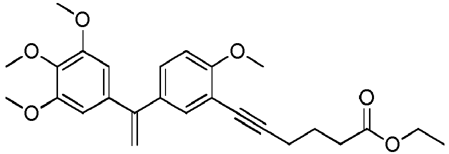

### Ethyl 6-(2-methoxy-5-(1-(3,4,5-trimethoxyphenyl)vinyl)phenyl)hex-5-ynoate (47).

Le composé **47** a été préparé selon la procédure générale B à partir du 5-(1-(3-iodo-4-methoxyphenyl)vinyl)-1,2,3-trimethoxybenzene (0,69 mmol) et d'hexyl-5-ynoate d'éthyle (1,04 mmol). Une purification par Chromatographie sur colonne sur du gel de silice a donné 220 mg de **47** (rendement 73%). Huile brune; Rf = 0,3 (EtOAc / cyclohexane 25/75, SiO₂); ¹H NMR (300 MHz, CDCl₃) δ 7.40 (d, *J* = 2.2 Hz, 1H), 7.22 (d, *J* = 8.6 Hz, 1H), 6.81 (d, *J* = 8.7 Hz, 1H), 6.53 (s, 2H), 5.34 (d, *J* = 7.4 Hz, 2H), 4.13 (q, *J* = 7.1 Hz, 2H), 3.92 - 3.78 (m, 12H), 2.53 (dd, *J* = 13.8, 7.0 Hz, 4H), 2.00 - 1.89 (m, 2H), 1.25 (t, *J* = 7.1 Hz, 4H); ¹³C NMR (75 MHz, CDCl₃) δ 159.8 (C), 153.0 (2C), 149.1 (C), 137.4 (C), 133.8 (C), 133.5 (CH), 129.2 (2CH), 113.1 (CH₂), 112.8 (C), 110.3 (CH), 105.8 (2CH), 93.5 (2C), 61.1 (CH₃), 60.5 (CH₂), 56.3 (2CH₃), 56.1 (CH₃), 33.3 (CH₂), 29.8 (C), 24.2 (CH₂), 19.4 (CH₂), 14.2 (CH₃) ; IR (film, cm⁻¹): 2938, 2837, 1731, 1651, 1581, 1502, 1465, 1414, 1375, 1333, 1271, 1237, 1180, 1127, 1026, 1004, 765; HRMS (ESI) (M+H)⁺, calcd for C₂₆H₃₁O₆: 439.2121, found: 439.2112.

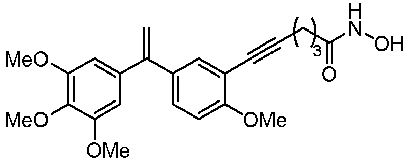

### N-hydroxy-6-(2-methoxy-5-(1-(3,4,5-trimethoxyphenyl)vinyl)phenyl)hex-5-ynamide (4).

L'ester **47** (0.34 mmol) est ajouté à la solution fraichement préparée d'hydroxylamine (2 mL) à 0 °C. Le mélange réactionnel est ramené à température ambiante et agité à 25 °C pendant 5 heures jusqu'à ce que l'ester **47** soit totalement converti (la réaction est suivie par CCM). Le solvant est évaporé sous vide donnant le produit brut de la réaction. La purification sur gel de silice permet d'obtenir 118 mg de **4** (rendement 82%). Huile brune; R_{f} = 0.4 (DCM/MeOH 95/5, SiO₂); ¹H NMR (300 MHz, CDCl₃) δ 7.38 (d, J = 2.2 Hz, 1H), 7.28 (d, J = 2.3 Hz, 1H), 6.87 (d, J = 8.6 Hz, 1H), 6.52 (s, 2H), 5.36 (d, J = 5.3 Hz, 2H), 4.01 - 3.81 (m, 12H), 2.51 (dd, J = 12.1, 5.8 Hz, 4H), 2.17 (d, J = 1.9 Hz, 1H), 1.95 (dd, J = 13.4, 6.5 Hz, 2H); ¹³C NMR (75 MHz, CDCl₃) δ 170.5 (C), 159.8 (C), 153.1 (2C), 149.0 (C), 137.2 (C), 134.2 (C), 133.0 (CH), 129.4 (CH), 113.4 (CH₂), 112.4 (C), 110.4 (CH), 105.8 (2CH), 92.9 (2C), 61.1 (CH₃), 56.3 (2CH₃), 56.1 (CH₃), 46.0 (C), 31.2 (CH₂), 23.4 (CH₂), 18.3 (CH₂); IR (film, cm⁻¹): 2925, 2360, 1666, 1580, 1501, 1464, 1412, 1343, 1294, 1272, 1254, 1236, 1180, 1126, 1082, 1026, 1004, 896, 846, 820, 780, 631; HRMS (ESI) (M + H)⁺, calcd for C₂₄H₂₈NO₆: 426.1917, found: 426.1926.

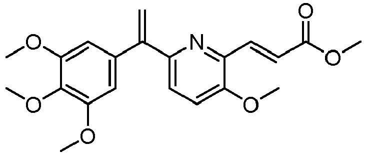

### Methyl (E)-3-(3-methoxy-6-(1-(3,4,5-trimethoxyphenyl)vinyl)pyridin-2-yl)acrylate (48).

Le composé **48** a été préparé selon le procédé C à partir du **2-bromo-3-methoxy-6-(1-(3,4,5-trimethoxyphenyl)vinyl)pyridine** (0,26 mmol) et d'acrylate de méthyle (1,56 mmol). Une purification par chromatographie flash sur du gel de silice a donné 78 mg de **48** (rendement 77%). Huile jaune. TLC: R_{f} = 0.23 (EtOAc: cyclohexane, 3:7); ¹H NMR (300 MHz, CDCl₃) (δ ppm):8.12 (d, J = 15.7 Hz, 1H), 7.22 (d, J = 8.6 Hz, 1H), 7.15 (d, J = 8.6 Hz, 1H), 7.13 (d, J = 15.7 Hz, 1H), 6.60 (s, 2H), 6.07 (d, J = 1.5 Hz, 1H), 5.48 (d, J = 1.5 Hz, 1H), 3.90 (s, 3H), 3.89 (s, 3H), 3.83 (s, 6H), 3.82 (s, 3H). ¹³C NMR (75 MHz, CDCl₃) (δ ppm): 153.0 (C=O), 152.8 (C), 148.3 (2 C), 145.1 (CH), 138.1 (CH), 136.5 (2 C), 137.8 (C), 132.4 (C), 131.5 (C), 131.0 (CH), 123.6 (CH), 117.8 (CH), 116.7 (CH₂), 105.9 (2 CH), 61.0 (OCH₃), 56.2 (2 OCH₃), 55.7 (OCH₃), 51.9 (OCH₃). HRMS (ESI) for C₂₁H₂₄NO₆ [M + H]⁺ : calcd 386.1604, found 386.1594.

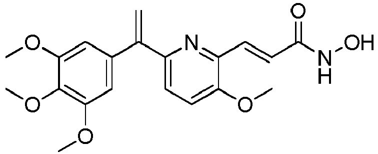

### (E)-N-hydroxy-3-(3-methoxy-6-(1-(3,4,5-trimethoxyphenyl)vinyl)pyridin-2-yl)acrylamide (17).

L'ester **48** (0.90 mmol) est ajouté à la solution fraichement préparée d'hydroxylamine (3 mL) à 0 °C. Le mélange réactionnel est ramené à température ambiante et agité à 25 °C pendant 5 heures jusqu'à ce que l'ester **48** soit totalement converti (la réaction est suivie par CCM). Le solvant est évaporé sous vide donnant le produit brut de la réaction. La purification sur gel de silice permet d'obtenir 201 mg de 17 (rendement 58%). Huile jaune; TLC: R_{f} = 0.17 (MeOH: dichloromethane, 5:95); ¹H NMR (300 MHz, DMSO-d₆) (δ ppm): 10.91 (s, 1H), 9.06 (s, 1H), 7.81 (d, J = 15.4 Hz, 1H), 7.49 (d, J = 8.7 Hz, 1H), 7.27 (d, J = 8.7 Hz, 1H), 6.97 (d, J = 15.4 Hz, 1H), 6.64 (s, 2H), 5.90 (s, 1H), 5.56 (s, 1H), 3.91 (s, 3H), 3.74 (s, 5H), 3.70 (s, 2H). ¹³C NMR (75 MHz, DMSO) (δ ppm): 153.1 (C=O), 152.6 (2 C), 148.8 (C), 147.5 (C), 137.2 (C), 135.4 (2 C), 133.3 (CH), 124.3 (CH), 122.7 (CH), 119.6 (CH), 116.2 (CH₂), 105.5 (2 CH), 60.0 (OCH₃), 55.8 (3 OCH₃). HRMS (ESI) for C₂₀H₂₃N₂O₆ [M+H]⁺: calcd 387.1529, found 387.1475.

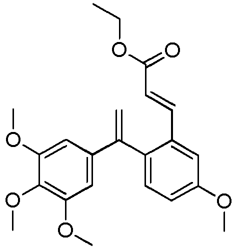

### (E)-ethyl 3-(5-methoxy-2-(1-(3,4,5-trimethoxyphenyl)vinyl)phenyl)acrylate (49).

Le composé **49** a été préparé selon le procédé C à partir de **5- (1-(2-bromo-3-méthoxyphényl)vinyl)-1,2,3-triméthoxybenzène** (0,92 mmol) et d'acrylate d'éthyle (5,5 mmol). Une purification par chromatographie sur colonne de gel de silice a donné 258 mg de **49** (rendement 70%). Huile jaune; R_{f} = 0.4 (EtOAc/Cyclohexane 20/80, SiO₂); ¹H NMR (300 MHz, CDCl₃) δ 7.74 (d, J = 15.9 Hz, 1H), 7.20 - 7.13 (m, J = 9.2, 5.5 Hz, 2H), 6.93 (dd, J = 8.5, 2.5 Hz, 1H), 6.46 (s, 2H), 6.31 (d, J = 15.9 Hz, 1H), 5.77 (s, 1H), 5.11 (s, 1H), 4.17 (q, J = 14.3, 7.1 Hz, 2H), 3.87 - 3.76 (m, 12H), 1.27 (t, J = 6.9 Hz, 3H); ¹³C NMR (75 MHz, CDCl₃) δ 166.8 (C), 159.2 (C), 153.1 (2C), 153.0 (C), 147.1 (C), 143.5 (CH), 137.2 (C), 135.4 (C), 134.6 (C), 131.9 (CH), 119.2 (CH), 116.9 (CH₂), 116.0 (CH), 111.0 (CH), 104.7 (2CH), 61.0 (CH₃), 60.5 (CH₂), 56.2 (2CH₃), 55.5 (CH₃), 14.4 (CH₃); IR (film, cm⁻¹): 2938, 2838, 1711, 1634, 1601, 1581, 1504, 1466, 1413, 1368, 1332, 1233, 1179, 1127, 1032, 1006, 843, 765; HRMS (ESI) (M + H)⁺, calcd for C₂₃H₂₆O₆ Na: 421.1627, found: 421.1638.

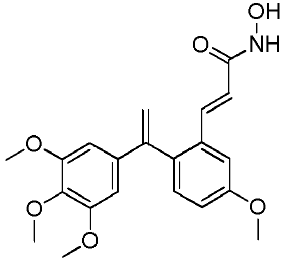

### (E)-N-hydroxy-3-(5-methoxy-2-(1-(3,4,5-trimethoxyphenyl)vinyl)phenyl)acrylamide (8).

L'ester **49** (0.42 mmol) est ajouté à la solution fraichement préparée d'hydroxylamine (2.2 mL) à 0 °C. Le mélange réactionnel est ramené à température ambiante et agité à 25 °C pendant 5 heures jusqu'à ce que l'ester **49** soit totalement converti (la réaction est suivie par CCM). Le solvant est évaporé sous vide donnant le produit brut de la réaction. La purification sur gel de silice permet d'obtenir 111 mg de 8 (rendement 69%). Huile brune; R_{f} = 0.3 (DCM/MeOH 95/5, SiO₂); ¹H NMR (300 MHz, CDCl₃) δ 7.54 (d, J = 15.4 Hz, 1H), 7.24 - 7.17 (m, 2H), 6.92 (d, J = 8.4 Hz, 1H), 6.41 (s, 2H), 6.23 (d, J = 16.7 Hz, 1H), 5.67 (s, 1H), 5.17 (s, 1H), 3.89 - 3.65 (m, 12H); ¹³C NMR (75 MHz, CDCl₃) δ 159.4 (C), 153.2 (2C), 148.3 (C), 148.1 (C), 140.4 (CH), 138.5 (C), 137.3 (C), 135.4 (C), 134.6 (C), 131.8 (CH), 116.7 (CH₂), 116.6 (CH), 115.8 (CH), 111.0 (CH), 104.9 (2CH), 61.1 (CH₃), 56.2 (2CH₃), 55.5 (CH₃); IR (film, cm⁻¹): 2998, 2938, 2835, 2364, 1623, 1603, 1581, 1505, 1465, 1412, 1343, 1291, 1237, 1168, 1127, 1058, 1033, 1005, 902, 845; HRMS (ESI) [M + H]⁺, calcd for C₂₁H₂₄NO₆: 386.1604, found: 386.1608.

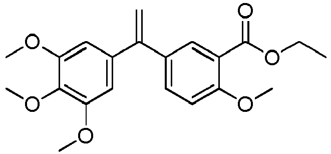

### Ethyl 2-methoxy-5-(1-(3,4,5-trimethoxyphenyl)vinyl)benzoate (50).

Du *t*-BuLi (2,33 ml, 1,7 M dans du pentane) est ajouté à une solution de 637 mg (1,68 mmol) de **5-(1-(3-bromo-4-méthoxyphényl)vinyl)-1,2,3-triméthoxybenzène** dans le THF (10 ml), le mélange est refroidi à -78 ° C pendant 30 minutes. Du chloroformiate d'éthyle (392 mg, 3,62 mmol) a été ajouté au milieu et le mélange est agité à température ambiante pendant 1 h. EtOAc (25 ml) a été ajouté au mélange brut, qui a été lavé avec une solution saturée de NH₄C1. Après extraction avec EtOAc, les extraits combinés ont été séchés sur MgSO₄ et concentrés. Une purification par chromatographie sur colonne de gel de silice a donné 228 mg de **50** (rendement 36%). Huile brune; R_{f} = 0.25 (EtOAc/Cyclohexane 25/75, SiO₂); ¹H NMR (300 MHz, CDCl₃) δ 7.79 (d, J = 2.3 Hz, 1H), 7.40 (dd, J = 8.7, 2.3 Hz, 1H), 6.93 (d, J = 8.7 Hz, 1H), 6.53 (s, 2H), 5.38 (d, J = 4.3 Hz, 2H), 4.35 (q, J = 7.1 Hz, 2H), 3.92 - 3.80 (m, 12H), 1.36 (t, J = 7.1 Hz, 3H); ¹³C NMR (75 MHz, CDCl₃) δ 166.4 (C), 158.8 (C), 153.1 (2C), 148.8 (C), 137.0 (C), 133.5 (C), 133.2 (CH), 131.2 (CH), 120.6 (C), 113.5 (CH₂), 113.0 (C), 111.9 (CH), 105.7 (2CH), 61.1 (CH₂), 61.0 (CH₃), 56.3 (3CH₃), 14.4 (CH₃); IR (film, cm⁻¹): 2838, 1728, 1606, 1579, 1502, 1464, 1451, 1411, 1348, 1305, 1270, 1233, 1180, 1124, 1076, 1024, 1003, 950, 895, 845, 823, 788, 763, 734, 660; HRMS (ESI) (M + H)⁺, calcd for C₂₁H₂₅O₆: 373.1651, found: 373.1645.

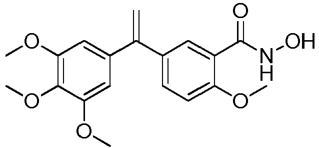

### N-hydroxy-2-methoxy-5-(1-(3,4,5-trimethoxyphenyl)vinyl)benzamide (1).

L'ester **50** (0.42 mmol) est ajouté à la solution fraichement préparée d'hydroxylamine (1.5 mL) à 0 °C. Le mélange réactionnel est ramené à température ambiante et agité à 25 °C pendant 5 heures jusqu'à ce que l'ester **50** soit totalement converti (la réaction est suivie par CCM). Le solvant est évaporé sous vide donnant le produit brut de la réaction. La purification sur gel de silice permet d'obtenir 75 mg de 1 (rendement 77%). Huile brun; Rf = 0.45 (DCM/MeOH 95/5, SiO₂); ¹H NMR (300 MHz, CDCl3) δ 10.35 (s, 1H), 8.26 (d, J = 1.9 Hz, 1H), 7.42 (dd, J = 8.6, 1.7 Hz, 1H), 6.96 (d, J = 8.6 Hz, 1H), 6.50 (s, 2H), 5.42 (d, J = 9.7 Hz, 2H), 4.07 - 3.78 (m, 12H); 13C NMR (75 MHz, CDCl3) δ 163.2 (C), 156.9 (C), 153.1 (2C), 148.7 (C), 137.0 (C), 135.1 (C), 133.3 (CH), 131.5 (CH), 117.9 (C), 114.2 (CH2), 111.2 (CH), 105.8 (2CH), 61.1 (CH3), 56.4 (CH3), 56.3 (2CH3), 29.8 (C); IR (film, cm-1): 2924, 1648, 1580, 1504, 1464, 1412, 1345, 1238, 1183, 1127, 1062, 1007, 895, 846, 824; HRMS (ESI) (M + H)+, calcd for C19H22NO6: 360.1447. found: 360.1446.

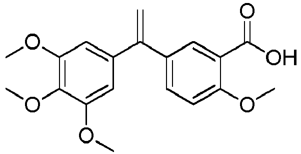

### 2-Methoxy-5-(1-(3,4,5-trimethoxyphenyl)vinyl)benzoic acid (51).

Du KOH (153 mg, 2,72 mmol) est ajouté à une solution de l'ester **50** (125 mg, 0,34 mmol) dans du THF (3 ml), le mélange a été agité à 40 °C pendant 4 h. Après refroidissement, de l'eau (3 ml) a été ajoutée et le mélange et lavée avec de l'éther diéthylique (3 ml). La solution aqueuse a été acidifiée à pH 1 et puis une extraction avec l'EtOAc a été réalisée, la phase organique a été séché sur MgSO₄, le solvant a été éliminé pour donner 73 mg d'acide **51** (rendement 62%). Huile brune; R*_{f}* = 0.1 (EtOAc/Cyclohexane 3/7, SiO₂); ¹H NMR (300 MHz, CDCl₃) δ 8.25 (d, *J* = 2.4 Hz, 1H), 7.53 (dd, *J* = 8.6, 2.4 Hz, 1H), 7.02 (s, 1H), 6.49 (s, 2H), 5.44 (d , *J* = 4.3 Hz, 2H), 3.98 - 3.76(m, 12H); ¹³C NMR (75 MHz, CDCl₃) δ 165.5 (C), 134.6 (CH), 114.5 (CH₂), 111.6 (CH), 107.7 (CH), 105.8 (2CH), 57.0 (CH₃), 56.5 (CH₃), 56.3 (2CH₃); IR (film, cm⁻¹): 2941, 2838, 1731, 1650, 1603, 1581, 1503, 1466, 1413, 1333, 1267, 1234, 1182, 1126, 1004, 827, 765; HRMS (ESI) [M + H]⁺, calcd for C₁₉H₂₀O₆: 367.1158, found: 367.1155.

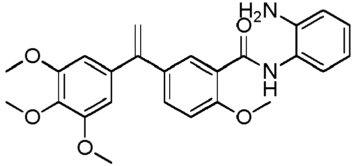

### N-(2-aminophenyl)-2-methoxy-5-(1-(3,4,5-trimethoxyphenyl)vinyl)benzamide (32)

De l'EDCl.HCl (46 mg, 0,24 mmol), DIEA (65 mg, 0,50 mmol), o-phénylènediamine (22 mg, 0,20 mmol), HOBt (37 mg, 0,24 mmol) sont ajoutés à une solution de **2-Methoxy-5-(1-(3,4,5-trimethoxyphenyl)vinyl)benzoic acid** (**51**) (70 mg, 0,20 mmol) dans du DMF (4 mL), le mélange est agité à température ambiante pendant 40 h. Le solvant a été éliminé et purifié par chromatographie sur du gel de silice pour donner 52 mg de **32** (rendement 60%). Huile jaune; R*_{f}* = 0.55 (EtOAc 100%, SiO₂); ¹H NMR (300 MHz, CDCl₃) δ 9.58 (s, 1H), 8.37 (d, *J = 2.4* Hz, 1H), 7.43 (t, *J* = 8.3 Hz, 2H), 7.07 (d, *J = 8.1* Hz, 1H), 7.01 (d, *J* = 8.6 Hz, 1H), 6.90 - 6.80 (m, 2H), 6.54 (s, 1H), 5.44 (d, *J* = 17.8 Hz, 2H), 3.98 - 3.74 (m, 12H); ¹³C NMR (75 MHz, CDCl₃) δ 163.5 (C), 157.2 (C), 153.1 (2C), 148.8 (C), 140.8 (C), 137.1 (C), 135.0 (C), 133.2 (CH), 132.4 (CH), 127.0 (CH), 125.4 (CH), 125.0 (C), 121.3 (C), 119.6 (CH), 118.0, (CH) 114.1 (CH₂), 111.4 (CH), 105.8 (2CH), 61.1 (CH₃), 56.5 (CH₃), 56.3 (2CH₃), 29.8 (C); IR (film, cm⁻¹): 2837, 1661, 1600, 1580, 1534, 1503, 1461, 1449, 1412, 1346, 1238, 1180, 1127, 1005, 823, 752; HRMS (ESI) [M + H]+, calcd for C₂₅H₂₇N₂O₅: 435.1920, found: 435.1909.

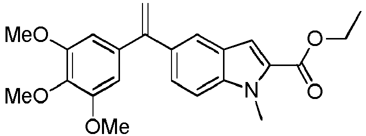

### Ethyl 1-methyl-5-(1-(3,4,5-trimetoxyphenyl)vinyl)-1H-indole-2-carboxylate (52).

Le composé **52** a été préparé selon le procédé A à partir de **(Z)-4-methyl-N'-(1-(3,4,5-trimethoxyphenyl)ethylidene)benzenesulfonohydrazide** (1 mmol) et de l'**ethyl 5-bromo-1-methyl-1H-indole-2-carboxylate** (1,5 mmol). Une purification par chromatographie sur gel de silice a donné 260 mg de **52** (rendement 66%). Huile incolore; R_{f} = 0.56 (EtOAc: cyclohexane, 2:8); ¹H NMR (300 MHz, CDCl₃) (δ ppm): 7.66 (d, *J* = 1.5 Hz, 1H), 7.38 (dd, *J* = 8.8, 1.5 Hz, 0H), 7.33 (d, *J* = 8.8 Hz, 1H), 7.28 (s, 1H), 6.60 (s, 2H), 5.43 (d, *J* = 1.3 Hz, 2H), 4.38 (q, *J* = 7.1 Hz, 2H), 4.09 (s, 3H), 3.89 (s, 3H), 3.80 (s, 6H), 1.41 (t, *J* = 7.1 Hz, 3H). ¹³C NMR (75 MHz, CDCl₃) (δ ppm): 162.1 (C=O), 152.9 (2 C), 150.5(C), 139.5 (C), 137.9 (2 C), 134.0 (C), 128.6 (C), 125.8 (CH), 125.8 (C), 122.3 (CH), 113.0 (CH₂), 110.4 (CH), 109.9 (CH), 105.8 (2 CH), 60.9 (OCH₃), 60.6 (OCH₂), 56.2 (2 OCH₃), 31.8 (CH₃), 14.4 (CH₃). HRMS (ESI) for C₂₃H₂₆NO₅ [M+H]⁺: calcd 396.1783, found 396.1802.

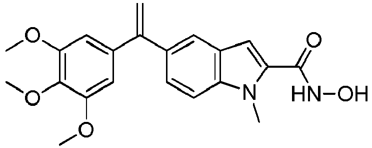

### N-hydroxy-1-methyl-5-(1-(3,4,5-trimethoxyphenyl)vinyl)-1H-indole-2-carboxamide (43).

Ce composé a été préparé selon le procédé D à partir de **Ethyl 1-methyl-5-(1-(3,4,5-trimetoxyphenyl)vinyl)-1H-indole-2-carboxylate (52).** La purification par HPLC a donné 168 mg de 43 (rendement 44%). Solide blanc; mp: 179-181 °C; TLC: R_{f} = 0.23 (MeOH: dichloromethane, 5:95); ¹H NMR (300 MHz, DMSO-*d*₆) (δ ppm): 7.55 (s, 1H), 7.51 (d, *J =* 8.8 Hz, 1H), 7.23 (d, *J* = 8.8 Hz, 1H), 6.90 (s, 1H), 6.57 (s, 2H), 5.43 (d, *J* = 3.6 Hz, 2H), 3.96 (s, 3H), 3.71 (s, 6H), 3.69 (s, 3H). ¹³C NMR (75 MHz, DMSO) (δ ppm): 169.7 (C), 152.6 (2 C), 149.8 (C), 137.9 (C), 137.1 (C), 132.9 (C), 125.6 (C), 123.7 (CH), 122.8 (CH), 122.1 (C), 120.8 (CH), 113.1 (CH₂), 110.1 (CH), 106.4 (C), 105.5 (2 CH), 60.0 (OCH₃), 55.8 (2 OCH₃), 31.2 (CH₃). HRMS (ESI) for C₂₁H₂₃N₂O₅ [M + H]⁺: calcd 383.1585, found 383.1590.

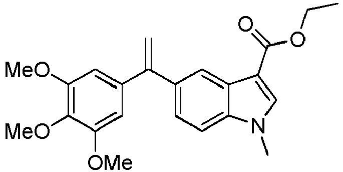

### Ethyl 1-methyl-5-(1-(3,4,5-trimetoxyphenyl)vinyl)-1H-indole-3-carboxylate (53).

Le composé **53** a été préparé selon le procédé A à partir de **(Z)-4-methyl-N'-(1-(3,4,5-trimethoxyphenyl)ethylidene)benzenesulfonohydrazide** (1 mmol) et de l'**ethyl 5-bromo-1-methyl-1H-indole-3-carboxylated'haloindole** (1,5 mmol). Une purification par chromatographie sur colonne sur du gel de silice a donné 352 mg de **53** (rendement 89%). Huile jaune; R_{f} = 0.15 (EtOAc: cyclohexane, 2:8); ¹H NMR (300 MHz, CDCl₃) (δ ppm): 8.24 (s, 1H), 7.80 (s, 1H), 7.29 (d, *J* = 8.7 Hz, 1H), 7.27 - 7.22 (m, overlapped with CDCl₃, 1H), 6.59 (s, 2H), 5.49 (d, *J* = 1.4 Hz, 1H), 5.43 (d, *J* = 1.4 Hz, 1H), 4.35 (q, *J* = 7.1 Hz, 2H), 3.88 (s, 3H), 3.85 (s, 3H), 3.80 (s, 6H), 1.36 (t, *J* = 7.1 Hz, 3H). ¹³C NMR (75 MHz, CDCl₃) (δ ppm): 165.1 (C=O), 152.9 (2 C), 150.8 (C), 138.1 (C), 137.8 (C), 137.0 (C), 135.6 (CH), 135.5 (C), 126.7 (C), 123.7 (CH), 121.6 (CH), 113.5 (CH₂), 109.4 (CH), 107.6 (C), 105.8 (2 CH), 61.0 (OCH₃), 59.8 (OCH₂), 56.2 (2 OCH₃), 33.6 (CH₃), 14.6 (CH₃). HRMS (ESI) for C₂₃H₂₆NO₅ [M+H]⁺: calcd 396.1783, found 396.1811.

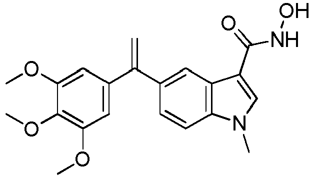

### N-hydroxy-1-methyl-5-(1-(3,4,5-trimethoxyphenyl)vinyl)-1H-indole-3-carboxamide (25)

Ce composé a été préparé selon le procédé D à partir de l**'Ethyl 1-methyl-5-(1-(3,4,5-trimetoxyphenyl)vinyl)-1*H*-indole-3-carboxylate (53).** La purification par HPLC a donné 206 mg de **25** (rendement 54%). Huile rose; TLC: R_{f} = 0.22 (MeOH: dichloromethane, 5:95); ¹H NMR (300 MHz, DMSO-*d*₆) (δ ppm): 11.97 (s, 1H), 8.09 - 7.98 (m, 2H), 7.51 (d, *J* = 8.5 Hz, 1H), 7.21 (dd, *J* = 8.5, 1.8 Hz, 1H), 6.59 (s, 2H), 5.48 (s, 1H), 5.41 (s, 1H), 3.86 (s, 3H), 3.71 (s, 6H), 3.70 (s, 3H). ¹³C NMR (75 MHz, DMSO) (δ ppm): 165.5 (C=O), 152.5 (2 C), 150.1 (C), 137.3 (C), 137.2 (C), 136.8 (C), 136.7 (CH), 134.1 (C), 126.3 (C), 122.7 (CH), 120.3 (CH), 113.5 (CH₂), 110.4 (CH), 106.4 (C), 105.5 (2 CH), 60.0 (OCH₃), 55.8 (2 OCH₃), 33.1 (CH₃). HRMS (ESI) for C₂₁H₂₂N₂O₅ [M + Na]⁺: calcd 405.1426, found 405.1415.

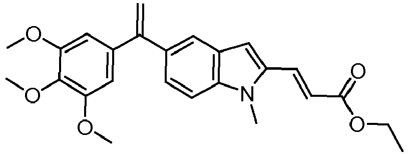

### Ethyl (E)-3-(1-methyl-5-(1-(3,4,5-trimethoxyphenyl)vinyl)-1H-indol-2-yl)acrylate (54)

Le composé **54** a été préparé selon le procédé A à partir de **(Z)-4-methyl-N'-(1-(3,4,5-trimethoxyphenyl)ethylidene)benzenesulfonohydrazide** (1 mmol) et de l'**ethyl (*E*)-3-(5-bromo-1-methyl-1H-indol-2-yl)acrylate** (1,5 mmol). Une purification par chromatographie sur colonne sur du gel de silice a donné 232 mg de 54 (rendement 55%). Huile jaune; TLC: R_{f} = 0.26 (EtOAc: cyclohexane, 2:8); ¹H NMR (300 MHz, CDCl₃) (δ ppm): 7.79 (d, *J* = 15.8 Hz, 1H), 7.32 - 7.24 (m, overlapped with CDCl₃, 1H), 6.94 (s, 1H), 6.63 - 6.54 (m, 3H), 6.51 (d, *J* = 15.8 Hz, 1H), 5.44 (d, *J* = 1.4 Hz, 1H), 5.39 (d, *J* = 1.4 Hz, 1H), 4.29 (q, *J* = 7.1 Hz, 3H), 3.89 (s, 3H), 3.85 (s, 2H), 3.80 (s, 6H), 1.35 (t, *J =* 7.1 Hz, 3H). ¹³C NMR (75 MHz, CDCl₃) (δ ppm): 167.8 (C=O), 153.1 (2 C), 150.7 (C), 137.9 (C), 137.9 (2 C), 137.7 (CH), 135.1 (C), 133.3 (CH), 126.2 (C), 124.1 (CH), 120.3 (CH), 113.6 (CH₂), 112.8 (CH), 112.5 (C), 109.5 (CH), 105.8 (CH), 61.0 (CH₃), 60.2 (CH₂), 56.2 (CH₃), 33.3 (CH₃), 14.5 (CH₃). HRMS (ESI) for C₂₅H₂₈NO₅ [M+H]⁺: calcd 422.1967, found 422.1961.

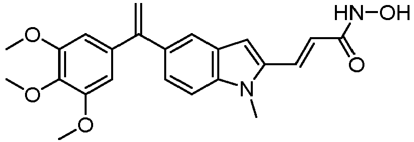

### (E)-N-hydroxy-3-(1-methyl-5-(1-(3,4,5-trimethoxyphenyl)vinyl)-1H-indol-2-yl)acrylamide (12).

Ce composé a été préparé selon le procédé D à partir de l**'Ethyl (*E*)-3-(1-methyl-5-(1-(3,4,5-trimethoxyphenyl)vinyl)-1*H*-indol-2-yl)acrylate (54)**. La purification par HPLC a donné 216 mg de **12** (rendement 53%). Huile jaune; TLC: R_{f} = 0.17 (MeOH: dichloromethane, 5:95); ¹H NMR (300 MHz, Acétone-*d*₆) (δ ppm): 7.74 (d, *J* = 16.4 Hz, 1H), 7.52 (d, *J* = 1.7 Hz, 1H), 7.43 (d, *J* = 8.7 Hz, 1H), 7.23 (dd, *J* = 8.7, 1.7 Hz, 1H), 6.93 (s, 1H), 6.74 - 6.55 (m, 3H), 5.40 (d, *J* = 1.6 Hz, 1H), 5.39 (d, *J* = 1.8 Hz, 1H), 3.90 (s, 3H), 3.75 (s, 9H). ¹³C NMR (75 MHz, Acetone-*d*₆) (δ ppm): 153.9 (C=O), 151.8 (2 C), 139.4 (C), 138.9 (C), 138.7 (C), 137.2 (C), 134.3 (2 C), 128.7 (CH), 128.3 (C), 124.4 (CH), 121.3 (CH), 119.3 (CH), 112.8 (CH₂), 110.3 (CH), 106.6 (2 CH), 103.0 (CH), 60.5 (OCH₃), 56.3 (2 OCH₃), 33.3 (CH₃). HRMS (ESI) for C₂₃H₂₅N₂O₅ [M+H]⁺: calcd 409. 1771, found 409.1763.

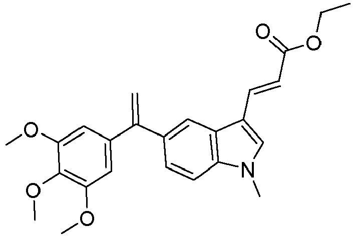

### Ethyl (E)-3-(1-methyl-5-(1-(3,4,5-trimethoxyphenyl)vinyl)-1H-indol-3-yl)acrylate (55).

Le composé **55** a été préparé selon le procédé A à partir de **(*Z*)-4-methyl-N'-(1-(3,4,5-trimethoxyphenyl)ethylidene)benzenesulfonohydrazide** (1 mmol) et de l'**ethyl (*E*)-3-(5-bromo-1-methyl-1H-indol-3-yl)acrylate** (1,5 mmol). Une purification par chromatographie sur colonne sur du gel de silice a donné 380 mg de 55 (rendement 90%). Huile jaune; TLC: R_{f} = 0.25 (EtOAc: cyclohexane, 2:8); ¹H NMR (300 MHz, CDCl₃) (δ ppm): 7.97 (s, 1H), 7.91 (d, *J* = 15.9 Hz, 1H), 7.39 (s, 1H), 7.31 (d, *J* = 8.6 Hz, 1H), 7.27 (d, *J* = 8.6 Hz, 1H), 6.63 (s, 2H), 6.40 (d, *J* = 15.9 Hz, 1H), 5.49 (s, 2H), 4.29 (q, *J* = 7.1 Hz, 2H), 3.92 (s, 3H), 3.85 (s, 3H), 3.83 (s, 6H), 1.37 (t, *J* = 7.1 Hz, 3H). ¹³C NMR (75 MHz, CDCl₃) (δ ppm): 168.3 (C=O), 153.0 (2 C), 150.8 (C), 138.0 (C), 137.9 (2 C), 137.7 (CH), 135.0 (C), 133.3 (CH), 126.1 (C), 124.1 (CH), 120.3 (CH), 113.6 (CH₂), 112.9 (CH), 112.5 (C), 109.5 (CH), 105.8 (CH), 61.0 (CH₃), 60.2 (CH₂), 56.2 (CH₃), 33.4 (CH₃), 14.5 (CH₃). HRMS (ESI) for C₂₅H₂₈NO₅ [M+H]⁺: calcd 422.1967, found 422.1953.

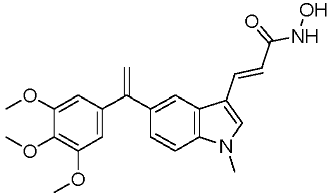

### (E)-N-hydroxy-3-(1-methyl-5-(1-(3,4,5-trimethoxyphenyl)vinyl)-1H-indol-3-yl)acrylamide (11).

Ce composé a été préparé selon le procédé D à partir de **Ethyl (*E*)-3-(1-methyl-5-(1-(3,4,5-trimethoxyphenyl)vinyl)-1*H*-indol-3-yl)acrylate (55)**. La purification par HPLC a donné 220 mg de **11** (rendement 54%). Huile jaune; TLC: R_{f} = 0.18 (MeOH: dichloromethane, 5:95); ¹H NMR (300 MHz, Acetone-*d*₆) (δ ppm): 10.14 (s, 1H), 8.45 (s, 1H), 7.96 (s, 1H), 7.80 (d, *J* = 15.5 Hz, 1H), 7.68 (s, 1H), 7.43 (d, *J* = 8.4, 1H), 7.16 (d, *J* = 8.4 Hz, 1H), 6.73 - 6.45 (m, 1H), 5.49 (s, 1H), 5.43 (s, 1H), 3.89 (s, 3H), 3.76 (s, 3H). ¹³C NMR (75 MHz, Acetone-*d*₆) (δ ppm): 154.0 (C=O), 152.0 (2 C), 151.9 (C), 138.8 (C), 138.4 (C), 135.3 (2C), 134.7 (CH), 126.8 (C), 124.3 (CH), 123.5 (CH), 120.5 (CH), 118.4 (CH), 113.5 (CH₂), 110.6 (CH), 106.7 (2 CH), 60.5 (OCH₃), 56.4 (2 OCH₃), 33.3 (CH₃). HRMS (ESI) for C₂₃H₂₅N₂O₅ [M+H]⁺: calcd 409. 1771, found 409.1782.

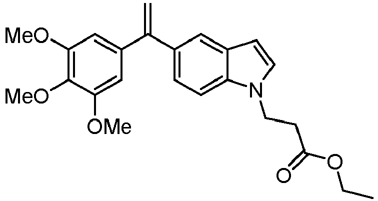

### Ethyl 3-(5-(1-(3,4,5-trimethoxyphenyl)vinyl-1H-indol-1-yl)propanoate (56).

Le composé 56 a été préparé selon le procédé A à partir de **(Z)-4-methyl-N'-(1-(3,4,5-trimethoxyphenyl)ethylidene)benzenesulfonohydrazide** (1 mmol) et de l'**ethyl 3-(5-bromo-1H-indol-1-yl)propanoate** (1,5 mmol). Une purification par chromatographie sur colonne sur du gel de silice a donné 143 mg de **56** (rendement 35 %). Huile jaune; TLC: R_{f} = 0.22 (EtOAc: cyclohexane, 2:8); ¹H NMR (300 MHz, CDCl₃) (δ ppm):7.62 (d, *J* = 1.6 Hz, 1H), 7.30 (d, *J* = 8.5 Hz, 1H), 7.23 (dd, *J* = 8.5, 1.6 Hz, 1H), 7.15 (d, *J* = 3.2 Hz, 1H), 6.61 (s, 2H), 6.47 (d, *J* = 3.2 Hz, 1H), 5.43 (d, *J* = 1.5 Hz, 1H), 5.36 (d, *J* = 1.5 Hz, 1H), 4.46 (t, *J* = 6.8 Hz, 2H), 4.13 (q, *J* = 7.1 Hz, 2H), 3.89 (s, 3H), 3.80 (s, 6H), 2.83 (t, *J* = 6.8 Hz, 2H), 1.22 (t, *J* = 7.1 Hz, 3H). ¹³C NMR (75 MHz, CDCl₃) (δ ppm): 164.7 (C=O), 152.9 (2 C), 150.3 (C), 133.0 (2 C), 129.2 (C), 128.6 (CH), 122.6 (CH), 121.1 (CH), 120.9 (C), 112.7 (CH₂), 109.7 (C), 108.8 (CH), 105.9 (2 CH), 102.1 (CH), 56.2 (3 OCH₃), 42.0 (OCH₂), 35.1 (CH₂), 29.8 (CH₂), 14.2 (CH₃). HRMS (ESI) for C₂₄H₂₈NO₅ ([M + H]⁺): calcd 410.1967, found 410.1967

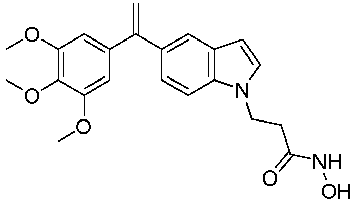

### N-hydroxy-3-(5-(1-(3,4,5-trimethoxyphenyl)vinyl)-1H-indol-1-yl)propanamide (44).

Ce composé a été préparé selon le procédé D à partir de l**'Ethyl 3-(5-(1-(3,4,5-trimethoxyphenyl)vinyl-1H-indol-1-yl)propanoate (56).** La purification par HPLC a donné 140 mg de **44** (rendement 35%). Huile brune; TLC: R_{f} = 0.18 (MeOH: dichloromethane, 5:95); ¹H NMR (300 MHz, CDCl₃) (δ ppm): 8.12 (s, 1H), 7.78 (d, *J* = 8.3 Hz, 1H), 7.42 (d, *J* = 8.3 Hz, 1H), 7.24 (d, *J* = 3.4 Hz, 2H), 7.07 (s, 2H), 6.59 (d, *J* = 3.4 Hz, 1H), 5.41 (s, 1H), 5.36 (s, 1H), 4.51 (t, *J* = 6.7 Hz, 2H), 3.94 (s, 3H), 3.86 (s, 6H), 2.93 (t, *J* = 6.7 Hz, 2H). ¹³C NMR (75 MHz, CDCl₃) (δ ppm): 164.1 (C=O), 152.8 (2C), 141.6 (C), 141.5 (C), 138.1 (2C), 134.1 (C), 129.6 (CH), 128.0 (C), 125.4 (CH), 124.1 (CH), 112.5 (CH₂), 109.1 (CH), 107.8 (2 CH), 103.7 (CH), 61.1 (OCH₃), 56.4 (2 OCH₃), 41.9 (CH₂), 34.6 (CH₂). HRMS (ESI) for C₂₂H₂₅N₂O₅ [M+H]⁺: calcd 397.1763, found 397.1763.

### Etude biologique in vitro des composés selon l'invention.

Des molécules représentantes ont été préparées et leur effet sur la prolifération de lignées cellulaires humaines cancéreuses ainsi que leur capacité à inhiber la polymérisation de la tubuline ont été étudiées.

### 1- Activité cytotoxique.

L'activité cytotoxique des composés préparés a été évaluée sur différentes lignées cellulaires cancéreuses humaines. La lignée sélectionnée a été incubée à 37 °C en présence de l'un des nouveaux composés, ajouté dans le milieu de culture à différentes concentrations. La concentration inhibitrice induisant la mort de 50% des cellules (CI₅₀) a été déterminée après 72 heures d'incubation pour chaque composé (Tableau 1 et 2).

**Tableau 1 : Activité cytotoxique des composés selon l'invention comparée à la référence isoCA-4.**

| Numéro | Structure | HCT116^{a} CI₅₀ (nM) | K562R CI₅₀ (nM) | IPT^{b} CI₅₀ (µM) |
|---|---|---|---|---|
| **1** | 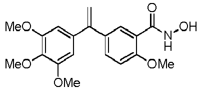 | **233 ± 22** | **nd** | **20 ± 1,2** |
| **2** | 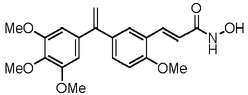 | **2,5** | **1,6** | **1,6 ± 0,4** |
| **3** | 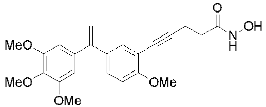 | **35** | **33** | **1,7 ± 0,3** |
| **4** | 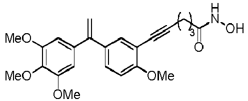 | **372 ± 5** | **nd** | **18 ± 1,2** |
| **8** | 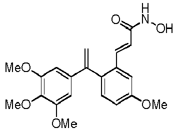 | **2 ± 0,1** | **nd** | **2.1 ± 0.4** |
| **11** | 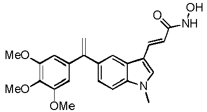 | **2.2 ± 0.26** | **nd** | **3.7 ± 0.5** |
| **12** | 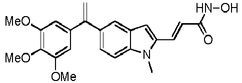 | **12 ± 2,5** | **nd** | **5.4 ± 1.5** |
| **17** | 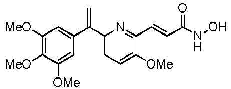 | **37.2 ± 0,60** | **nd** | **7.5 ± 1.2** |
| **25** | 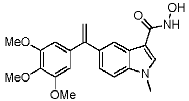 | **30 ± 1,5** | **nd** | **7.3 ± 3.3** |
| **43** | 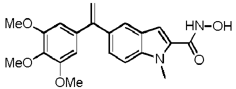 | **36 ± 4** | **nd** | **81 ± 15** |
| **44** | 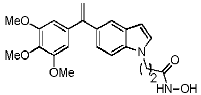 | **393 ± 4,1** | **nd** | **22 ± 2** |
| isoCA-4 | 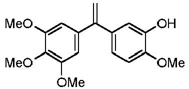 | 2 | nd | 1,8 **±** 0,4 |

Le composé **2** a montré une excellente activité antiproliférative avec une CI₅₀ de 2,5 nM sur la lignée cellulaire HCT116 (carcinome colorectal), similaire à celle de la molécule de référence, l'isoCA-4. De plus **2** est également cytotoxique à des concentrations nanomolaires (CI₅₀ = 1,6 nM) sur la lignée cellulaire K562R (leucémie humaine issues de tumeurs résistantes à l''imatinib). Le composé **3** possédant une fonction acétylénique s'est révélé également être cytotoxique à des concentrations nanomolaires. Les composés **11, 12, 25** et **43** possédants un noyau indole présentent également une excellente activité antiproliférative, ainsi que le composé **17** possédant un noyau pyridine.

**Tableau 2 : Activité cytotoxique du composé 2 sur diverses lignées tumorales humaines.**

| Cytotoxicité de **2** CI₅₀ (nM) | | | | | | | | |
|---|---|---|---|---|---|---|---|---|
| A549 | K562 | MCF7 | PC3 | U87 | U2OS | SOJ6 | BxPC3 | MiaPaCa 2 |
| 1,7 | 1,6 | 2,0 | 2,4 | 1,6 | 1,9 | 6,8 | 2,9 | 5,1 |

Le composé **2** a montré également une cytotoxicité envers d'autres lignées tumorales humaines, telles que le cancer du poumon (A549), la leucémie myéloïde chronique (K562), le cancer du sein (MCF7), le cancer de la prostate (PC3), le glioblastome (U87), l'ostéosarcome (U2OS), et sur les lignées cellulaires tumorales pancréatiques (SOJ6, BxPC-3 et MiaPaCa-2) avec des CI₅₀ comprises entre 1,6 nM et 6,8 nM.

### 2- Inhibition de la polymérisation de la tubuline (IPT).

Pour vérifier si les activités antiprolifératives des nouvelles molécules **2** et **3** corrèlent avec leurs effets antitubulines, il a été mesuré *in vitro* leur capacité à inhiber la polymérisation de la tubuline en microtubules. La tubuline a été purifiée à partir de cervelles de brebis selon la méthode de Shelanski par deux cycles d'assemblage-désassemblage. La solution mère (15-20 mg/mL), stockée à -196 °C, est décongelée et diluée dans le tampon d'assemblage (0.1 M MES, 0.5 mM MgCl₂, 1 mM EGTA, and 1 mM GTP, pH 6.6) pour avoir une concentration finale de 10 µM. L'assemblage de la tubuline a été suivi par fluorescence sur plaques 96-puits selon la méthode de Barron et al. A la solution de tubuline (10 µM, 100 µL par puits) a été ajouté l'inhibiteur (DMSO, 1 µL) et la solution a été incubée 45 min à température ambiante. Le GTP (1 mM final) a été ensuite ajouté, la solution est mélangée rapidement et la fluorescence (λex = 350 nm, λem = 440 nm) a été mesurée sur un fluorimètre Wallac Victor (Perkin Elmer). La détermination de l'inhibition de 50% du taux d'assemblage maximum (CI₅₀) a été réalisée en duplicate ou triplicate sur 10 concentrations encadrant la CI₅₀. Pour comparaison, la désoxypodophyllotoxine et l'isoCA-4 ont été utilisés comme contrôle positif.

Les composés **2** et **3** possèdent une forte capacité à inhiber l'assemblage de la tubuline avec des valeurs de CI₅₀ de 1,6 et 1,7 µM, respectivement, très proche de celles de la molécule témoin, l'isoCA-4. Une forte corrélation entre l'inhibition de la polymérisation de la tubuline et l'activité cytotoxique est ainsi établie au sein de ces deux composés.

### 3- Inhibition de l'activité HDAC.

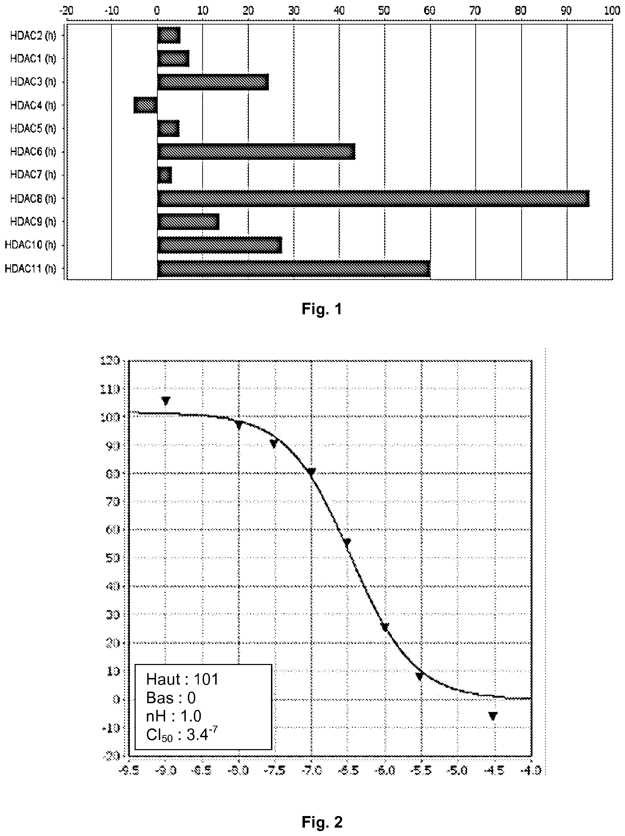

Il a été également testé l'activité de composés **2** et **3** sur l'inhibition des HDAC 1 à 11. Le composé **2** a montré une activité inhibitrice sélective vis-à-vis de HDAC8 (Figure 1) avec une CI₅₀ (0.34 µM, Figure 2) similaire à celle de la molécule de référence, le trichostatin A, (CI₅₀ = 0,32 µM).

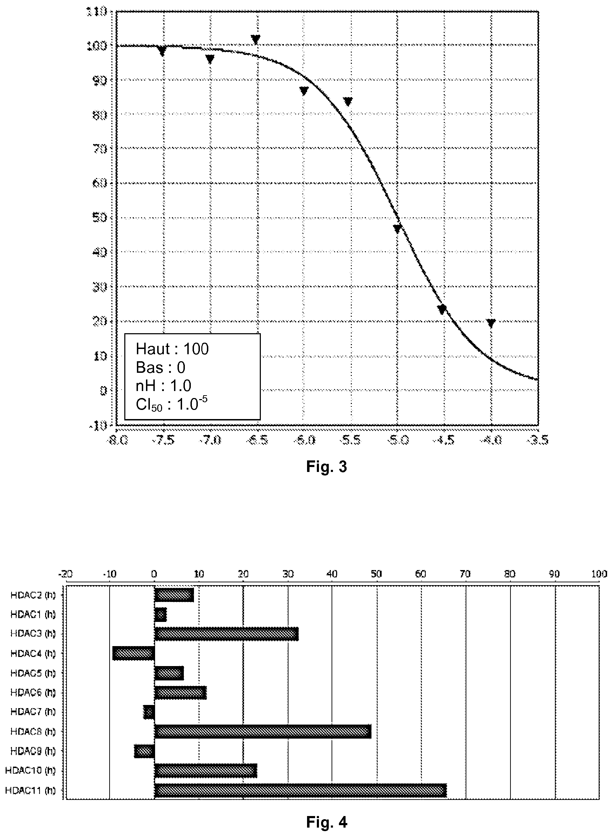

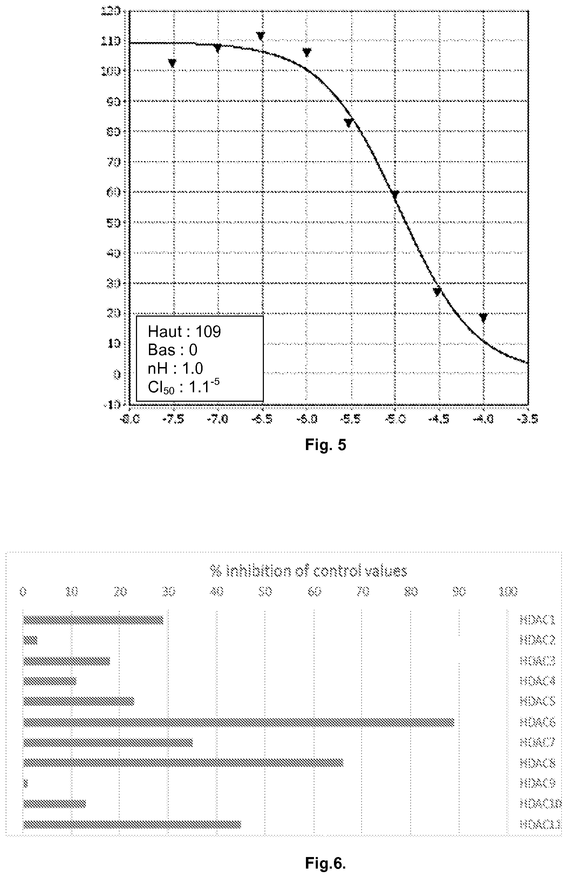

Avantageusement, les HDAC8 ont montré une activité thérapeutique dans le cas de lymphomes malins. Le composé **3** a montré une activité inhibitrice sélective vis-à-vis des HDAC8 et des HDAC11 (Figure 4) avec une CI₅₀ (11 µM, Figure 5).

Le composé **11** a montré une activité inhibitrice sélective vis-à-vis des HDAC6 et des HDAC8 (Figure 6).

Le composé **8** a montré une activité inhibitrice sélective vis-à-vis des HDAC6, des HDAC8 et des HDAC11 (Figure 7), tout comme le composé **12** (Figure 8).

## Revendications

1. Composé de formule (I) suivante : 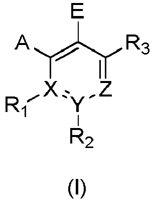 dans laquelle :
- R₂ et R₃ sont différents et l'un des deux parmi R₂ et R₃ représente un groupement A1 de formule générale suivante : 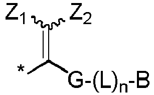 dans lequel :
- B représente un groupement chélatant du zinc choisi parmi la liste comprenant les motifs de formules générales suivantes : 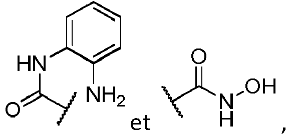
- n représente un entier choisi parmi 0 ou 1 ;
- L représente :
- -(CH₂)ᵣ- ;
- -CH=CH-(CH₂)ᵣ- ;
- -CH=CH-CH=CH-(CH₂)ᵣ- ;
- -C=C-CH≡CH-(CH₂)r⁻ ;
- -C≡C-(CH₂)ᵣ- ;
- -C≡C-CH=CH-(CH₂)ᵣ- ;
où r est un entier allant de 0 à 6, de préférence allant de 0 à 4 ;
- Z₁ représente un atome d'hydrogène, d'halogène ;
- Z₂ représente un atome choisi parmi un hydrogène, et un halogène, un groupement choisi parmi un nitrile, et un groupement B, à la condition que si Z₂=B alors le groupement -(L)ₙ-B est absent de G ;
- les liaisons 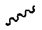 signifient que la double liaison portant Z₁, respectivement, Z₂ est de stéréochimie E ou Z ;
- * est l'atome de carbone portant R₂ ou R₃ ;
- G représente un phényle ou un hétéroaryle :
- Lorsque G est un phényle, il est substitué par un groupement R₂₀ choisi parmi OMe et SMe en position *para,* relativement à la position de la double liaison portant Z₁ et Z₂ ;
- Lorsque G est un hétéroaryle, il est choisi parmi pyridines, indoles, 1-méthylindoles, indolines, carbazoles, benzothiophènes et benzofuranes ;
- l'autre parmi R₂ et R₃ représente :
- un groupement OMe, lorsque X=Y=Z est un atome de carbone ;
- lorsque l'un de X, Y, Z est un atome d'azote ;
▪ un atome d'hydrogène ;
▪ un atome d'halogène ;
▪ un groupe hydroxyle ;
▪ un groupe cyano ;
▪ un groupe -COYR₁₀ avec Y désignant O ou N et R₁₀ désignant H ou un groupe alkyle en C₁ à C₆, un groupe alcényle en C₂ à C4, un groupe alcynyle en C₂ à C₄ ;
▪ un groupe -SO₂NR₁₀R₁₁ avec R₁₀, R₁₁, désignant chacun, indépendamment l'un de l'autre, H ou un groupe alkyle en C₁ à C₆, un groupe alcényle en C₂ à C₄, un groupe alcynyle en C₂ à C₄ ;
▪ un groupe -NHSO₂R₁₂ avec R₁₂ désignant un groupe alkyle en C₁ à C₆, un groupe alcényle en C₂ à C₄, un groupe alcynyle en C₂ à C₄, un groupe aryle, un groupe hétéroaryle ;
▪ un groupe alkyle en C₁ à C₆ ;
▪ un groupe alcényle en C₂ à C₄ ;
▪ un groupe alcynyle en C₂ à C₄ ;
▪ un groupe alkoxy en C₁ à C₆ ; ou
▪ un groupe -NR₁₃R₁₄ avec R₁₃ et R₁₄ représentant, indépendamment l'un de l'autre un hydrogène ou un groupe alkyle en C₁ à C₆ ;
- X, Y et Z représentent, indépendamment l'un de l'autre, un atome de carbone ou d'azote à la condition que si X et Z représentent un atome d'azote, Y représente un atome de carbone;
- E représente :
- un atome d'hydrogène, un groupement -OMe, lorsque X=Y=Z= carbone et R₂ = A₁ ;
- un atome d'hydrogène, d'halogène, lorsque X=Y=Z= carbone et R₃ = A₁;
- un atome d'hydrogène, d'halogène, un groupement nitrile, lorsque l'un de X, Y ou Z = azote ;
- A représente :
- un groupement -OMe, lorsque X=Y=Z= carbone ;
- un atome d'hydrogène, d'halogène, un groupement nitrile, lorsque l'un de X, Y ou Z = azote ;
- R₁ représente :
- un atome d'hydrogène, un groupement -OMe, lorsque X=Y=Z= carbone et R₃ = A₁ ;
- un atome d'hydrogène, d'halogène, lorsque X=Y=Z= carbone et R₂ = A₁ ;
- un atome d'hydrogène, d'halogène, un groupement nitrile, lorsque l'un de X, Y ou Z = azote ;
ou bien,
- A et E participent ensemble à un cycle aromatique accolé de formule suivante : 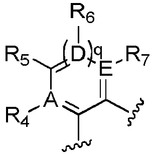
- Alors A, E, D représentent :
- un atome de carbone ou d'azote ;
- R₁, R₄, R₅, R₆, R₇, si présents, représentent, indépendamment l'un de l'autre :
• un atome d'hydrogène ;
• un atome d'halogène ;
• un groupe hydroxyle ;
• un groupe alkyle en C₁ à C₆ ;
• un groupe alcényle en C₂ à C₄ ;
• un groupe alcynyle en C₂ à C₄ ;
• un groupe alkoxy en C₁ à C₆ ; ou
• un groupe -NR₁₃R₁₄ avec R₁₃ et R₁₄ représentant, indépendamment l'un de l'autre un hydrogène ou un groupe alkyle en C₁ à C₆ ;
- q représente un entier compris entre 0 et 2 ; et au moins un de A, D, E, X, Y et Z représente un atome d'azote à la condition que si X et Z représentent un atome d'azote, Y représente un atome de carbone ;
ainsi que ses sels pharmaceutiquement acceptables, ses stéréoisomères et ses prodrogues de conjugaison avec un antigène.

2. Composé selon la revendication précédente, **caractérisé en ce que** B est un acide hydroxamique selon le formule "-CONHOH".

3. Composé selon l'une quelconque des revendications précédentes, **caractérisé en ce qu'**il répond à la formule générale suivante : 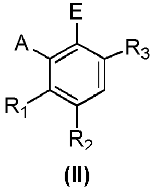 R₁, R₂, R₃, A et E sont tels que définis à la revendication 1.

4. Composé selon l'une quelconque des revendications précédentes, **caractérisé en ce qu'**il répond à la formule générale suivante : 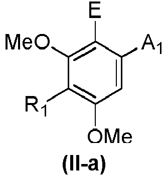 E, R₁ et A₁ sont tels que définis à la revendication 1.

5. Composé selon la revendication 1, **caractérisé en ce qu'**il répond à la formule générale suivante : 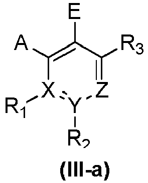 où au moins un de X, Y, Z représente un atome d'azote et A, E, R₁, R₂, R₃ sont tels que définis à la revendication 1.

6. Composé selon la revendication 5, **caractérisé en ce qu'**il répond à la formule générale suivante : 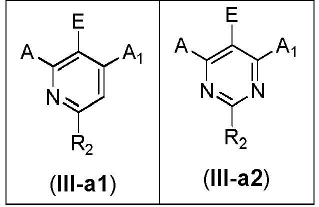 où A, E, R₂, A₁ sont tels que définis à la revendication 1.

7. Composé selon la revendication 1, **caractérisé en ce qu'**il répond à la formule générale suivante : 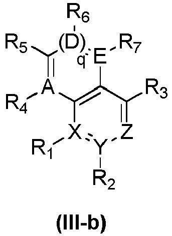 où au moins l'un de A, D, E, X, Y et Z représente un atome d'azote, et X, Y, Z, A, E, D, R₁, R₂, R₃, R₄, R₅, R₆, R₇, et q sont tels que définis à la revendication 1.

8. Composé selon la revendication 7, **caractérisé en ce qu'**il répond à la formule générale suivante : 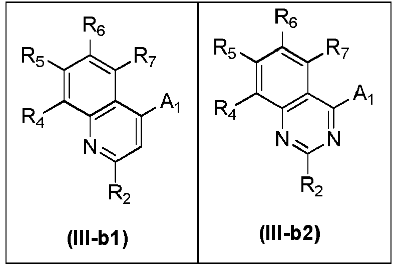 où R₂, A₁, R₄, R₅, R₆, R₇, sont tels que définis à la revendication 1.

9. Composé selon l'une quelconque des revendications précédentes **caractérisé en ce que** le groupement A₁ est choisi parmi les groupements de formule générale suivante : 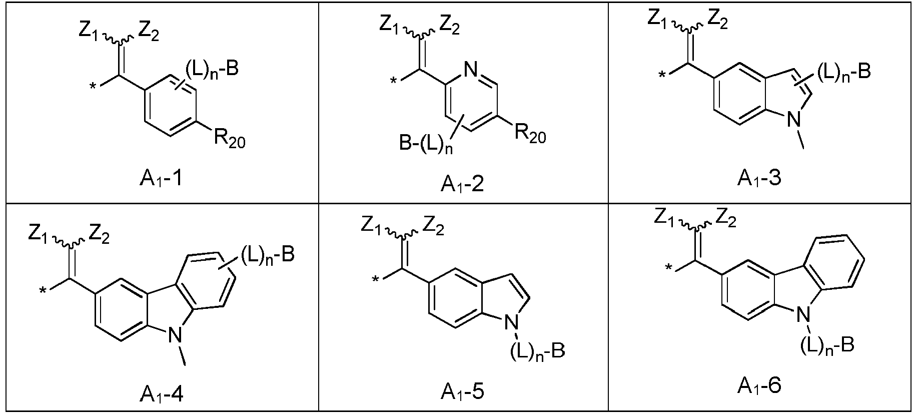 où :
- Z₁ et Z₂ représentent chacun indépendamment de l'autre un atome d'hydrogène, un atome d'halogène choisi parmi le fluor, le chlore et le brome, de préférence le fluor, ou un groupement nitrile ;
- R₂₀, B, L et n sont tel que définis précédemment, et ;
- les liaisons 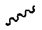 signifient que la double liaison portant Z₁, respectivement, Z₂ est de stéréochimie E ou Z ;
- * est l'atome de carbone portant R₂ ou R₃.

10. Composé selon l'une quelconque des revendications 1 à 8 **caractérisé en ce que** le groupement A₁ est choisi parmi les groupements de formule générales suivantes : 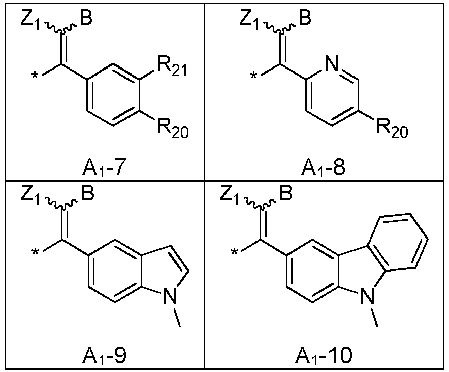 où :
- Z₁ représente un atome d'hydrogène, un atome d'halogène choisi parmi le fluor, le chlore et le brome, de préférence le fluor, ou un groupement nitrile ;
- R₂₀, B, sont tel que définis précédemment ;
- R₂₁ représente un atome d'hydrogène, un groupement choisi parmi -OH, -NH₂, F N₃, -C=CH, -C≡C(CH₂)ₘOH, où m est un entier compris entre 0 et 5, (E)-CH=CHCH₂OH, (*E*)-CH=CHCOOR, où R est un atome d'hydrogène ou un groupement alkyle en C₁ à C₄, et ;
- les liaisons 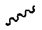 signifient que la double liaison portant Z₁, respectivement, Z₂ est de stéréochimie E ou Z ;
* est l'atome de carbone portant R₂ ou R₃.

11. Composé selon l'une quelconque des revendications précédentes, **caractérisé en ce qu'**il répond à l'une des formules suivantes :
| | |
|---|---|
| 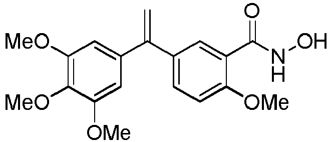 | 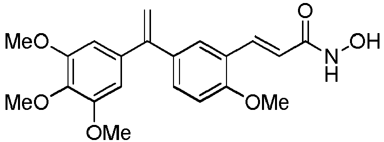 |
| **1** | **2** |
| 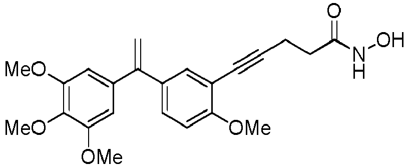 | 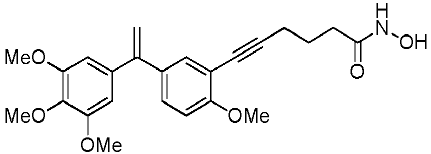 |
| **3** | **4** |
| 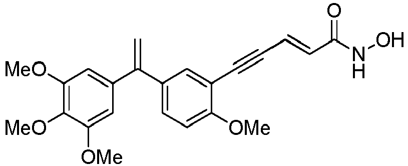 | 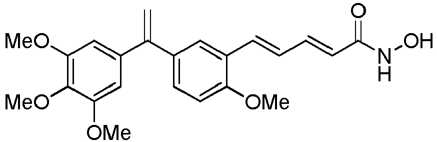 |
| **5** | **6** |
| 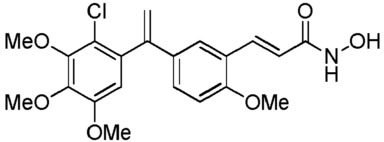 | 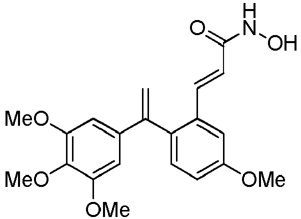 |
| **7** | **8** |
| 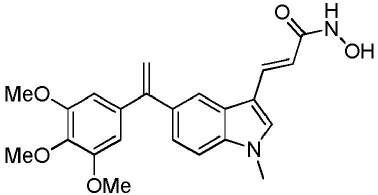 | 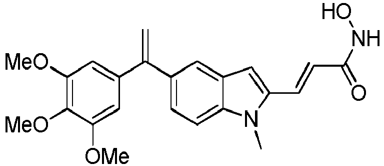 |
| **11** | **12** |
| 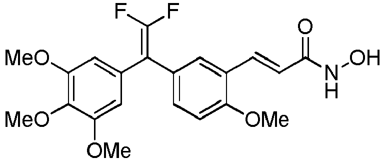 | 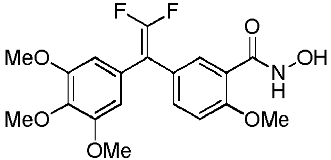 |
| **13** | **14** |
| 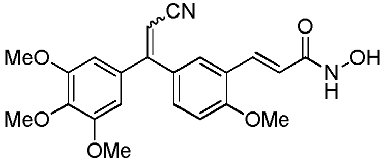 | 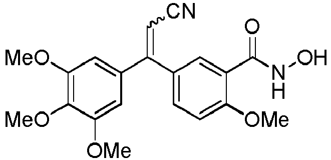 |
| **15** | **16** |
| 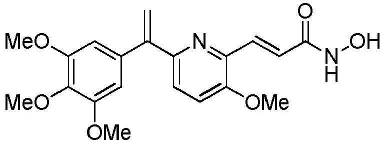 | 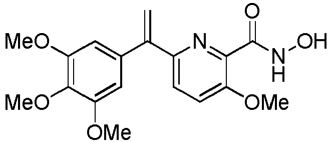 |
| **17** | **18** |
| 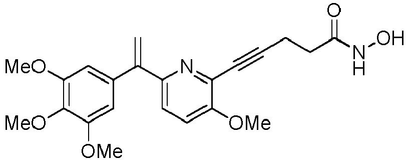 | 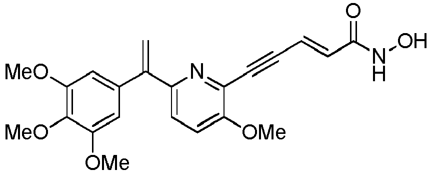 |
| **19** | **20** |
| 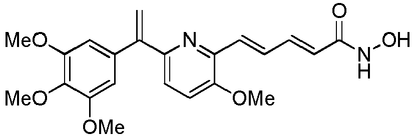 | 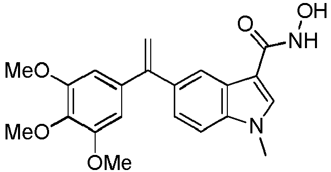 |
| **21** | **25** |
| 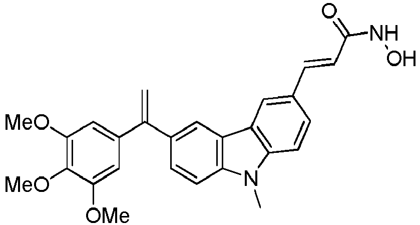 | 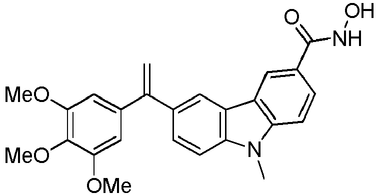 |
| **26** | **27** |
| 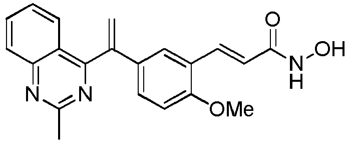 **28** | 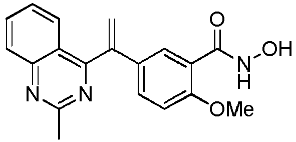 **29** |
| 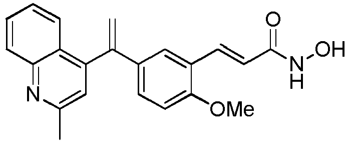 | 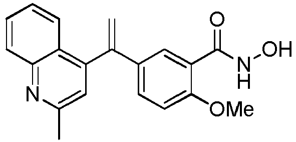 |
| **30** | **31** |
| 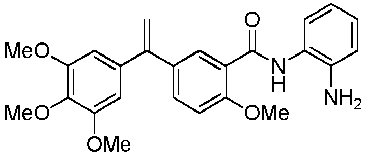 | 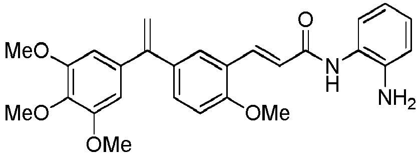 |
| **32** | **33** |
| 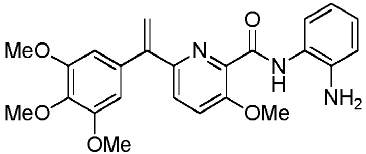 | 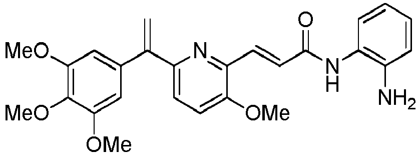 |
| **34** | **35** |
| 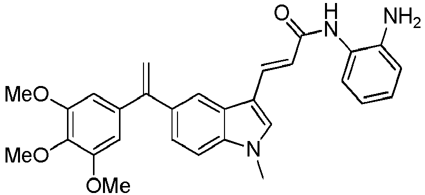 | 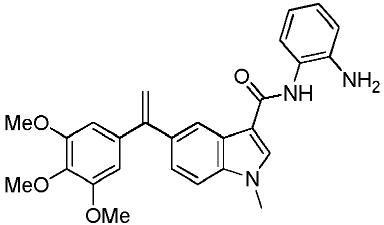 |
| **38** | **39** |
| 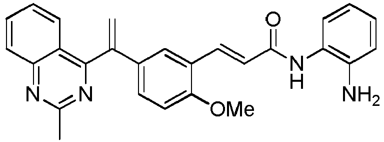 | 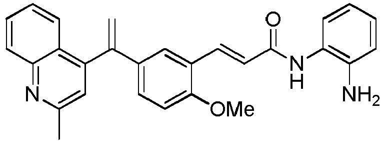 |
| **40** | **41** |
| 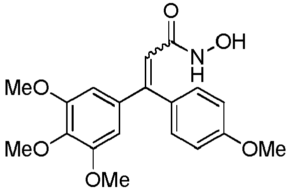 | 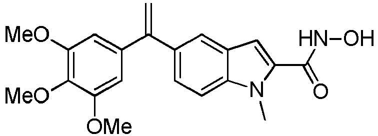 |
| **42** | **43** |
| 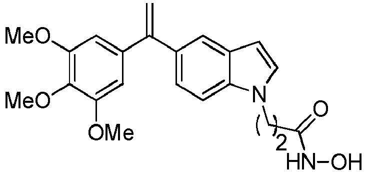 | |
| **44** | |

12. Composé selon l'une quelconque des revendications précédentes, **caractérisé en ce qu'**il répond à l'une des formules suivantes :
| | |
|---|---|
| 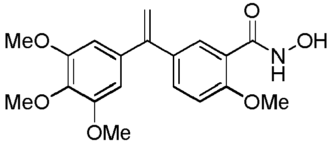 | 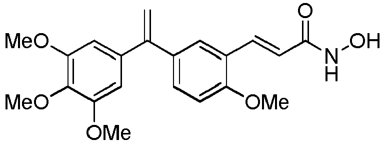 |
| **1** | **2** |
| 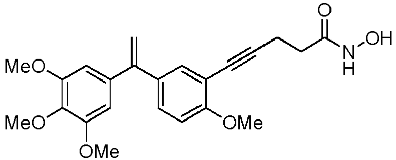 | 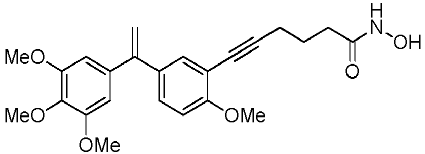 |
| **3** | **4** |
| 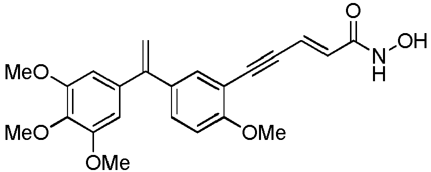 | 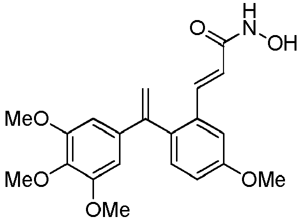 |
| **5** | **8** |
| 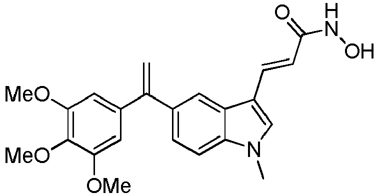 | 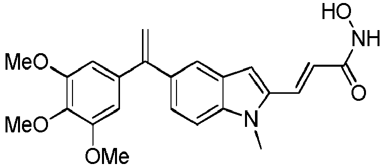 |
| **11** | **12** |
| 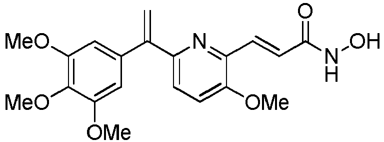 | 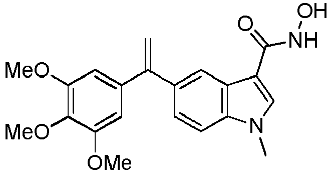 |
| **17** | **25** |
| 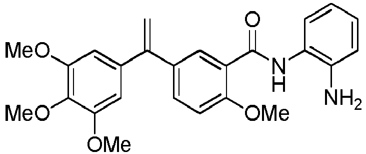 | 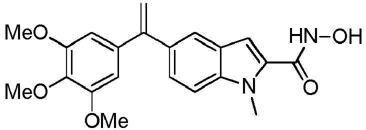 |
| **32** | **43** |
| 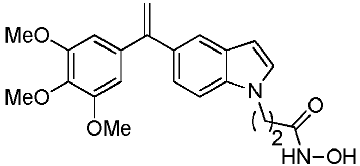 | |
| **44** | |

13. Procédé de préparation d'un composé de formule (**II**), (**III-a**) ou (**III-b1**) comprenant les étapes successives suivantes :
1. Réaction du composé de formule générale suivante : 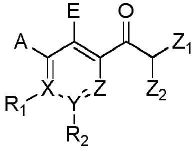 avec la tosylhydrazine pour conduire à la tosylhydrazone de formule générale suivante : 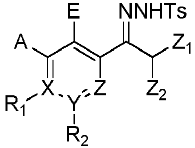 où Z₁, Z₂, X, Y, Z, A, E, R₁, R₂, sont tels que définis précédemment ;
2. Couplage métallo-catalysé de la tosylhydrazone obtenue à l'étape précédente avec un composé de formule générale I-G-Hal pour l'obtention du composé de formule générale suivante : 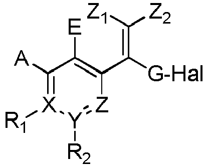 le couplage métallo-catalysé étant avantageusement réalisé avec du palladium, et Hal représente un halogène choisi parmi un atome de brome ou un atome de chlore, où G est tel que défini précédemment ;
3. Couplage métallo-catalysé réalisé sur le composé obtenu à l'étape précédente suivi d'un traitement permettant l'introduction du groupement - (L)ₙ-B et l'obtention du composé de formule générale suivante : 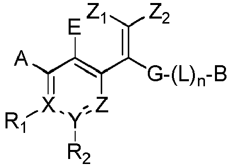 avantageusement le couplage métallo catalysé est réalisé avec du palladium ou du cuivre, et où L, n et B sont tels que définis précédemment.

14. Composition pharmaceutique comprenant au moins un composé de formule (I) selon l'une quelconque des revendications 1 à 12, ainsi que leurs sels pharmaceutiquement acceptables, en association avec un ou plusieurs excipients pharmaceutiquement acceptables.

15. Composition pharmaceutique selon la revendication 14, **caractérisée en ce qu'**elle comprend au moins un autre principe actif, avantageusement choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide, le lanréotide, l'acide (Z)-3-[2,4-diméthyl-5-(2-oxo-1,2-dihydro-indol-3-ylidèneméthyl)-**1**H-pyrrol-3-yl]-propionique, l'acide 4-((9-chloro-7-(2,6-difluorophényl)-5H-pyrimidol(5,4-d)(2)benzazépin-2-yl)amino)benzoïque, l'acide 5,6-diméthylxanthénone-4-acétique et l'acide 3-(4-(1,2-diphénylbut-1-ényl)phényl)acrylique.

16. Composition pharmaceutique comprenant :
(i) au moins un composé de formule (I) selon l'une quelconque des revendications 1 à 12,
et
(ii) au moins un autre principe actif,
en tant que produit de combinaison pour une utilisation simultanée, séparée ou étalée dans le temps, avantageusement pour la prévention ou le traitement du cancer, en particulier les lymphomes malins et les leucémies humaines.

17. Composé de formule générale (**I**) selon l'une quelconque des revendications 1 à 12 ou composition pharmaceutique selon l'une des revendications 14 à 16 utilisé à la fois pour diminuer ou inhiber la polymérisation de la tubuline, et diminuer ou inhiber les HDAC, notamment *in vitro* et également *in vivo.*

18. Composé ou composition selon la revendication 17 utilisé dans la prévention ou le traitement du cancer, typiquement les lymphomes malins, les leucémies humaines, le carcinome colorectal, le cancer du poumon, les leucémies myéloïdes chroniques, les leucémies myéloïdes chroniques résistantes à l'imatinib, le cancer du sein, le cancer de la prostate, les glioblastomes, les ostéosarcomes, et lignées cellulaires tumorales pancréatiques.

19. Conjugué comprenant l'association de :
- un anticorps, un fragment d'anticorps ou un équivalent, de préférence l'anticorps est un anticorps monoclonal ;
- une molécule de liaison, et ;
- un composé selon l'une quelconque des revendications 1 à 12 ; liés entre eux de façon covalente.

## Patentansprüche

1. Verbindung mit der folgenden Formel (I): 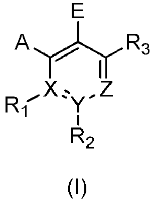 in der:
- R₂ und R₃ unterschiedlich sind und eines von beiden von R₂ und R₃ eine Gruppe A1 mit der folgenden allgemeinen Formel darstellt: 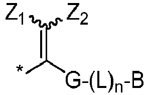
in der:
- B eine zinkchelatbildende Gruppe darstellt, die aus der Liste ausgewählt ist, die die Strukturelemente mit den folgenden allgemeinen Formeln umfassen: 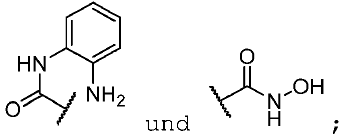
- n eine Ganzzahl ist, die aus 0 oder 1 ausgewählt ist;
- L darstellt:
- - (CH₂)ᵣ-;
- -CH=CH- (CH₂)ᵣ-;
- -CH=CH-CH=CH-(CH₂)ᵣ-;
- -C=C-CH≡CH-(CH₂)ᵣ-;
- -C_{≡}C-(CH₂)ᵣ-;
- -C≡C-CH=CH- (CH₂)ᵣ-;
wo r eine Ganzzahl zwischen 0 und 6, vorzugsweise zwischen 0 und 4, ist;
- Z₁ ein Wasserstoff-, Halogenatom darstellt;
- Z₂ ein Atom, das aus einem Wasserstoff und einem Halogen ausgewählt ist, eine Gruppe, die aus einem Nitril und eine Gruppe B ausgewählt ist, darstellt, unter der Bedingung, dass, wenn Z₂=B, dann die Gruppe -(L)ₙ-B nicht in G vorhanden ist;
- die 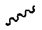 -Bindungen bedeuten, dass die Doppelbindung, die Z₁ beziehungsweise Z₂ trägt, E- oder Z-Stereochemie aufweist;
- * das Kohlenstoffatom ist, das R₂ oder R₃ trägt;
- G ein Phenyl oder ein Heteroaryl darstellt:
- wenn G ein Phenyl ist, es mit einer Gruppe R₂₀ substituiert wird, die aus OMe und SMe an der para-Position in Bezug auf die Position der Doppelbindung, die Z₁ und Z₂ trägt, ausgewählt wird;
- wenn G ein Heteroaryl ist, es aus Pyridinen, Indolen, 1-Methylindolen, Indolinen, Carbazolen, Benzothiophenen und Benzofuranen ausgewählt wird;
- das andere von R₂ und R₃ darstellt:
- eine OMe-Gruppe, wenn X=Y=Z ein Kohlenstoffatom ist;
- wenn eines von X, Y, Z ein Stickstoffatom ist;
▪ ein Wasserstoffatom;
▪ ein Halogenatom;
▪ eine Hydroxylgruppe;
▪ eine Cyanogruppe;
▪ eine -COYR₁₀-Gruppe, wobei Y O oder N bezeichnet und R₁₀ H oder eine Alkylgruppe an C₁ bis C₆, eine Alkenylgruppe an C₂ bis C₄, eine Alkinylgruppe an C₂ bis C₄ bezeichnet;
▪ eine -SO₂NR₁₀R₁₁-Gruppe, wobei R₁₀, R₁₁ jeweils unabhängig voneinander H oder eine Alkylgruppe an C₁ bis C₆, eine Alkenylgruppe an C₂ bis C₄, eine Alkinylgruppe an C₂ bis C₄ bezeichnen;
▪ eine -NHSO₂R₁₂-Gruppe, wobei R₁₂ eine Alkylgruppe an C₁ bis C₆, eine Alkenylgruppe an C₂ bis C₄, eine Alkinylgruppe an C₂ bis C₄, eine Arylgruppe, eine Heteroarylgruppe bezeichnet;
▪ eine Alkylgruppe an C₁ bis C₆;
▪ eine Alkenylgruppe an C₂ bis C₄;
▪ eine Alkinylgruppe an C₂ bis C₄;
▪ eine Alkoxygruppe an C₁ bis C₆; oder
▪ eine -NR₁₃R₁₄-Gruppe, wobei R₁₃ und R₁₄ unabhängig voneinander einen Wasserstoff oder eine Alkylgruppe an C₁ bis C₆ darstellen;
- X, Y und Z unabhängig voneinander ein Kohlenstoff- oder Stickstoffatom darstellen, unter der Bedingung, dass, wenn X und Z ein Stickstoffatom darstellen, Y ein Kohlenstoffatom darstellt;
- E darstellt:
- ein Wasserstoffatom, eine -OMe-Gruppe, wenn X=Y=Z= Kohlenstoff und R₂ = A₁;
- ein Wasserstoff-, Halogenatom, wenn X=Y=Z= Kohlenstoff und R₃ = A₁;
- ein Wasserstoff-, Halogenatom, eine Nitrilgruppe, wenn eines von X, Y oder Z = Stickstoff;
- A darstellt:
- eine -OMe-Gruppe, wenn X=Y=Z= Kohlenstoff;
- ein Wasserstoff-, Halogenatom, eine Nitrilgruppe, wenn eines von X, Y oder Z = Stickstoff;
- R₁ darstellt:
- ein Wasserstoffatom, eine -OMe-Gruppe, wenn X=Y=Z= Kohlenstoff und R₃ = A₁;
- ein Wasserstoff-, Halogenatom, wenn X=Y=Z= Kohlenstoff und R₂ = A₁;
- ein Wasserstoff-, Halogenatom, eine Nitrilgruppe, wenn eines von X, Y oder Z = Stickstoff;
oder
- A und E zusammen an einem fusionierten aromatischen Ring mit der folgenden Formel beteiligt sind: 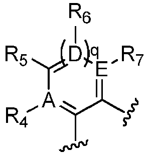
- A, E, D dann darstellen:
- ein Kohlenstoff- oder Stickstoffatom;
- R₁, R₄, R₅, R₆, R₇, falls vorhanden, unabhängig voneinander darstellen:
• ein Wasserstoffatom;
• ein Halogenatom;
• eine Hydroxylgruppe;
• eine Alkylgruppe an C₁ bis C₆;
• eine Alkenylgruppe an C₂ bis C₄;
• eine Alkinylgruppe an C₂ bis C₄;
• eine Alkoxygruppe an C₁ bis C₆; oder
• eine -NR₁₃R₁₄-Gruppe, wobei R₁₃ und R₁₄ unabhängig voneinander einen Wasserstoff oder eine Alkylgruppe an C₁ bis C₆ darstellen;
- q eine Ganzzahl zwischen 0 und 2 darstellt;
und mindestens eines von A, D, E, X, Y und Z ein Stickstoffatom darstellt, unter der Bedingung, dass, wenn X und Z ein Stickstoffatom darstellen, Y ein Kohlenstoffatom darstellt;
sowie ihre pharmazeutisch annehmbaren Salze, ihre Stereoisomere und ihre pro-Arzneimittel zur Konjugation mit einem Antigen.

2. Verbindung nach dem vorhergehenden Anspruch, **dadurch gekennzeichnet, dass** B eine Hydroxamsäure nach der Formel "-CONHOH" ist.

3. Verbindung nach einem der vorhergehenden Ansprüche, **dadurch gekennzeichnet, dass** sie die folgende allgemeine Formel erfüllt: 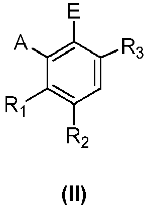 wobei R₁, R₂, R₃, A und E nach Anspruch 1 definiert sind.

4. Verbindung nach einem der vorhergehenden Ansprüche, **dadurch gekennzeichnet, dass** sie die folgende allgemeine Formel erfüllt: 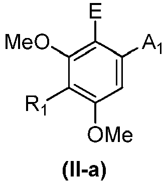 wobei E, R₁ und A₁ nach Anspruch 1 definiert sind.

5. Verbindung nach Anspruch 1, **dadurch gekennzeichnet, dass** sie die folgende allgemeine Formel erfüllt: 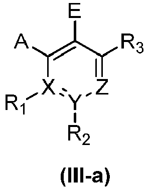 wobei mindestens eines von X, Y, Z ein Stickstoffatom darstellt und A, E, R₁, R₂, R₃ nach Anspruch 1 definiert sind.

6. Verbindung nach Anspruch 5, **dadurch gekennzeichnet, dass** sie die folgende allgemeine Formel erfüllt: 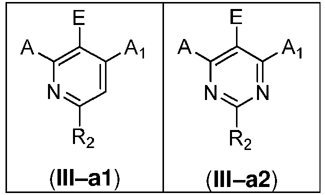 wo A, E, R₂, A₁ nach Anspruch 1 definiert sind.

7. Verbindung nach Anspruch 1, **dadurch gekennzeichnet, dass** sie die folgende allgemeine Formel erfüllt: 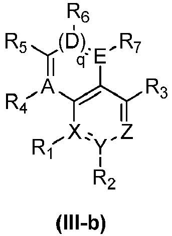 wobei mindestens eines von A, D, E, X, Y und Z ein Stickstoffatom darstellt und X, Y, Z, A, E, D, R₁, R₂, R₃, R₄, R₅, R₆, R₇ und q nach Anspruch 1 definiert sind.

8. Verbindung nach Anspruch 7, **dadurch gekennzeichnet, dass** sie die folgende allgemeine Formel erfüllt: 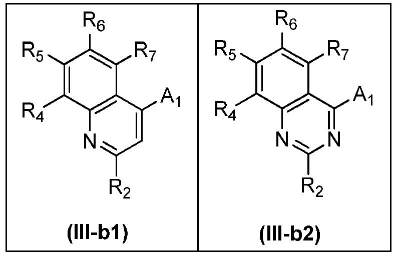 wo R₂, A₁, R₄, R₅, R₆, R₇ nach Anspruch 1 definiert sind.

9. Verbindung nach einem der vorhergehenden Ansprüche, **dadurch gekennzeichnet, dass** die Gruppe A₁ aus den Gruppen mit der folgenden allgemeinen Formel ausgewählt ist: 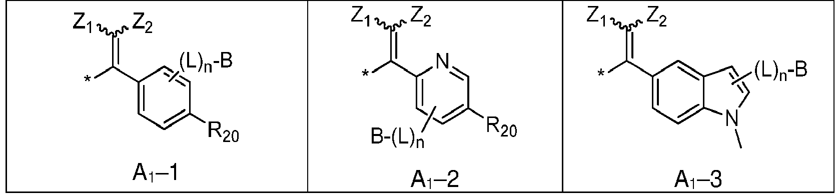 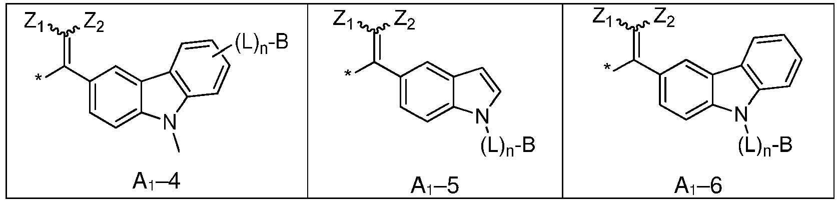 wo:
- Z₁ und Z₂ jeweils unabhängig voneinander ein Wasserstoffatom, ein Halogenatom, das aus Fluor, Chlor und Brom, vorzugsweise Fluor, ausgewählt ist, oder eine Nitrilgruppe sind;
- R₂₀, B, L und n wie vorhergehend definiert sind, und;
- die 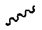 -Bindungen bedeuten, dass die Doppelbindung, die Z₁ beziehungsweise Z₂ trägt, E- oder Z-Stereochemie aufweist;
- * das Kohlenstoffatom ist, das R₂ oder R₃ trägt.

10. Verbindung nach einem der Ansprüche 1 bis 8, **dadurch gekennzeichnet, dass** die Gruppe A₁ unter den Gruppen mit den folgenden allgemeinen Formeln ausgewählt ist: 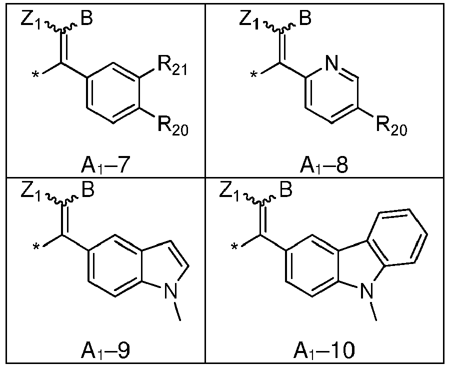 wo:
- Z₁ ein Wasserstoffatom, ein Halogenatom, das aus Fluor, Chlor und Brom, vorzugsweise Fluor, ausgewählt ist, oder eine Nitrilgruppe darstellt;
- R₂₀, B sind wie vorhergehend definiert;
- R₂₁ ein Wasserstoffatom, eine Gruppe darstellt, die ausgewählt ist aus -OH, -NH₂, F N₃, -C=CH, - C≡C(CH₂)ₘOH, wo m eine Ganzzahl zwischen 0 und 5 ist, (*E*)-CH=CHCH₂OH, (E)-CH=CHCOOR, wo R ein Wasserstoffatom oder eine Alkylgruppe an C₁ bis C₄ ist, und;
- die 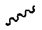 -Bindungen bedeuten, dass die Doppelbindung, die Z₁ beziehungsweise Z₂ trägt, E- oder Z-Stereochemie aufweist;
- * das Kohlenstoffatom ist, das R₂ oder R₃ trägt.

11. Verbindung nach einem der vorhergehenden Ansprüche, **dadurch gekennzeichnet, dass** sie mindestens eine der folgenden Formeln erfüllt:
| | |
|---|---|
| 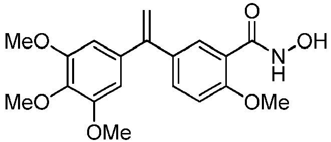 | 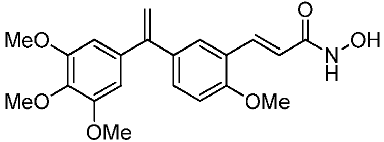 |
| **1** | **2** |
| 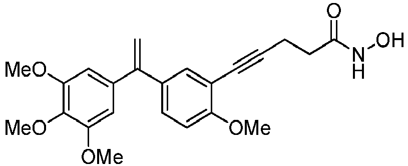 | 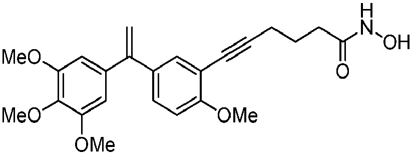 |
| **3** | **4** |
| 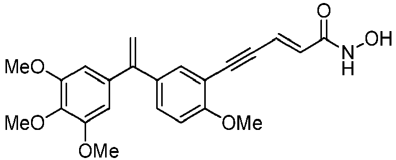 | 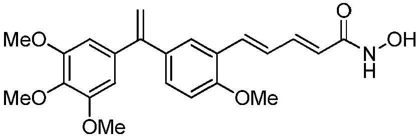 |
| **5** | **6** |
| 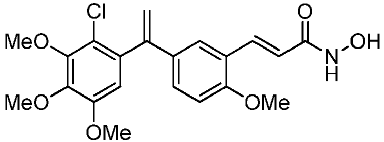 | 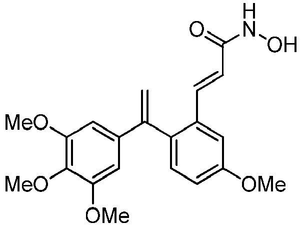 |
| **7** | **8** |
| 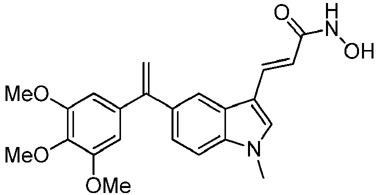 | 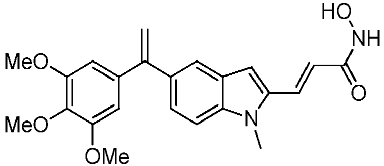 |
| **11** | **12** |
| 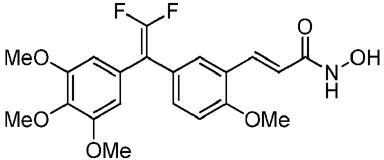 | 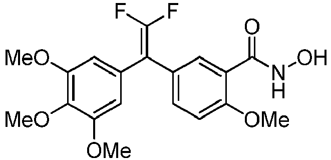 |
| **13** | **14** |
| 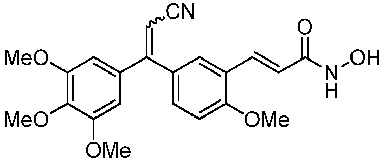 | 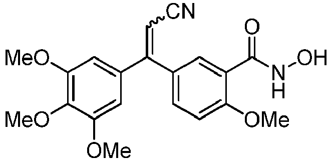 |
| **15** | **16** |
| 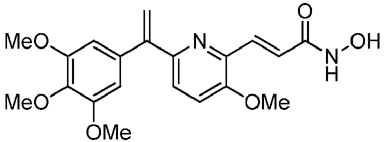 | 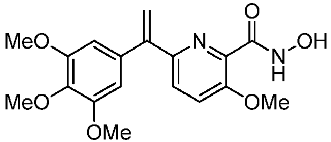 |
| **17** | **18** |
| 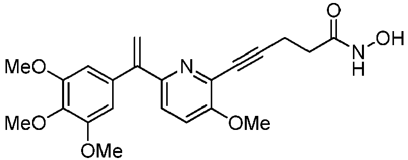 | 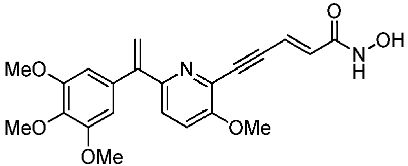 |
| **19** | **20** |
| 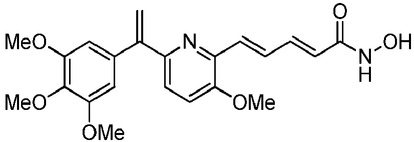 | 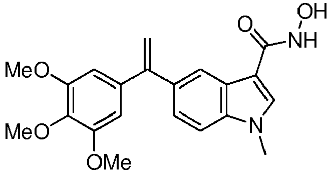 |
| **21** | **25** |
| 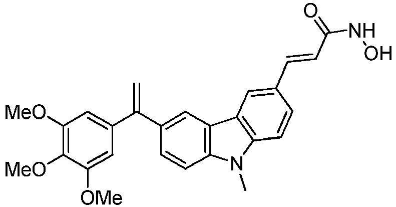 | 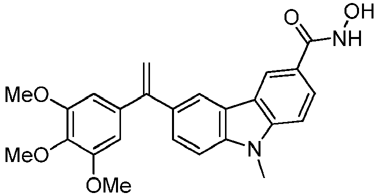 |
| **26** | **27** |
| 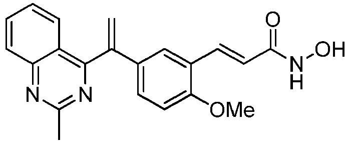 | 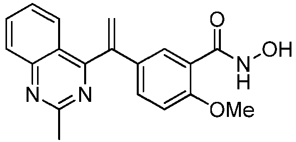 |
| **28** | **29** |
| 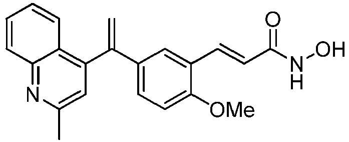 | 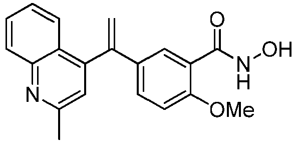 |
| **30** | **31** |
| 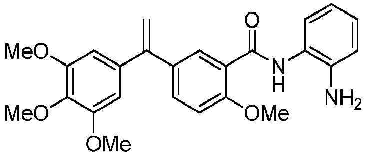 | 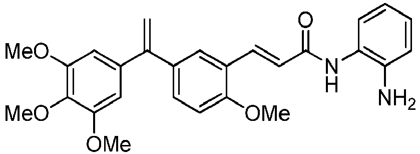 |
| **32** | **33** |
| 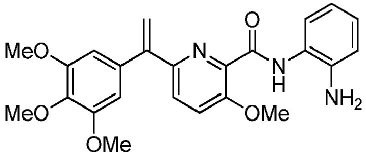 | 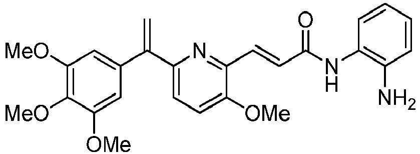 |
| **34** | **35** |
| 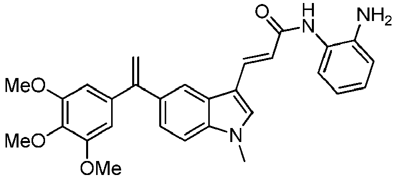 | 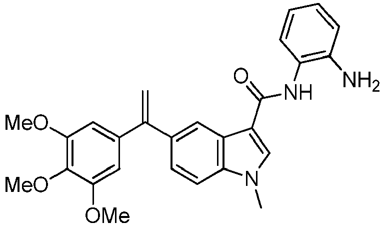 |
| **38** | **39** |
| 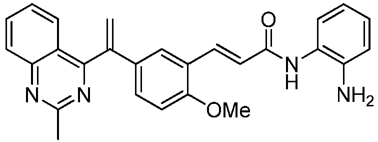 | 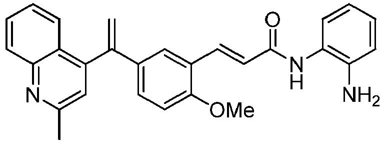 |
| **40** | **41** |
| 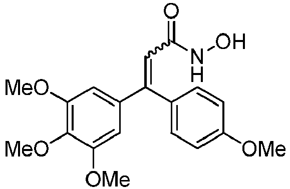 | 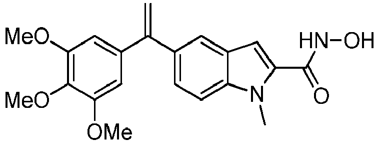 |
| **42** | **43** |
| 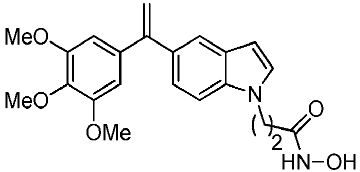 | |
| **44** | |

12. Verbindung nach einem der vorhergehenden Ansprüche, **dadurch gekennzeichnet, dass** sie eine der folgenden Formeln erfüllt:
| | |
|---|---|
| 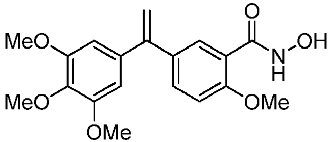 | 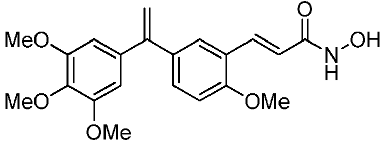 |
| **1** | **2** |
| 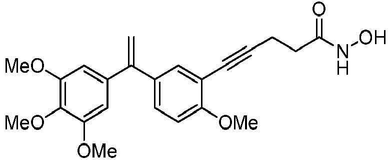 | 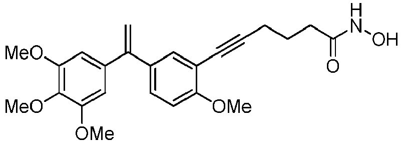 |
| **3** | **4** |
| 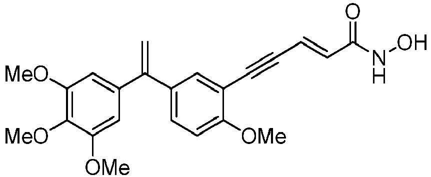 | 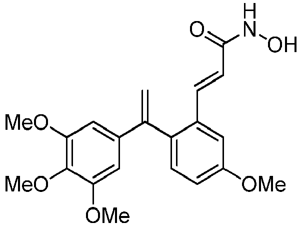 |
| **5** | **8** |
| 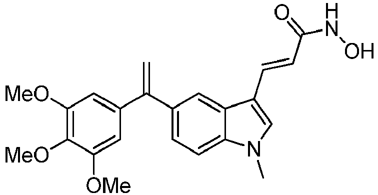 | 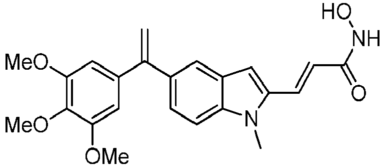 |
| **11** | **12** |
| 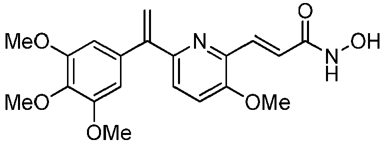 | 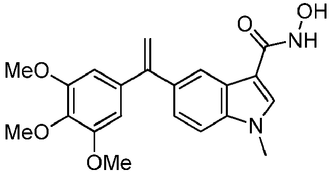 |
| **17** | **25** |
| 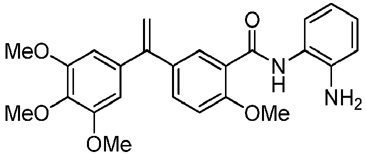 | 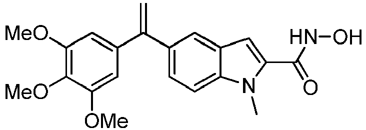 |
| **32** | **43** |
| 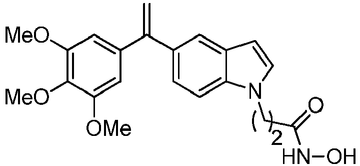 | |
| **44** | |

13. Verfahren zur Herstellung einer Verbindung mit der Formel **(II),** (**III-a**) oder (**III-b1**), das die folgenden aufeinanderfolgenden Schritte umfasst:
1. Reaktion der Verbindung mit der folgenden allgemeinen Formel: 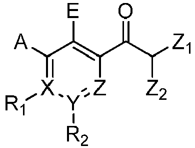 mit Tosylhydrazin, um zu dem Tosylhydrazon mit der folgenden allgemeinen Formel zu führen: 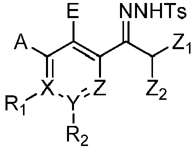 wo Z₁, Z₂, X, Y, Z, A, E, R₁, R₂ wie vorhergehend definiert sind;
2. Metallkatalysierte Kupplung des im vorhergehenden Schritt erhaltenen Tosylhydrazons mit einer Verbindung mit der allgemeinen Formel I-G-Hal für den Erhalt der Verbindung mit der folgenden allgemeinen Formel: 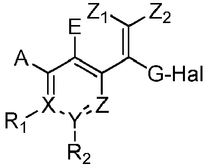 wobei die metallkatalysierte Kupplung vorzugsweise mit Palladium ausgeführt wird und Hal ein Halogen darstellt, das aus einem Bromatom oder einem Chloratom ausgewählt wird, wobei G ist wie vorhergehend definiert;
3. Metallkatalysierte Kupplung, die auf der im vorhergehenden Schritt erhaltenen Verbindung ausgeführt wird, gefolgt von einer Behandlung, die die Einführung der (L)ₙ-B-Gruppe und den Erhalt der Verbindung mit der folgenden allgemeinen Formel ermöglicht: 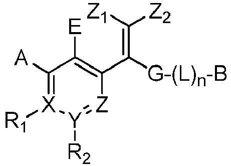 wobei die metallkatalysierte Kupplung vorzugsweise mit Palladium oder Kupfer ausgeführt wird und wo L, n und B sind wie vorhergehend definiert.

14. Pharmazeutische Zusammensetzung, die mindestens eine Verbindung mit der Formel (I) nach einem der Ansprüche 1 bis 12 sowie eines ihrer pharmazeutisch annehmbaren Salze in Verbindung mit einem oder mehreren pharmazeutisch annehmbaren Hilfsstoffen umfasst.

15. Pharmazeutische Zusammensetzung nach Anspruch 14, **dadurch gekennzeichnet, dass** sie mindestens einen anderen Wirkstoff umfasst, der vorteilhafterweise ausgewählt ist aus 6-Mercaptopurin, Fludarabin, Cladribin, Pentostatin, Cytarabin, 5-Fluorouracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid, Lanreotid, (Z)-3-[2,4-dimethyl-5-(2-oxo-1,2-dihydro-indol-3-ylidenmethyl)-1H-pyrrol-3-yl]-Propionsäure, 4-((9-Chlor-7-(2,6-difluorphenyl)-5H-pyrimidol(5,4-d) (2)benzazepin-2-yl)amino)-Benzoesäure, 5,6-Dimehtylxanthenon-4-Essigsäure und 3-(4-(1,2-Diphenylbut-1-enyl)phenyl)-Acrylsäure.

16. Pharmazeutische Zusammensetzung, die umfasst:
(i) mindestens eine Verbindung mit der Formel (I) nach einem der Ansprüche 1 bis 12,
und
(ii) mindestens einen anderen Wirkstoff,
als Kombinationsprodukt für eine gleichzeitige, getrennte oder zeitlich versetzte Verwendung, vorteilhafterweise zur Prävention oder zur Behandlung von Krebs, insbesondere maligner Lymphome und menschlicher Leukämien.

17. Verbindung mit der allgemeinen Formel (I) nach einem der Ansprüche 1 bis 12 oder pharmazeutische Zusammensetzung nach einem der Ansprüche 14 bis 16, die zugleich zur Verminderung oder Inhibition der Tubulinpolymerisierung und Verminderung oder Inhibition der HDAC, insbesondere *in vitro* und auch *in vivo,* verwendet wird.

18. Verbindung oder Zusammensetzung nach Anspruch 17, die bei der Prävention oder der Behandlung von Krebs, typischerweise maligner Lymphome, menschlicher Leukämien, kolorektalem Karzinom, Lungenkrebs, chronischer myeloischer Leukämien, gegen Imatinib resistenten chronischen myeloischen Leukämien, Brustkrebs, Prostatakrebs, Glioblastomen, Osteosarkomen, Pankreastumor-Zelllinien, verwendet wird.

19. Konjugat, das die Verbindung von Folgendem umfasst:
- einem Antikörper, einem Antikörperfragment oder einem Äquivalent, wobei der Antikörper vorzugsweise ein monoklonaler Antikörper ist;
- einem Bindungsmolekül, und;
- einer Verbindung nach einem der Ansprüche 1 bis 12;
die untereinander kovalent gebunden sind.

## Claims

1. A compound having the following formula (I): 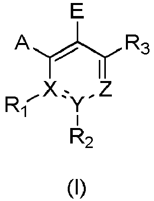 wherein:
- R₂ and R₃ are different and one of R₂ and R₃ represents a group A1 having the following general formula: 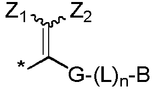 wherein:
- B represents a zinc chelating group selected from the list comprising the units having the following general formulas: 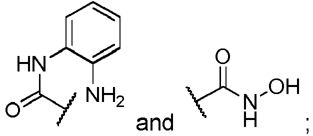
- n represents an integer selected from 0 or 1;
- L represents:
- -(CH₂)ᵣ-;
- -CH=CH-(CH₂)ᵣ-;
- -CH=CH-CH=CH-(CH₂)ᵣ-;
- -C=C-CH≡CH-(CH₂)ᵣ-;
- -C≡C-(CH₂)ᵣ-;
- -C≡C-CH=CH-(CH₂)ᵣ-;
where r is an integer from 0 to 6, preferably from 0 to 4;
- Z₁ represents a hydrogen atom, a halogen atom;
- Z₂ represents an atom selected from a hydrogen and a halogen, a group selected from a nitrile, and a group B, provided that if Z₂=B then the group -(L)ₙ-B is absent from G;
- the bonds 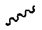 mean that the double bond bearing Z₁, respectively, Z₂ is of E or Z stereochemistry;
- * is the carbon atom bearing R₂ or R₃;
- G represents a phenyl or a heteroaryl:
- When G is a phenyl, it is substituted by a group R₂₀ selected from OMe and SMe in the para position, relative to the position of the double bond bearing Z₁ and Z₂;
- When G is a heteroaryl, it is selected from pyridines, indoles, 1-methylindoles, indolines, carbazoles, benzothiophenes and benzofurans;
- the other among R₂ and R₃ represents:
- an OMe group, when X=Y=Z is a carbon atom;
- when one of X, Y, Z is a nitrogen atom;
▪ a hydrogen atom;
▪ a halogen atom;
▪ a hydroxyl group;
▪ a cyano group;
▪ a group -COYR₁₀ with Y denoting O or N and R₁₀ denoting H or a (C₁ to C₆)alkyl group, a (C₂ to C₄)alkenyl group, a (C₂ to C₄)alkynyl group;
▪ a group -SO₂NR₁₀R₁₁ with R₁₀, R₁₁ each independently denoting H or a (C₁ to C₆)alkyl group, a (C₂ to C₄)alkenyl group, a (C₂ to C₄)alkynyl group;
▪ a group -NHSO₂R₁₂ with R₁₂ denoting a (C₁ to C₆)alkyl group, a (C₂ to C₄)alkenyl group, a C₂ to C₄ alkynyl group, an aryl group, a heteroaryl group;
▪ a (C₁ to C₆)alkyl group;
▪ a (C₂ to C₄)alkenyl group;
▪ a (C₂ to C₄)alkynyl group_{;}
▪ a (C₁ to C₆)alkoxy group; or
▪ a group -NR₁₃R₁₄ with R₁₃ and R₁₄ independently representing a hydrogen or a (C₁ to C₆)alkyl group;
- X, Y and Z independently represent a carbon or nitrogen atom provided that if X and Z represent a nitrogen atom, Y represents a carbon atom;
- E represents:
- a hydrogen atom, an -OMe group, when X=Y=Z= carbon and R₂ = A_{1;}
- a hydrogen atom, a halogen atom, when X=Y=Z= carbon and R₃ = A_{1;}
- a hydrogen atom, a halogen atom, a nitrile group, when one of X, Y or Z = nitrogen;
- A represents:
- an -OMe group, when X=Y=Z= carbon;
- a hydrogen atom, a halogen atom, a nitrile group, when one of X, Y or Z = nitrogen;
- R₁ represents:
- a hydrogen atom, an -OMe group, when X=Y=Z= carbon and R₃ = A_{1;}
- a hydrogen atom, a halogen atom, when X=Y=Z= carbon and R₂ = A_{1;}
- a hydrogen atom, a halogen atom, a nitrile group, when one of X, Y or Z = nitrogen;
or else,
- A and E together are part of a fused aromatic ring having the following formula: 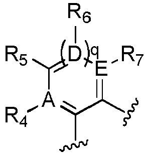
- Then A, E, D represent:
- a carbon or nitrogen atom;
- R₁, R₄, R₅, R₆, R₇, if present, independently represent:
• a hydrogen atom;
• a halogen atom;
• a hydroxyl group;
• a (C₁ to C₆)alkyl group;
• a (C₂ to C₄)alkenyl group;
• a (C₂ to C₄)alkynyl group_{;}
• a (C₁ to C₆)alkoxy group; or
• a group -NR₁₃R₁₄ with R₁₃ and R₁₄ independently representing a hydrogen or a (C₁ to C₆)alkyl group;
- q represents an integer between 0 and 2;
and at least one of A, D, E, X, Y and Z represents a nitrogen atom provided that if X and Z represent a nitrogen atom, Y represents a carbon atom;
as well as the pharmaceutically acceptable salts, stereoisomers and antigen conjugation prodrugs thereof.

2. The compound according to the preceding claim, **characterised in that** B is a hydroxamic acid according to the formula "-CONHOH".

3. The compound according to any one of the preceding claims, **characterised in that** it has the following general formula: 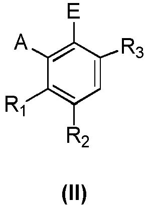 R₁, R₂, R₃, A and E are as defined in claim 1.

4. The compound according to any one of the preceding claims, **characterised in that** it has the following general formula: 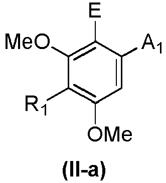 E, R₁ and A₁ are as defined in claim 1.

5. The compound according to claim 1, **characterised in that** it has the following general formula: 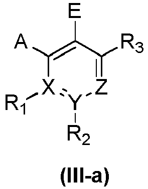 where at least one of X, Y, Z represents a nitrogen atom and A, E, R₁, R₂, R₃ are as defined in claim 1.

6. The compound according to claim 5, **characterised in that** it has the following general formula: 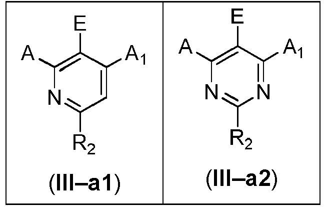 where A, E, R₂, A₁ are as defined in claim 1.

7. The compound according to claim 1, **characterised in that** it has the following general formula: 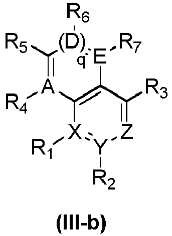 where at least one of A, D, E, X, Y and Z represents a nitrogen atom, and X, Y, Z, A, E, D, R₁, R₂, R₃, R₄, R₅, R₆, R₇, and q are as defined in claim 1.

8. The compound according to claim 7, **characterised in that** it has the following general formula: 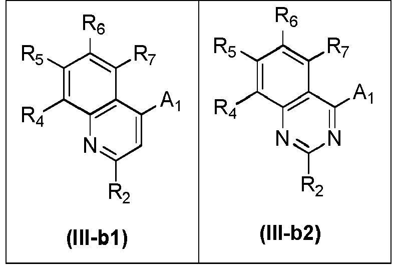 where R₂, A₁, R₄, R₅, R₆, R₇, are as defined in claim 1.

9. The compound according to any one of the preceding claims **characterised in that** the group A₁ is selected from the groups having the following general formulas: 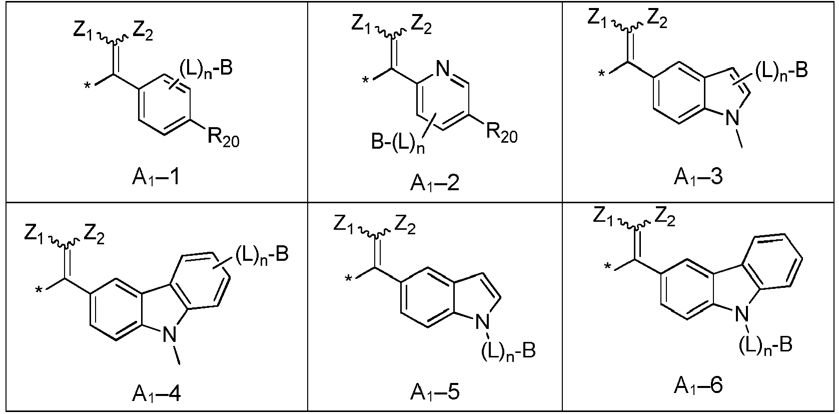 where:
- Z₁ and Z₂ each independently represent a hydrogen atom, a halogen atom selected from fluorine, chlorine and bromine, preferably fluorine, or a nitrile group;
- R₂₀, B, L and n are as defined above, and;
- the bonds 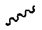 mean that the double bond bearing Z₁, respectively, Z₂ is of E or Z stereochemistry;
- * is the carbon atom bearing R₂ or R₃.

10. The compound according to any one of claims 1 to 8 **characterised in that** group A₁ is selected from the groups having the following general formulas: 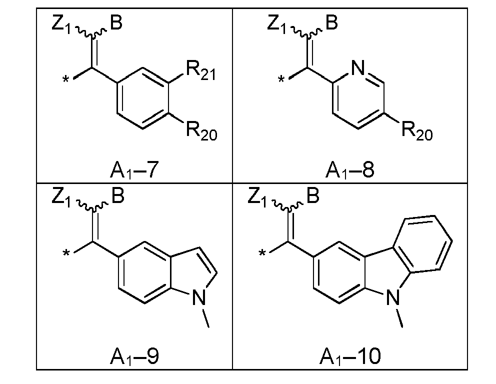 where:
- Z₁ represents a hydrogen atom, a halogen atom selected from fluorine, chlorine and bromine, preferably fluorine, or a nitrile group;
- R₂₀, B, are as defined above;
- R₂₁ represents a hydrogen atom, a group selected from -OH, -NH₂, F N₃, -C≡CH, -C≡C(CH₂)ₘOH, where m is an integer between 0 and 5, (*E*)-CH=CHCH₂OH, (*E*)-CH=CHCOOR, where R is a hydrogen atom or a (C₁ to C₄)alkyl group, and;
- the bonds 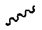 mean that the double bond bearing Z₁, respectively, Z₂ is of E or Z stereochemistry;
* is the carbon atom bearing R₂ or R₃.

11. The compound according to any one of the preceding claims, **characterised in that** it has one of the following formulas:
| | |
|---|---|
| 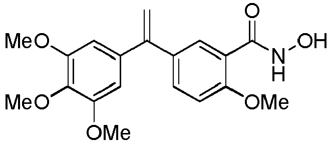 | 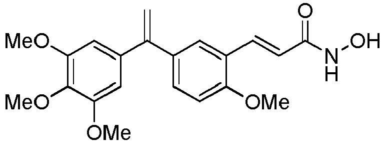 |
| **1** | **2** |
| 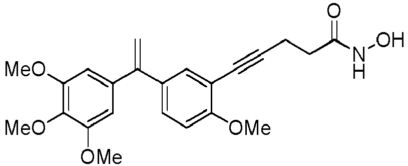 | 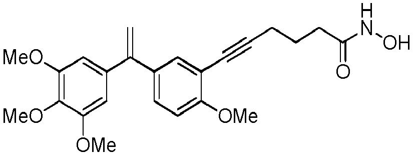 |
| **3** | **4** |
| 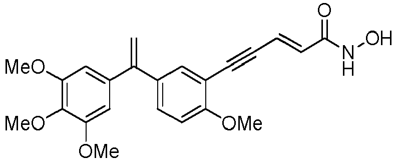 | 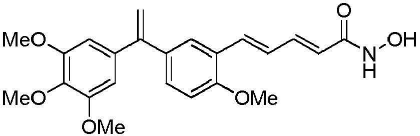 |
| **5** | **6** |
| 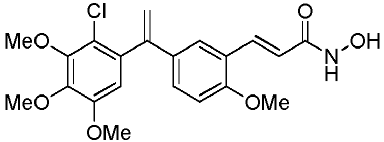 | 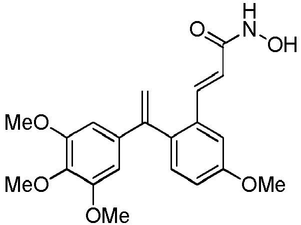 |
| **7** | **8** |
| 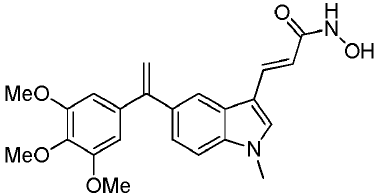 | 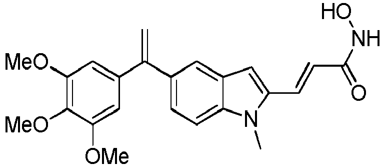 |
| **11** | **12** |
| 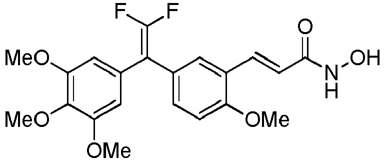 | 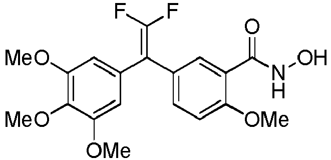 |
| **13** | **14** |
| 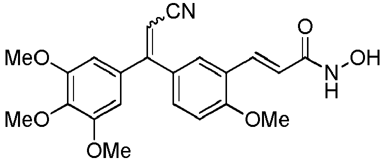 | 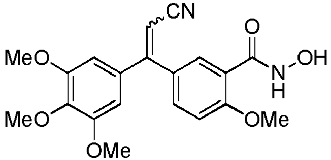 |
| **15** | **16** |
| 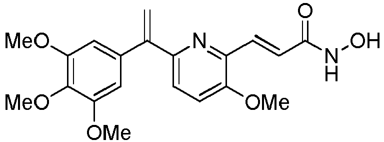 | 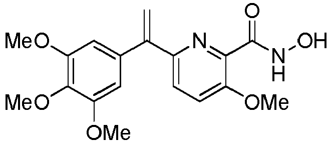 |
| **17** | **18** |
| 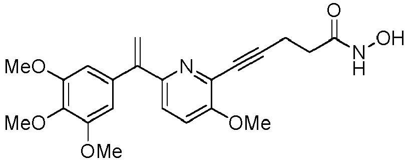 | 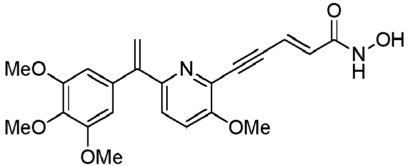 |
| **19** | **20** |
| 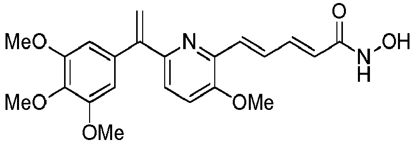 | 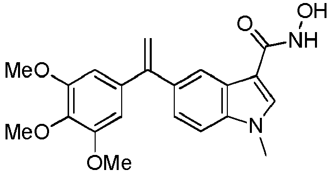 |
| **21** | **25** |
| 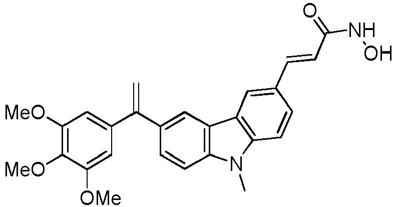 | 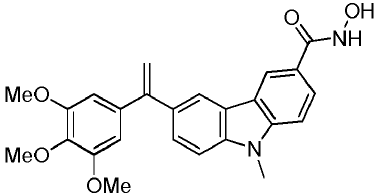 |
| **26** | **27** |
| 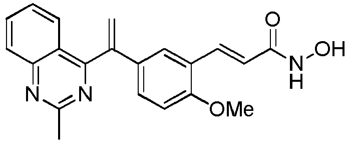 | 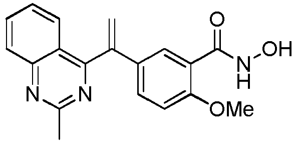 |
| **28** | **29** |
| 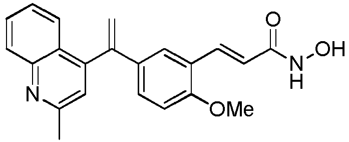 | 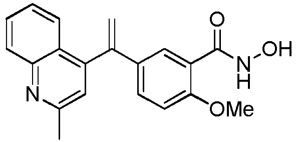 |
| **30** | **31** |
| 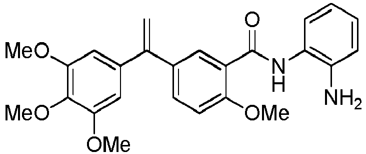 | 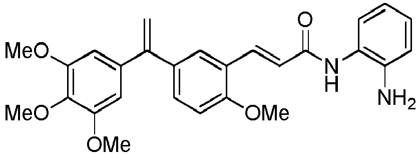 |
| **32** | **33** |
| 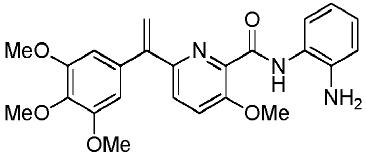 | 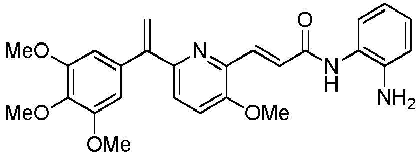 |
| **34** | **35** |
| 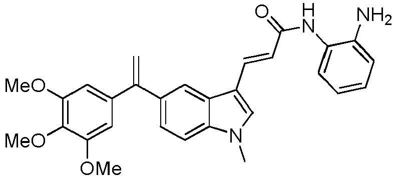 | 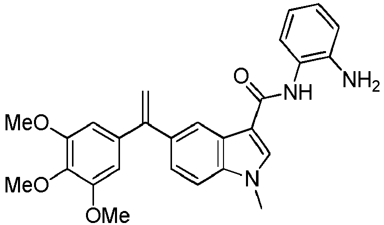 |
| **38** | **39** |
| 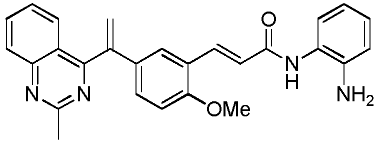 | 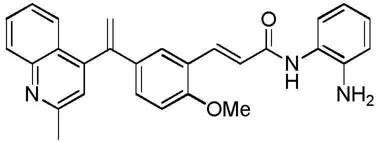 |
| **40** | **41** |
| 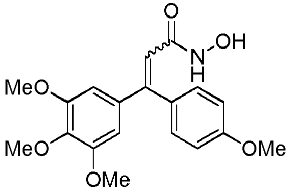 | 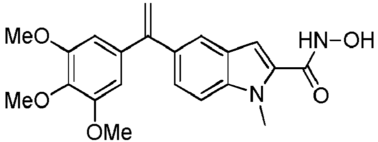 |
| **42** | **43** |
| 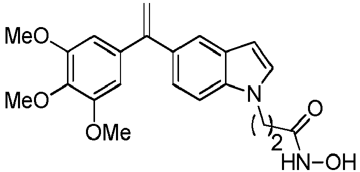 | |
| **44** | |

12. The compound according to any one of the preceding claims, **characterised in that** it has one of the following formulas:
| | |
|---|---|
| 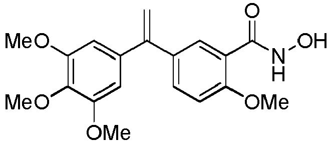 | 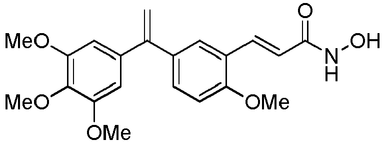 |
| **1** | **2** |
| 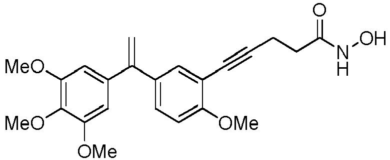 | 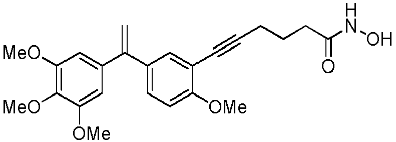 |
| **3** | **4** |
| 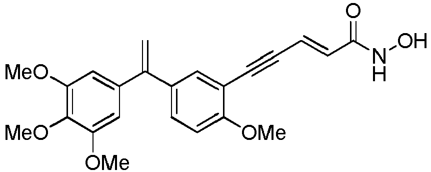 | 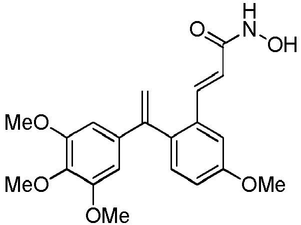 |
| **5** | **8** |
| 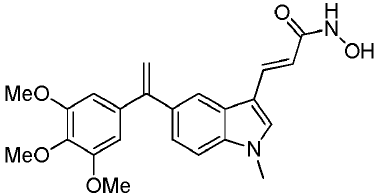 | 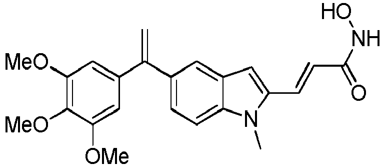 |
| **11** | **12** |
| 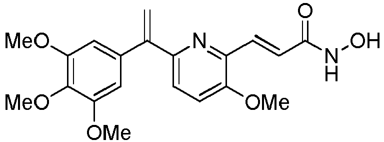 | 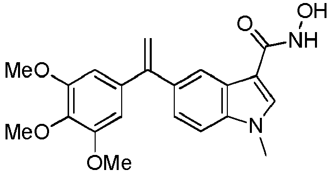 |
| **17** | **25** |
| 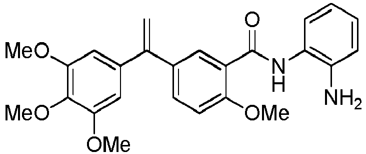 | 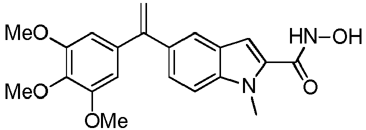 |
| **32** | **43** |
| 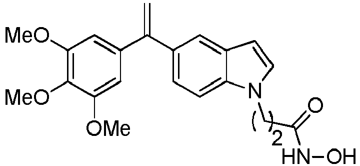 | |
| **44** | |

13. A process for preparing a compound of formula (**II**), (**III-a**) or (**III-b1**) comprising the following successive steps:
1. Reaction of the compound having the following general formula: 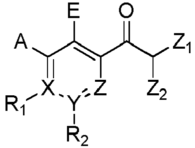 with tosylhydrazine to give the tosylhydrazone having the following general formula: 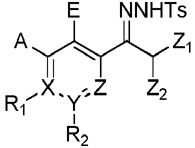 where Z₁, Z₂, X, Y, Z, A, E, R₁, R₂, are as defined above;
2. Metal-catalysed coupling of the tosylhydrazone obtained in the preceding step with a compound having the general formula I-G-Hal to obtain the compound having the following general formula: 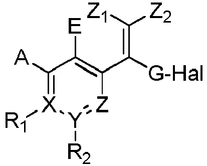 the metal-catalysed coupling being advantageously carried out with palladium, and Hal represents a halogen selected from a bromine atom or a chlorine atom, where G is as defined above;
3. Metal-catalysed coupling carried out on the compound obtained in the preceding step followed by a treatment allowing the introduction of the group -(L)ₙ-B and obtaining the compound having the following general formula: 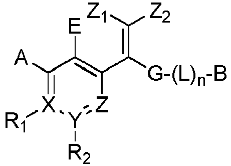 advantageously the metal-catalysed coupling is carried out with palladium or copper, and where L, n and B are as defined above.

14. A pharmaceutical composition comprising at least one compound of formula (I) according to any one of claims 1 to 12, as well as the pharmaceutically acceptable salts thereof, in combination with one or more pharmaceutically acceptable excipients.

15. The pharmaceutical composition according to claim 14, **characterised in that** it comprises at least one other active principle, advantageously selected from 6-mercaptopurine, fludarabine, cladribine, pentostatin, cytarabine, 5-fluorouracil, gemcitabine, methotrexate, raltitrexed, irinotecan, topotecan, etoposide, daunorubicin, doxorubicin, epirubicin, idarubicin, pirarubicin, mitoxantrone, chlormethine, cyclophosphamide, ifosfamide, melphalan, chlorambucil, busulfan, carmustine, fotemustine, streptozocine, carboplatin, cisplatin, oxaliplatin, procarbazine, dacarbazine, bleomycin, vinblastine, vincristine, vindesine, vinorelbine, paclitaxel, docetaxel, L-asparaginase, flutamide, nilutamide, bicalutamide, cyproterone acetate, triptorelin, leuprorelin, goserelin, buserelin, formestane, aminoglutethimide, anastrozole, letrozole, tamoxifen, octreotide, lanreotide, (Z)-3-[2,4-dimethyl-5-(2-oxo-1,2-dihydro-indol-3-ylidenemethyl)-1 H-pyrrol-3-yl]propionic acid, 4-((9-chloro-7-(2,6-difluorophenyl)-5H-pyrimidol(5,4-d)(2)benzazepin-2-yl)amino)benzoic acid, 5,6-dimethylxanthenone-4-acetic acid and 3-(4-(1,2-diphenylbut-1-enyl)phenyl)acrylic acid.

16. A pharmaceutical composition comprising:
(i) at least one compound of formula (I) according to any one of claims 1 to 12,
and
(ii) at least one other active principle,
as a combination product for simultaneous, separate or sequential use, advantageously for the prevention or treatment of cancer, in particular malignant lymphomas and human leukaemias.

17. The compound of general formula (I) according to any one of claims 1 to 12 or the pharmaceutical composition according to one of claims 14 to 16 used both to decrease or inhibit tubulin polymerisation and to decrease or inhibit HDACs, notably *in vitro* and also *in vivo.*

18. The compound or composition according to claim 17 used in the prevention or treatment of cancer, typically malignant lymphomas, human leukaemias, colorectal carcinoma, lung cancer, chronic myeloid leukaemias, imatinib-resistant chronic myeloid leukaemias, breast cancer, prostate cancer, glioblastomas, osteosarcomas, and pancreatic tumour cell lines.

19. A conjugate comprising the combination of:
- an antibody, an antibody fragment or equivalent, preferably the antibody is a monoclonal antibody;
- a linker molecule, and;
- a compound according to any one of claims 1 to 12;
covalently linked to each other.
